# EUROPEAN PATENT APPLICATION

(11) **EP 1 818 404 A2**
(43) Date of publication of application: **15.08.2007**
(21) Application number: 07100827.0
(22) Date of filing: 24.03.2005
(51) Int. Cl.: C12N 15/82

(54) **Genes and uses for plant improvement**

(30) Priority: 25.03.2004 US 556841 P
(62) Divisional of application: 05102392.7
(71) Applicant: Monsanto Technology LLC, St. Louis, Missouri 63167 (US)
(72) Inventor: Abad, Scott Mark, Webster Grove, MA 63119 (US); Goldman, Barry, S., St. Louis, MO 63124 (US); Cotter, Jason, Raleigh, NC 27616 (US); Ferguson, Angie, Morrisville, NC 27560 (US); Hartsuyker, Kindle Karen, Kirkwood, MO 63122 (US); James, Todd, F., Otterville, Ontario N0J 1RO (CA); Karunanandaa, Balasulojini, Creve Coeur, MO 63141 (US); D'Ordine, Robert, Ballwin, MO 63011 (US); Norris, Susan, University City, MO 63130 (US); Zhou, Li, Belmont, MA 02478 (US); Stein, Joshua, Acton, MA 01720 (US); Rounsley, Steven, D., Melrose, MA 02176 (US)
(74) Representative: Helbing, Jörg

(57) **Abstract**

Transgenic seed for crops with improved traits are provided by trait-improving recombinant DNA where plants grown from such transgenic seed exhibit one or more improved traits as compared to a control plant. Of particular interest are transgenic plants that have increased yield. The present invention also provides recombinant DNA molecules for expression of a protein, and recombinant DNA molecules for expression of mRNA complementary to at least a portion of an mRNA native to the target plant for use in gene suppression to suppress the expression of a protein.

## Description

### CROSS REFERENCE TO RELATED APPLICATIONS

This application claims benefit under 35USC § 119(e) of United States provisional application Serial No. 60/556,841 filed 03/25/2004, herein incorporated by reference

### INCOPORATION OF SEQUENCE LISTING

Two copies of the sequence listing (Copy 1 and Copy 2) and a computer readable form (CRF) of the sequence listing, all on CD-ROMs, each containing the file named pa_01123.rpt, which is 33,475 kilo bytes (measured in MS-WINDOWS) and was created on March 18, 2005, are herein incorporated by reference.

### FIELD OF THE INVENTION

Disclosed herein are inventions in the field of plant genetics and developmental biology. More specifically, the present invention provides transgenic seeds for crops, wherein the genome of said seed comprises recombinant DNA, the expression of which results in the production of transgenic plants that have improved trait(s).

### BACKGROUND OF THE INVENTION

Transgenic plants with improved traits such as improved yield, environmental stress tolerance, pest resistance, herbicide tolerance, modified seed compositions, and the like are desired by both farmers and consumers. Although considerable efforts in plant breeding have provided significant gains in desired traits, the ability to introduce specific DNA into plant genomes provides further opportunities for generation of plants with improved and/or unique traits. The ability to develop transgenic plants with improved traits depends in part on the identification of genes that are useful in recombinant DNA constructs for production of transformed plants with improved properties.

### SUMMARY OF THE INVENTION

This invention provides transgenic seeds, transgenic plants and DNA constructs with trait-improving recombinant DNA from a gene or homolog which has been demonstrated for trait improvement in a model plant. More specifically, such recombinant DNA is from a gene identified in a model plant screen as disclosed herein or homologues of such gene, e.g. from related species or in some cases from a broad range of unrelated species. In particular aspects of the invention the recombinant DNA will express a protein having an amino acid sequence with at least 90% identity to a consensus amino acid sequence in the group consisting of the consensus amino acid sequence of SEQ ID NO:240 and its homologs through SEQ ID NO:478 and its homologs, but excluding SEQ ID NO:391 and its homologs. The amino acid sequences of homologs are SEQ ID NO: 479 through SEQ ID NO: 12463. Tables 2 identifying the sequences of homologs for proteins encoded by the trait-improving genes described *supra* is provided herein as appendix. In some cases of trait improvement, the recombinant DNA encodes a protein; in other cases, the recombinant DNA suppresses endogenous protein expression. In a broad aspect this invention provides transgenic seed for growing crop plants with improved traits, such crop plants with improved traits and the plant parts including transgenic seed produced by such crop plants. The improved trait provided by the recombinant DNA in the transgenic crop plant of this invention is identified by comparison to a control plant, i.e. a plant without the trait-improving recombinant DNA. In one aspect of the invention, transgenic crop plant grown from the transgenic seed has improved yield, as compared to the yield of a control plant, e.g. a plant without the recombinant DNA that produces the increased yield. Increased yield may be characterized as plant yield increase under non-stress conditions, or by plant yield increase under one or more environmental stress conditions including, but not limited to, water deficit stress, cold stress, heat stress, high salinity stress, shade stress, and low nitrogen availability stress. Still another aspect of the present invention also provides transgenic plants having other improved phenotypes, such as improved plant development, plant morphology, plant physiology or seed composition as compared to a corresponding trait of a control plant. The various aspects of this invention are especially useful for transgenic seed and transgenic plants having improved traits in corn (also know as maize), soybean, cotton, canola (rape), wheat, sunflower, sorghum, alfalfa, barley, millet, rice, tobacco, fruit and vegetable crops, and turfgrass.

The invention also comprises recombinant DNA constructs. In one aspect such recombinant DNA constructs useful for the transgenic seed and transgenic plants of this invention comprise a promoter functional in a plant cell operably linked to a DNA segment for expressing a protein associated with a trait in a model plant or a homologue. In another aspect the recombinant DNA constructs useful for the transgenic seed and transgenic plants of this invention comprise a promoter functional in a plant cell operably linked to a DNA segment for suppressing the level of an endogenous plant protein which is a homologue to a model-plant protein, the suppression of which is associated with an improved trait. Suppression can be effected by any of a variety of methods known in the art, e.g. post transcriptional suppression by anti-sense, sense, dsRNA and the like or by transcriptional suppression.

This invention also provides a method of producing a transgenic crop plant having at least one improved trait, wherein the method comprises providing to a grower of transgenic seeds comprising recombinant DNA for expression or suppression of a trait-improving gene provided herein, and growing transgenic plant from said transgenic seed. Such methods can be used to generate transgenic crop plants having at least one improved traits under one or more environmental stress conditions including, but not limited to, water deficit stress, cold stress, heat stress, high salinity stress, shade stress, and low nitrogen availability stress. In another aspect, such method also can be used to generate transgenic crop plants having improved plant development, plant morphology, plant physiology or seed component phenotype as compared to a corresponding phenotype of a control plant. Of particular interest are uses of such methods to generate transgenic crop plants having increased yield under non-stress condition, or under one or more stress conditions.

One a particular embodiment of this invention provides transgenic seeds comprising trait improving recombinant DNA in its genome for the expression of a bacterial phytochrome protein. Transgenic plants resulting from such invention have improved tolerance to water deficit stress, cold stress and low nitrogen availability stress. In another aspect, transgenic crop plants overexpressing the bacterial phytochrome protein have increased yield under non-stress condition, or under one or more stress conditions.

### DETAILED DESCRIPTION OF THE INVENTION

The present invention is directed to transgenic plant seed, wherein the genome of said transgenic plant seed comprises a trait-improving recombinant DNA as provided herein, and transgenic plant grown from such seed possesses an improved trait as compared to the trait of a control plant. In one aspect, the present invention relates to transgenic plants wherein the improved trait is one or more traits including improved drought stress tolerance, improved heat stress tolerance, improved cold stress tolerance, improved high salinity stress tolerance, improved low nitrogen availability stress tolerance, improved shade stress tolerance, improved plant growth and development at the stages of seed imbibition through early vegetative phase, and improved plant growth and development at the stages of leaf development, flower production and seed maturity. Of particular interest are the transgenic plants grown from transgenic seeds provided herein wherein the improved trait is increased seed yield. Recombinant DNA constructs disclosed by the present invention comprise recombinant polynucleotides providing for the production of mRNA to modulate gene expression, imparting improved traits to plants.

As used herein, "gene" refers to chromosomal DNA, plasmid DNA, cDNA, synthetic DNA, or other DNA that encodes a peptide, polypeptide, protein, or RNA molecule, and regions flanking the coding sequences involved in the regulation of expression.

As used herein, "transgenic seed" refers to a plant seed whose genome has been altered by the incorporation of recombinant DNA, e.g. by transformation as described herein. The term "transgenic plant" is used to refer to the plant produced from an original transformation event, or progeny from later generations or crosses of a plant to a transformed plant, so long as the progeny contains the recombinant DNA in its genome. As used herein, "recombinant DNA" refers to a polynucleotide having a genetically engineered modification introduced through combination of endogenous and/or exogenous elements in a transcription unit, manipulation via mutagenesis, restriction enzymes, and the like or simply by inserting multiple copies of a native transcription unit.. Recombinant DNA may comprise DNA segments obtained from different sources, or DNA segments obtained from the same source, but which have been manipulated to join DNA segments which do not naturally exist in the joined form. A recombinant polynucleotide may exist outside of the cell, for example as a PCR fragment, or integrated into a genome, such as a plant genome.

As used herein, "trait" refers to a physiological, morphological, biochemical, or physical characteristic of a plant or particular plant material or cell. In some instances, this characteristic is visible to the human eye, such as seed or plant size, or can be measured by biochemical techniques, such as detecting the protein, starch, or oil content of seed or leaves, or by observation of a metabolic or physiological process, e.g. by measuring uptake of carbon dioxide, or by the observation of the expression level of a gene or genes, e.g., by employing Northern analysis, RT-PCR, microarray gene expression assays, or reporter gene expression systems, or by agricultural observations such as stress tolerance, yield, or pathogen tolerance.

As used herein, "control plant" is a plant without trait-improving recombinant DNA. A control plant is used to measure and compare trait improvement in a transgenic plant with such trait-improving recombinant DNA. A suitable control plant may be a non-transgenic plant of the parental line used to generate a transgenic plant herein. Alternatively, control plant may be a transgenic plant that comprises an empty vector or marker gene, but does not contain the recombinant DNA that produces the trait improvement. A control plant may also be a negative segregant progeny of hemizygous transgenic plant. In certain demonstrations of trait improvement, the use of a limited number of control plants can cause a wide variation in the control dataset. To minimize the effect of the variation within the control dataset, a "reference" is used. As use herein a "reference" is a trimmed mean of all data from both transgenic and control plants grown under the same conditions and at the same developmental stage. The trimmed mean is calculated by eliminating a specific percentage, i.e. 20%, of the smallest and largest observation from the data set and then calculating the average of the remaining observation..

As used herein, "trait improvement" refers to a detectable and desirable difference in a characteristic in a transgenic plant relative to a control plant or a reference. In some cases, the trait improvement can be measured quantitatively. For example, the trait improvement can entail at least a 2% desirable difference in an observed trait, at least a 5% desirable difference, at least about a 10% desirable difference, at least about a 20% desirable difference, at least about a 30% desirable difference, at least about a 50% desirable difference, at least about a 70% desirable difference, or at least about a 100% difference, or an even greater desirable difference. In other cases, the trait improvement is only measured qualitatively. It is known that there can be a natural variation in a trait. Therefore, the trait improvement observed entails a change of the normal distribution of the trait in the transgenic plant compared with the trait distribution observed in a control plant or a reference, which is evaluated by statistical methods provided herein. Trait improvement includes, but not limited to, yield increase, including increased yield under non-stress conditions and increased yield under environmental stress conditions. Stress conditions may include, for example, drought, shade, fungal disease, viral disease, bacterial disease, insect infestation, nematode infestation, cold temperature exposure, heat exposure, osmotic stress, reduced nitrogen nutrient availability, reduced phosphorus nutrient availability and high plant density. Many agronomic traits can affect "yield", including without limitation, plant height, pod number, pod position on the plant, number of internodes, incidence of pod shatter, grain size, efficiency of nodulation and nitrogen fixation, efficiency of nutrient assimilation, resistance to biotic and abiotic stress, carbon assimilation, plant architecture, resistance to lodging, percent seed germination, seedling vigor, and juvenile traits. Other traits that can affect yield include, efficiency of germination (including germination in stressed conditions), growth rate (including growth rate in stressed conditions), ear number, seed number per ear, seed size, composition of seed (starch, oil, protein) and characteristics of seed fill. Also of interest is the generation of transgenic plants that demonstrate desirable phenotypic properties that may or may not confer an increase in overall plant yield. Such properties include improved plant morphology, plant physiology or improved components of the mature seed harvested from the transgenic plant.

As used herein, "yield-limiting environment" refers to the condition under which a plant would have the limitation on yield including environmental stress conditions.

As used herein, "stress condition" refers to the condition unfavorable for a plant, which adversely affect plant metabolism, growth and/or development. A plant under the stress condition typically shows reduced germination rate, retarded growth and development, reduced photosynthesis rate, and eventually leading to reduction in yield. Specifically, "water deficit stress" used herein preferably refers to the sub-optimal conditions for water and humidity needed for normal growth of natural plants. Relative water content (RWC) can be used as a physiological measure of plant water deficit. It measures the effect of osmotic adjustment in plant water status, when a plant is under stressed conditions. Conditions which may result in water deficit stress include heat, drought, high salinity and PEG induced osmotic stress.

"Cold stress" used herein preferably refers to the exposure of a plant to a temperatures below (two or more degrees Celsius below) those normal for a particular species or particular strain of plant.

As used herein, "sufficient nitrogen growth condition" refers to the growth condition where the soil or growth medium contains or receives enough amounts of nitrogen nutrient to sustain a healthy plant growth and/or for a plant to reach its typical yield for a particular plant species or a particular strain. As used herein, "nitrogen nutrient" means any one or any mix of the nitrate salts commonly used as plant nitrogen fertilizer, including, but not limited to, potassium nitrate, calcium nitrate, sodium nitrate, ammonium nitrate. The term ammonium as used herein means any one or any mix of the ammonium salts commonly used as plant nitrogen fertilizer, e.g. ammonium nitrate, ammonium chloride, ammonium sulfate, etc. One skilled in the art would recognize what constitute such soil, media and fertilizer inputs for most plant species. "Low nitrogen availability stress" used herein refers to a plant growth condition that does not contain sufficient nitrogen nutrient to maintain a healthy plant growth and/or for a plant to reach its typical yield under a sufficient nitrogen growth condition, and preferably refers to a growth condition with 50% or less of the conventional nitrogen inputs.

"Shade stress" used herein preferably refers to limited light availability that triggers the shade avoidance response in plant. Plants are subject to shade stress when localized at lower part of the canopy, or in close proximity of neighboring vegetation. Shade stress may become exacerbated when the planting density exceeds the average prevailing density for a particular plant species. The average prevailing densities per acre of a few other examples of crop plants in the USA in the year 2000 were: wheat 1,000,000-1,500,000; rice 650,000-900,000; soybean 150,000-200,000, canola 260,000-350,000, sunflower 17,000-23,000 and cotton 28,000-55,000 plants per acre (Cheikh, et al., (2003) U.S. Patent Application No. .20030101479).

As used herein, "increased yield" of a transgenic plant of the present invention may be evidenced and measured in a number of ways, including test weight, seed number per plant, seed weight, seed number per unit area (i.e. seeds, or weight of seeds, per acre), bushels per acre, tons per acre, tons per acre, kilo per hectare. For example, maize yield may be measured as production of shelled corn kernels per unit of production area, e.g. in bushels per acre or metric tons per hectare, often reported on a moisture adjusted basis, e.g. at 15.5 % moisture. Increased yield may result from improved utilization of key biochemical compounds, such as nitrogen, phosphorous and carbohydrate, or from improved responses to environmental stresses, such as cold, heat, drought, salt, and attack by pests or pathogens. Trait-improving recombinant DNA may also be used to provide transgenic plants having improved growth and development, and ultimately increased yield, as the result of modified expression of plant growth regulators or modification of cell cycle or photosynthesis pathways.

As used herein, "expression" refers to transcription of DNA to produce RNA. The resulting RNA may be without limitation mRNA encoding a protein, antisense RNA that is complementary to an mRNA encoding a protein, or an RNA transcript comprising a combination of sense and antisense gene regions, such as for use in RNAi technology. Expression as used herein may also refer to production of encoded protein from mRNA.

As used herein, "promoter" includes reference to a region of DNA upstream from the start of transcription and involved in recognition and binding of RNA polymerase and other proteins to initiate transcription. A "plant promoter" is a promoter capable of initiating transcription in plant cells whether or not its origin is a plant cell. Exemplary plant promoters include, but are not limited to, those that are obtained from plants, plant viruses, and bacteria which comprise genes expressed in plant cells such Agrobacterium or Rhizobium. Examples of promoters under developmental control include promoters that preferentially initiate transcription in certain tissues, such as leaves, roots, or seeds. Such promoters are referred to as "tissue preferred". Promoters which initiate transcription only in certain tissues are referred to as "tissue specific". A "cell type" specific promoter primarily drives expression in certain cell types in one or more organs, for example, vascular cells in roots or leaves. An "inducible" or "repressible" promoter is a promoter which is under environmental control. Examples of environmental conditions that may effect transcription by inducible promoters include anaerobic conditions, or certain chemicals, or the presence of light. Tissue specific, tissue preferred, cell type specific, and inducible promoters constitute the class of "non-constitutive" promoters. A "constitutive" promoter is a promoter which is active under most conditions. As used herein, "antisense orientation" includes reference to a polynucleotide sequence that is operably linked to a promoter in an orientation where the antisense strand is transcribed. The antisense strand is sufficiently complementary to an endogenous transcription product such that translation of the endogenous transcription product is often inhibited.
As used herein, "operably linked" refers to the association of two or more nucleic acid fragments on a single nucleic acid fragment so that the function of one is affected by the other. For example, a promoter is operably linked with a coding sequence when it is capable of affecting the expression of that coding sequence (i.e., that the coding sequence is under the transcriptional control of the promoter). Coding sequences can be operably linked to regulatory sequences in sense or antisense orientation.

As used herein, "consensus sequence" refers to an artificial, amino acid sequence of conserved parts of the proteins encoded by homologous genes, e.g. as determined by a CLUSTALW alignment of amino acid sequence of homolog proteins.

As used herein, "homolog" refers to a gene related to a second gene by descent from a common ancestral DNA sequence. The term, homolog, may apply to the relationship between genes separated by the event of speciation (see ortholog) or to the relationship between genes separated by the event of genetic duplication (see paralog). Homologs can be from the same or a different organism that performs the same biological function. "Orthologs" refer to a set of homologous genes in different species that evolved from a common ancestral gene by specification. Normally, orthologs retain the same function in the course of evolution; and "paralogs" refer to a set of homologous genes in the same species that have diverged from each other as a consequence of genetic duplication.

Percent identity refers to the extent to which two optimally aligned DNA or protein segments are invariant throughout a window of alignment of components, e.g. nucleotide sequence or amino acid sequence. An "identity fraction" for aligned segments of a test sequence and a reference sequence is the number of identical components which are shared by sequences of the two aligned segments divided by the total number of sequence components in the reference segment over a window of alignment which is the smaller of the full test sequence or the full reference sequence. "Percent identity" ("% identity") is the identity fraction times 100. "% identity to a consensus amino acid sequence" is 100 times the identity fraction in a window of alignment of an amino acid sequence of a test protein optimally aligned to consensus amino acid sequence of this invention.

As used herein *"Arabidopsis"* means plants of *Arabidopsis thaliana.*

### Recombinant DNA constructs

The present invention provides recombinant DNA constructs comprising one or more polynucleotides disclosed herein for imparting one or more improved traits to transgenic plant. Such constructs also typically comprise a promoter operatively linked to said polynucleotide to provide for expression in a target plant. Other construct components may include additional regulatory elements, such as 5' or 3' untranslated regions (such as polyadenylation sites), intron regions, and transit or signal peptides. Such recombinant DNA constructs can be assembled using methods known to those of ordinary skill in the art.

In a preferred embodiment, a polynucleotide of the present invention is operatively linked in a recombinant DNA construct to a promoter functional in a plant to provide for expression of the polynucleotide in the sense orientation such that a desired polypeptide is produced. Also provided are embodiments wherein a polynucleotide is operatively linked to a promoter functional in a plant to provide for expression of the polynucleotide in the antisense orientation such that a complementary copy of at least a portion of an mRNA native to the target plant host is produced.

Recombinant constructs prepared in accordance with the present invention may also generally include a 3' untranslated DNA region (UTR) that typically contains a polyadenylation sequence following the polynucleotide coding region. Examples of useful 3' UTRs include those from the nopaline synthase gene of *Agrobacterium tumefaciens* (*nos*), a gene encoding the small subunit of a ribulose-1,5-bisphosphate carboxylase-oxygenase (rbcS), and the T7 transcript of *Agrobacterium tumefaciens.*
Constructs and vectors may also include a transit peptide for targeting of a gene target to a plant organelle, particularly to a chloroplast, leucoplast or other plastid organelle. For descriptions of the use of chloroplast transit peptides, see U.S. Patent 5, 188,642 and U.S. Patent No. 5,728,925, incorporated herein by reference.

Table1 provides a list of genes that can provide recombinant DNA that was used in a model plant to discover associate improved traits and that can be used with homologs to define a consensus amino acid sequence for characterizing recombinant DNA in the transgenic seeds, transgenic plants, DNA constructs and methods of this invention. "NUC SEQ ID NO" refers to a SEQ ID NO. for particular DNA sequence in the Sequence Listing.
"PEP SEQ ID NO" refers to a SEQ ID NO. in the Sequence Listing for the amino acid sequence of a protein cognate to a particular DNA "construct_id" refers to an arbitrary number used to identify a particular recombinant DNA construct comprising the particular DNA.
"gene" refers to an arbitrary name used to identify the particular DNA.
"orientation" refers to the orientation of the particular DNA in a recombinant DNA construct relative to the promoter.
"species" refers to the organism from which the particular DNA was derived.

**Table 1**

| **Nuc SEQ ID** | **Pep SEQ ID** | **construct_id** | **Gene** | **orientation** | **Species** |
|---|---|---|---|---|---|
| 1 | 240 | 19867 | CGPG4046 | Sense | Glycine max |
| 2 | 241 | 74518 | CGPG6792 | Sense | Pseudomonas fluorescens PfO-1 |
| 3 | 242 | 15816 | CGPG2244 | Sense | Arabidopsis thaliana |
| 4 | 243 | 17918 | CGPG2774 | Sense | Arabidopsis thaliana |
| 5 | 244 | 15306 | CGPG1909 | AntiSense | Arabidopsis thaliana |
| 6 | 245 | 12038 | CGPG1087 | Sense | Arabidopsis thaliana |
| 7 | 246 | 12046 | CGPG1106 | Sense | Arabidopsis thaliana |
| 8 | 247 | 13432 | CGPG1525 | Sense | Arabidopsis thaliana |
| 9 | 248 | 13711 | CGPG1114 | Sense | Arabidopsis thaliana |
| 10 | 249 | 14809 | CGPG692 | Sense | Arabidopsis thaliana |
| 11 | 250 | 14951 | CGPG1636 | Sense | Arabidopsis thaliana |
| 12 | 251 | 15632 | CGPG1469 | Sense | Arabidopsis thaliana |
| 13 | 252 | 16147 | CGPG2088 | Sense | Arabidopsis thaliana |
| 14 | 253 | 16158 | CGPG2169 | Sense | Arabidopsis thaliana |
| 15 | 254 | 16170 | CGPG2192 | Sense | Arabidopsis thaliana |
| 16 | 255 | 16171 | CGPG2194 | Sense | Arabidopsis thaliana |
| 17 | 256 | 16175 | CGPG2204 | Sense | Arabidopsis thaliana |
| 18 | 257 | 17430 | CGPG2478 | Sense | Arabidopsis thaliana |
| 19 | 258 | 17819 | CGPG2587 | Sense | Arabidopsis thaliana |
| 20 | 259 | 17921 | CGPG2878 | Sense | Arabidopsis thaliana |
| 21 | 260 | 17928 | CGPG2739 | Sense | Arabidopsis thaliana |
| 22 | 261 | 18637 | CGPG3450 | Sense | Arabidopsis thaliana |
| 23 | 262 | 18816 | CGPG2406 | Sense | Arabidopsis thaliana |
| 24 | 263 | 19227 | CGPG3025 | Sense | Arabidopsis thaliana |
| 25 | 264 | 19429 | CGPG3486 | Sense | Arabidopsis thaliana |
| 26 | 265 | 70235 | CGPG96 | Sense | Arabidopsis thaliana |
| 27 | 266 | 72634 | CGPG4855 | Sense | Arabidopsis thaliana |
| 28 | 267 | 72752 | CGPG5532 | Sense | Saccharomyces cerevisiae |
| 29 | 268 | 12007 | CGPG1089 | AntiSense | Arabidopsis thaliana |
| 30 | 269 | 12290 | CGPG977 | AntiSense | Arabidopsis thaliana |
| 31 | 270 | 12343 | CGPG581 | AntiSense | Arabidopsis thaliana |
| 32 | 271 | 14348 | CGPG1692 | AntiSense | Arabidopsis thaliana |
| 33 | 272 | 15708 | CGPG2167 | AntiSense | Arabidopsis thaliana |
| 34 | 273 | 17615 | CGPG2458 | Anti-Sense | Arabidopsis thaliana |
| 35 | 274 | 17622 | CGPG2454 | Anti-Sense | Arabidopsis thaliana |
| 36 | 275 | 70714 | CGPG1480 | Anti-sense | Arabidopsis thaliana |
| 37 | 276 | 17925 | CGPG2883 | Sense | Arabidopsis thaliana |
| 38 | 277 | 18541 | CGPG2971 | Sense | Arabidopsis thaliana |
| 39 | 278 | 11425 | CGPG628 | Sense | Arabidopsis thaliana |
| 40 | 279 | 12263 | CGPG799 | Sense | Arabidopsis thaliana |
| 41 | 280 | 12288 | CGPG811 | Sense | Arabidopsis thaliana |
| 42 | 281 | 12910 | CGPG985 | Sense | Arabidopsis thaliana |
| 43 | 282 | 14335 | CGPG1685 | Sense | Arabidopsis thaliana |
| 44 | 283 | 17427 | CGPG2475 | Sense | Arabidopsis thaliana |
| 45 | 284 | 19140 | CGPG1758 | Sense | Arabidopsis thaliana |
| 46 | 285 | 19179 | CGPG740 | Sense | Arabidopsis thaliana |
| 47 | 286 | 19251 | CGPG3118 | Sense | Arabidopsis thaliana |
| 48 | 287 | 19443 | CGPG2834 | Sense | Arabidopsis thaliana |
| 49 | 288 | 19607 | CGPG3397 | Sense | Arabidopsis thaliana |
| 50 | 289 | 19915 | CGPG4072 | Sense | Glycine max |
| 51 | 290 | 70222 | CGPG28 | Sense | Arabidopsis thaliana |
| 52 | 291 | 70464 | CGPG3773 | Sense | Arabidopsis thaliana |
| 53 | 292 | 70474 | CGPG3806 | Sense | Arabidopsis thaliana |
| 54 | 293 | 70484 | CGPG3853 | Sense | Arabidopsis thaliana |
| 55 | 294 | 72474 | CGPG4667 | Sense | Glycine max |
| 56 | 295 | 13047 | CGPG1324 | ANTI-SENSE | Arabidopsis thaliana |
| 57 | 296 | 13304 | CGPG1282 | ANTI-SENSE | Arabidopsis thaliana |
| 58 | 297 | 13474 | CGPG1600 | ANTI-SENSE | Arabidopsis thaliana |
| 59 | 298 | 19252 | CGPG3121 | SENSE | Arabidopsis thaliana |
| 60 | 299 | 12612 | CGPG1181 | SENSE | Arabidopsis thaliana |
| 61 | 300 | 12926 | CGPG1299 | SENSE | Arabidopsis thaliana |
| 62 | 301 | 13230 | CGPG1276 | SENSE | Arabidopsis thaliana |
| 63 | 302 | 14235 | CGPG1665 | SENSE | Arabidopsis thaliana |
| 64 | 303 | 17305 | CGPG2261 | SENSE | Arabidopsis thaliana |
| 65 | 304 | 17470 | CGPG2606 | SENSE | Arabidopsis thaliana |
| 66 | 305 | 17718 | CGPG1791 | SENSE | Arabidopsis thaliana |
| 67 | 306 | 17904 | CGPG1912 | SENSE | Arabidopsis thaliana |
| 68 | 307 | 18280 | CGPG3547 | SENSE | Arabidopsis thaliana |
| 69 | 308 | 18287 | CGPG3563 | SENSE | Arabidopsis thaliana |
| 70 | 309 | 18501 | CGPG2237 | SENSE | Arabidopsis thaliana |
| 71 | 310 | 18877 | CGPG3097 | SENSE | Arabidopsis thaliana |
| 72 | 311 | 19531 | CGPG3028 | SENSE | Arabidopsis thaliana |
| 73 | 312 | 70405 | CGPG1672 | SENSE | Arabidopsis thaliana |
| 74 | 313 | 72136 | CGPG5320 | SENSE | Glycine max |
| 75 | 314 | 72611 | CGPG4812 | SENSE | Arabidopsis thaliana |
| 76 | 315 | 12627 | CGPG1003 | SENSE | Arabidopsis thaliana |
| 77 | 316 | 12813 | CGPG825 | SENSE | Arabidopsis thaliana |
| 78 | 317 | 14945 | CGPG1776 | SENSE | Arabidopsis thaliana |
| 79 | 318 | 15345 | CGPG1504 | SENSE | Arabidopsis thaliana |
| 80 | 319 | 15348 | CGPG1514 | SENSE | Arabidopsis thaliana |
| 81 | 320 | 16325 | CGPG2195 | SENSE | Arabidopsis thaliana |
| 82 | 321 | 16702 | CGPG531 | SENSE | Arabidopsis thaliana |
| 83 | 322 | 16836 | CGPG2283 | SENSE | Arabidopsis thaliana |
| 84 | 323 | 17002 | CGPG1926 | SENSE | Arabidopsis thaliana |
| 85 | 324 | 17012 | CGPG2073 | SENSE | Arabidopsis thaliana |
| 86 | 325 | 17017 | CGPG1722 | SENSE | Arabidopsis thaliana |
| 87 | 326 | 17344 | CGPG2404 | SENSE | Arabidopsis thaliana |
| 88 | 327 | 17426 | CGPG2474 | SENSE | Arabidopsis thaliana |
| 89 | 328 | 17655 | CGPG2899 | SENSE | Arabidopsis thaliana |
| 90 | 329 | 17656 | CGPG2714 | SENSE | Arabidopsis thaliana |
| 91 | 330 | 17906 | CGPG2145 | SENSE | Arabidopsis thaliana |
| 92 | 331 | 18278 | CGPG3544 | SENSE | Arabidopsis thaliana |
| 93 | 332 | 18822 | CGPG2398 | SENSE | Arabidopsis thaliana |
| 94 | 333 | 18881 | CGPG3126 | SENSE | Arabidopsis thaliana |
| 95 | 334 | 19213 | CGPG3622 | SENSE | Arabidopsis thaliana |
| 96 | 335 | 19239 | CGPG3197 | SENSE | Arabidopsis thaliana |
| 97 | 336 | 19247 | CGPG3112 | SENSE | Arabidopsis thaliana |
| 98 | 337 | 19460 | CGPG2824 | SENSE | Arabidopsis thaliana |
| 99 | 338 | 19512 | CGPG2898 | SENSE | Arabidopsis thaliana |
| 100 | 339 | 19533 | CGPG3032 | SENSE | Arabidopsis thaliana |
| 101 | 340 | 19603 | CGPG3385 | SENSE | Arabidopsis thaliana |
| 102 | 341 | 72126 | CGPG5310 | SENSE | Glycine max |
| 103 | 342 | 72437 | CGPG5068 | SENSE | Arabidopsis thaliana |
| 104 | 343 | 72441 | CGPG5079 | SENSE | Arabidopsis thaliana |
| 105 | 344 | 72639 | CGPG4861 | SENSE | Arabidopsis thaliana |
| 106 | 345 | 14825 | CGPG1883 | Anti-Sense | Arabidopsis thaliana |
| 107 | 346 | 17931 | CGPG2890 | Sense | Arabidopsis thaliana |
| 108 | 347 | 18854 | CGPG3524 | Sense | Arabidopsis thaliana |
| 109 | 348 | 12237 | CGPG1206 | Sense | Arabidopsis thaliana |
| 110 | 349 | 13414 | CGPG1246 | Sense | Arabidopsis thaliana |
| 111 | 350 | 16160 | CGPG2172 | Sense | Arabidopsis thaliana |
| 112 | 351 | 16226 | CGPG1980 | Sense | Arabidopsis thaliana |
| 113 | 352 | 16803 | CGPG2179 | Sense | Arabidopsis thaliana |
| 114 | 353 | 18260 | CGPG3373 | Sense | Arabidopsis thaliana |
| 115 | 354 | 18642 | CGPG3230 | Sense | Arabidopsis thaliana |
| 116 | 355 | 18721 | CGPG3618 | Sense | Arabidopsis thaliana |
| 117 | 356 | 19254 | CGPG3123 | Sense | Arabidopsis thaliana |
| 118 | 357 | 70247 | CGPG34 | Sense | Arabidopsis thaliana |
| 119 | 358 | 70650 | CGPG4337 | Sense | Arabidopsis thaliana |
| 120 | 359 | 11787 | CGPG951 | ANTI-SENSE | Arabidopsis thaliana |
| 120 | 359 | 12635 | CGPG951 | Sense | Arabidopsis thaliana |
| 121 | 360 | 13641 | CGPG1211 | ANTI-SENSE | Arabidopsis thaliana |
| 122 | 361 | 14515 | CGPG1115 | ANTI-SENSE | Arabidopsis thaliana |
| 123 | 362 | 14920 | CGPG2027 | ANTI-SENSE | Arabidopsis thaliana |
| 124 | 363 | 15204 | CGPG2000 | ANTI-SENSE | Arabidopsis thaliana |
| 125 | 364 | 15216 | CGPG1906 | ANTI-SENSE | Arabidopsis thaliana |
| 125 | 364 | 19058 | CGPG1906 | SENSE | Arabidopsis thaliana |
| 126 | 365 | 15330 | CGPG1237 | ANTI-SENSE | Arabidopsis thaliana |
| 127 | 366 | 19610 | CGPG3419 | SENSE | Arabidopsis thaliana |
| 128 | 367 | 14338 | CGPG1706 | SENSE | Arabidopsis thaliana |
| 129 | 368 | 17809 | CGPG2436 | SENSE | Arabidopsis thaliana |
| 130 | 369 | 72471 | CGPG4648 | SENSE | Glycine max |
| 131 | 370 | 16403 | CGPG1983 | SENSE | Arabidopsis thaliana |
| 132 | 371 | 17737 | CGPG2623 | SENSE | Arabidopsis thaliana |
| 133 | 372 | 18395 | CGPG2994 | SENSE | Arabidopsis thaliana |
| 134 | 373 | 72772 | CGPG2418 | SENSE | Arabidopsis thaliana |
| 135 | 374 | 19441 | CGPG2783 | SENSE | Arabidopsis thaliana |
| 136 | 375 | 11409 | CGPG136 | SENSE | Arabidopsis thaliana |
| 137 | 376 | 10486 | CGPG137 | SENSE | Arabidopsis thaliana |
| 138 | 377 | 12104 | CGPG693 | SENSE | Arabidopsis thaliana |
| 139 | 378 | 12258 | CGPG836 | SENSE | Arabidopsis thaliana |
| 140 | 379 | 12909 | CGPG1195 | SENSE | Arabidopsis thaliana |
| 141 | 380 | 14310 | CGPG1037 | SENSE | Arabidopsis thaliana |
| 142 | 381 | 14317 | CGPG1150 | SENSE | Arabidopsis thaliana |
| 143 | 382 | 14709 | CGPG990 | SENSE | Arabidopsis thaliana |
| 144 | 383 | 15123 | CGPG1730 | SENSE | Arabidopsis thaliana |
| 145 | 384 | 16013 | CGPG978 | SENSE | Arabidopsis thaliana |
| 146 | 385 | 16185 | CGPG2025 | SENSE | Arabidopsis thaliana |
| 147 | 386 | 16719 | CGPG1817 | SENSE | Arabidopsis thaliana |
| 148 | 387 | 17490 | CGPG2638 | SENSE | Arabidopsis thaliana |
| 149 | 388 | 17905 | CGPG21 01 | SENSE | Arabidopsis thaliana |
| 150 | 389 | 18385 | CGPG3609 | SENSE | Arabidopsis thaliana |
| 151 | 390 | 18392 | CGPG2989 | SENSE | Arabidopsis thaliana |
| 153 | 392 | 18531 | CGPG3215 | SENSE | Arabidopsis thaliana |
| 154 | 393 | 18603 | CGPG3423 | SENSE | Arabidopsis thaliana |
| 155 | 394 | 19530 | CGPG3026 | SENSE | Arabidopsis thaliana |
| 156 | 395 | 70202 | CGPG3949 | SENSE | Glycine max |
| 157 | 396 | 72009 | CGPG5273 | SENSE | Saccharomyces cerevisiae |
| 158 | 397 | 72119 | CGPG5332 | SENSE | Glycine max |
| 159 | 398 | 10188 | CGPG147 | Anti-sense | Arabidopsis thaliana |
| 160 | 399 | 10404 | CGPG25 | Anti-Sense | Arabidopsis thaliana |
| 161 | 400 | 11333 | CGPG583 | Anti-Sense | Arabidopsis thaliana |
| 162 | 401 | 11719 | CGPG710 | Anti-Sense | Arabidopsis thaliana |
| 163 | 402 | 13663 | CGPG1241 | Anti-sense | Arabidopsis thaliana |
| 164 | 403 | 13958 | CGPG1711 | Anti-Sense | Arabidopsis thaliana |
| 165 | 404 | 15214 | CGPG1904 | Anti-Sense | Arabidopsis thaliana |
| 166 | 405 | 10483 | CGPG447 | Sense | Arabidopsis thaliana |
| 167 | 406 | 11711 | CGPG466 | Sense | Arabidopsis thaliana |
| 168 | 407 | 11909 | CGPG471 | Sense | Arabidopsis thaliana |
| 169 | 408 | 12216 | CGPG1091 | Sense | Arabidopsis thaliana |
| 170 | 409 | 12236 | CGPG1193 | Sense | Arabidopsis thaliana |
| 171 | 410 | 12256 | CGPG824 | Sense | Arabidopsis thaliana |
| 172 | 411 | 12806 | CGPG714 | Sense | Arabidopsis thaliana |
| 173 | 412 | 12904 | CGPG204 | Sense | Arabidopsis thaliana |
| 174 | 413 | 13212 | CGPG1384 | Sense | Arabidopsis thaliana |
| 175 | 414 | 13232 | CGPG1281 | Sense | Arabidopsis thaliana |
| 176 | 415 | 13912 | CGPG1283 | Sense | Arabidopsis thaliana |
| 177 | 416 | 14327 | CGPG1606 | Sense | Arabidopsis thaliana |
| 178 | 417 | 14704 | CGPG1066 | Sense | Arabidopsis thaliana |
| 179 | 418 | 14714 | CGPG1431 | Sense | Arabidopsis thaliana |
| 180 | 419 | 15142 | CGPG1917 | Sense | Arabidopsis thaliana |
| 181 | 420 | 17450 | CGPG2684 | Sense | Arabidopsis thaliana |
| 182 | 421 | 18607 | CGPG3496 | Sense | Arabidopsis thaliana |
| 183 | 422 | 19409 | CGPG2691 | Sense | Arabidopsis thaliana |
| 184 | 423 | 19412 | CGPG2727 | Sense | Arabidopsis thaliana |
| 185 | 424 | 13005 | CGPG724 | ANTI-SENSE | Arabidopsis thaliana |
| 186 | 425 | 10203 | CGPG272 | ANTI-SENSE | Arabidopsis thaliana |
| 187 | 426 | 11327 | CGPG551 | ANTI-SENSE | Arabidopsis thaliana |
| 188 | 427 | 11814 | CGPG1041 | ANTI-SENSE | Arabidopsis thaliana |
| 188 | 427 | 12018 | CGPG1041 | SENSE | Arabidopsis thaliana |
| 189 | 428 | 13003 | CGPG673 | ANTI-SENSE | Arabidopsis thaliana |
| 190 | 429 | 13949 | CGPG1686 | ANTI-SENSE | Arabidopsis thaliana |
| 191 | 430 | 16416 | CGPG2258 | ANTI-SENSE | Arabidopsis thaliana |
| 192 | 431 | 16438 | CGPG1847 | ANTI-SENSE | Arabidopsis thaliana |
| 193 | 432 | 17124 | CGPG2432 | ANTI-SENSE | Arabidopsis thaliana |
| 194 | 433 | 19132 | CGPG1755 | ANTI-SENSE | Arabidopsis thaliana |
| 195 | 434 | 17922 | CGPG2880 | SENSE | Arabidopsis thaliana |
| 196 | 435 | 19719 | CGPG4171 | SENSE | Glycine max |
| 197 | 436 | 17336 | CGPG1732 | SENSE | Arabidopsis thaliana |
| 197 | 436 | 14274 | CGPG1732 | ANTI-SENSE | Arabidopsis thaliana |
| 198 | 437 | 17735 | CGPG2423 | SENSE | Arabidopsis thaliana |
| 199 | 438 | 19249 | CGPG3115 | SENSE | Arabidopsis thaliana |
| 200 | 439 | 18513 | CGPG3485 | SENSE | Arabidopsis thaliana |
| 201 | 440 | 11517 | CGPG224 | SENSE | Arabidopsis thaliana |
| 202 | 441 | 12363 | CGPG981 | SENSE | Arabidopsis thaliana |
| 203 | 442 | 12922 | CGPG1294 | SENSE | Arabidopsis thaliana |
| 204 | 443 | 15360 | CGPG1719 | SENSE | Arabidopsis thaliana |
| 205 | 444 | 16028 | CGPG2047 | SENSE | Arabidopsis thaliana |
| 206 | 445 | 16648 | CGPG2504 | SENSE | Agrobacterium tumefaciens |
| 207 | 446 | 16705 | CGPG1005 | SENSE | Arabidopsis thaliana |
| 208 | 447 | 16715 | CGPG2273 | SENSE | Arabidopsis thaliana |
| 209 | 448 | 17316 | CGPG2146 | SENSE | Arabidopsis thaliana |
| 210 | 449 | 17331 | CGPG1708 | SENSE | Arabidopsis thaliana |
| 211 | 450 | 17339 | CGPG2461 | SENSE | Arabidopsis thaliana |
| 212 | 451 | 17420 | CGPG2465 | SENSE | Arabidopsis thaliana |
| 213 | 452 | 17446 | CGPG2728 | SENSE | Arabidopsis thaliana |
| 214 | 453 | 17487 | CGPG2633 | SENSE | Arabidopsis thaliana |
| 215 | 454 | 17740 | CGPG2605 | SENSE | Arabidopsis thaliana |
| 216 | 455 | 17752 | CGPG2831 | SENSE | Arabidopsis thaliana |
| 217 | 456 | 18021 | CGPG685 | SENSE | Arabidopsis thaliana |
| 218 | 457 | 18245 | CGPG3343 | SENSE | Arabidopsis thaliana |
| 219 | 458 | 18617 | CGPG3521 | SENSE | Arabidopsis thaliana |
| 220 | 459 | 18734 | CGPG3198 | SENSE | Arabidopsis thaliana |
| 221 | 460 | 18823 | CGPG2830 | SENSE | Arabidopsis thaliana |
| 222 | 461 | 19222 | CGPG3017 | SENSE | Arabidopsis thaliana |
| 223 | 462 | 19430 | CGPG3487 | SENSE | Arabidopsis thaliana |
| 224 | 463 | 12332 | CGPG356 | AntiSense | Arabidopsis thaliana |
| 225 | 464 | 13649 | CGPG1544 | Anti-Sense | Arabidopsis thaliana |
| 226 | 465 | 16113 | CGPG2128 | AntiSense | Arabidopsis thaliana |
| 227 | 466 | 12069 | CGPG1188 | Sense | Arabidopsis thaliana |
| 228 | 467 | 12906 | CGPG313 | Sense | Arabidopsis thaliana |
| 229 | 468 | 13443 | CGPG1233 | Sense | Arabidopsis thaliana |
| 230 | 469 | 14707 | CGPG1141 | Sense | Arabidopsis thaliana |
| 231 | 470 | 15116 | CGPG1509 | Sense | Arabidopsis thaliana |
| 232 | 471 | 16117 | CGPG2234 | Sense | Arabidopsis thaliana |
| 233 | 472 | 16136 | CGPG2144 | Sense | Arabidopsis thaliana |
| 234 | 473 | 19077 | CGPG1808 | Sense | Arabidopsis thaliana |
| 235 | 474 | 19178 | CGPG3683 | Sense | Saccharomyces cerevisiae |
| 236 | 475 | 70752 | CGPG4465 | Sense | Arabidopsis thaliana |
| 237 | 476 | 70753 | CGPG4469 | Sense | Arabidopsis thaliana |
| 238 | 477 | 70809 | CGPG388 | Sense | Arabidopsis thaliana |
| 239 | 478 | 72091 | CGPG5264 | Sense | Saccharomyces cerevisiae |

### Recombinant DNA

Exemplary DNA for use in the present invention to improve traits in plants are provided herein as SEQ ID NO:1 through SEQ ID NO: 151 and SEQ ID NO: 153 through SEQ ID NO: 239. A subset of the exemplary DNA includes fragments of the disclosed full polynucleotides consisting of oligonucleotides of at least 15, preferably at least 16 or 17, more preferably at least 18 or 19, and even more preferably at least 20 or more, consecutive nucleotides. Such oligonucleotides are fragments of the larger molecules having a sequence selected from the group consisting of SEQ ID NO: 1 through SEQ ID NO: 151 and SEQ ID NO: 153 through SEQ ID NO: 239, and find use, for example as probes and primers for detection of the polynucleotides of the present invention.

Also of interest in the present invention are variants of the DNA provided herein. Such variants may be naturally occurring, including DNA from homologous genes from the same or a different species, or may be non-natural variants, for example DNA synthesized using chemical synthesis methods, or generated using recombinant DNA techniques. Degeneracy of the genetic code provides the possibility to substitute at least one base of the protein encoding sequence of a gene with a different base without causing the amino acid sequence of the polypeptide produced from the gene to be changed. Hence, a DNA useful in the present invention may have any base sequence that has been changed from the sequences provided herein by substitution in accordance with degeneracy of the genetic code.

Homologs of the genes providing DNA of demonstrated as useful in improving traits in model plants disclosed herein will generally demonstrate significant identity with the DNA provided herein. DNA is substantially identical to a reference DNA if, when the sequences of the polynucleotides are optimally aligned there is about 60% nucleotide equivalence; more preferably 70%; more preferably 80% equivalence; more preferably 85% equivalence; more preferably 90%; more preferably 95%; and/or more preferably 98% or 99% equivalence over a comparison window. A comparison window is preferably at least 50-100 nucleotides, and more preferably is the entire length of the polynucleotide provided herein. Optimal alignment of sequences for aligning a comparison window may be conducted by algorithms; preferably by computerized implementations of these algorithms (for example, the Wisconsin Genetics Software Package Release 7.0-10.0, Genetics Computer Group, 575 Science Dr., Madison, WI). The reference polynucleotide may be a full-length molecule or a portion of a longer molecule. Preferentially, the window of comparison for determining polynucleotide identity of protein encoding sequences is the entire coding region.

### Recombinant DNA

Proteins useful for imparting improved traits are entire proteins or at least a sufficient portion of the entire protein to impart the relevant biological activity of the protein. The term "protein" also includes molecules consisting of one or more polypeptide chains. Thus, a protein useful in the present invention may constitute an entire protein having the desired biological activity, or may constitute a portion of an oligomeric protein having multiple polypeptide chains. Proteins useful for generation of transgenic plants having improved traits include the proteins with an amino acid sequence provided herein as SEQ ID NO: 240 through SEQ ID NO: 390 and SEQ ID NO: 392 through SEQ ID NO: 478, as well as homologs of such proteins.

Homologs of the proteins useful in the present invention may be identified by comparison of the amino acid sequence of the protein to amino acid sequences of proteins from the same or different plant sources, e.g. manually or by using known homology-based search algorithms such as those commonly known and referred to as BLAST, FASTA, and Smith-Waterman. As used herein, a homolog is a protein from the same or a different organism that performs the same biological function as the polypeptide to which it is compared. An orthologous relation between two organisms is not necessarily manifest as a one-to-one correspondence between two genes, because a gene can be duplicated or deleted after organism phylogenetic separation, such as speciation. For a given protein, there may be no ortholog or more than one ortholog. Other complicating factors include alternatively spliced transcripts from the same gene, limited gene identification, redundant copies of the same gene with different sequence lengths or corrected sequence. A local sequence alignment program, e.g. BLAST, can be used to search a database of sequences to find similar sequences, and the summary Expectation value (E-value) used to measure the sequence base similarity. As a protein hit with the best E-value for a particular organism may not necessarily be an ortholog or the only ortholog, a reciprocal BLAST search is used in the present invention to filter hit sequences with significant E-values for ortholog identification. The reciprocal BLAST entails search of the significant hits against a database of amino acid sequences from the base organism that are similar to the sequence of the query protein. A hit is a likely ortholog, when the reciprocal BLAST's best hit is the query protein itself or a protein encoded by a duplicated gene after speciation. Thus, homolog is used herein to described protein that are assumed to have functional similarity by inference from sequence base similarity. The relationship of homologs with amino acid sequences of SEQ ID NO: 479 through SEQ ID NO: 12463 to the proteins with amino acid sequences of SEQ ID NO: 240 through SEQ ID NO: 478 is found is found in Table 2 appended.

A further aspect of the invention comprises functional homolog proteins which differ in one or more amino acids from those of a trait-improving protein disclosed herein as the result of one or more of the well-known conservative amino acid substitutions, e.g. valine is a conservative substitute for alanine and threonine is a conservative substitute for serine. Conservative substitutions for an amino acid within the native sequence can be selected from other members of a class to which the naturally occurring amino acid belongs. Representative amino acids within these various classes include, but are not limited to: (1) acidic (negatively charged) amino acids such as aspartic acid and glutamic acid; (2) basic (positively charged) amino acids such as arginine, histidine, and lysine; (3) neutral polar amino acids such as glycine, serine, threonine, cysteine, tyrosine, asparagine, and glutamine; and (4) neutral nonpolar (hydrophobic) amino acids such as alanine, leucine, isoleucine, valine, proline, phenylalanine, tryptophan, and methionine. Conserved substitutes for an amino acid within a native amino acid sequence can be selected from other members of the group to which the naturally occurring amino acid belongs. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Naturally conservative amino acids substitution groups are: valine-leucine, valine-isoleucine, phenylalanine-tyrosine, lysine-arginine, alanine-valine, aspartic acid-glutamic acid, and asparagine-glutamine. A further aspect of the invention comprises proteins that differ in one or more amino acids from those of a described protein sequence as the result of deletion or insertion of one or more amino acids in a native sequence.

Homologs of the trait-improving proteins disclosed provided herein will generally demonstrate significant sequence identity. Of particular interest are proteins having at least 50% sequence identity, more preferably at least about 70% sequence identity or higher, e.g. at least about 80% sequence identity with an amino acid sequence of SEQ ID NO: 240 through SEQ ID NO: 390 and SEQ ID NO: 392 through SEQ ID NO: 478. Of course useful proteins also include those with higher identity, e.g. 90% to 99% identity. Identity of protein homologs is determined by optimally aligning the amino acid sequence of a putative protein homolog with a defined amino acid sequence and by calculating the percentage of identical and conservatively substituted amino acids over the window of comparison. The window of comparison for determining identity can be the entire amino acid sequence disclosed herein, e.g. the full sequence of any of SEQ ID NO: 479 through SEQ ID NO: 12463.

Genes that are homologous to each other can be grouped into families and included in multiple sequence alignments. Then a consensus sequence for each group can be derived. This analysis enables the derivation of conserved and class- (family) specific residues or motifs that are functionally important. These conserved residues and motifs can be further validated with 3D protein structure if available. The consensus sequence can be used to define the full scope of the invention, e.g. to identify proteins with a homolog relationship. Thus, the present invention contemplates that protein homologs include proteins with an amino acid sequence that has at least 90% identity to such a consensus amino acid sequence sequences.

### Promoters

Numerous promoters that are active in plant cells have been described in the literature. These include promoters present in plant genomes as well as promoters from other sources, including nopaline synthase (NOS) promoter and octopine synthase (OCS) promoters carried on tumor-inducing plasmids of *Agrobacterium tumefaciens,* caulimovirus promoters such as the cauliflower mosaic virus or figwort mosaic virus promoters. For instance, see U.S. Patents No. 5,858,742 and 5,322,938 which disclose versions of the constitutive promoter derived from cauliflower mosaic virus (CaMV35S), US Patent No. 5,378,619 which discloses a Figwort Mosaic Virus (FMV) 35S promoter, U.S. Patent 6,437,217 which discloses a maize RS81 promoter, U.S. Patent 5,641,876 which discloses a rice actin promoter, U.S. Patent 6,426,446 which discloses a maize RS324 promoter, U.S. Patent 6,429,362 which discloses a maize PR-1 promoter, U.S. Patent 6,232,526 which discloses a maize A3 promoter, U.S. Patent 6,177,611 which discloses constitutive maize promoters, U.S. Patent 6,433,252 which discloses a maize L3 oleosin promoter, U.S. Patent 6,429,357 which discloses a rice actin 2 promoter and intron, U.S. Patent 5,837,848 which discloses a root specific promoter, U.S. Patent 6,084,089 which discloses cold inducible promoters, U.S. Patent 6,294,714 which discloses light inducible promoters, U.S. Patent 6,140,078 which discloses salt inducible promoters, U.S. Patent 6,252,138 which discloses pathogen inducible promoters, U.S. Patent 6,175,060 which discloses phosphorus deficiency inducible promoters, U.S. Patent Application Publication 2002/0192813A1 which discloses 5', 3' and intron elements useful in the design of effective plant expression vectors, U.S. patent application Serial No. 09/078,972 which discloses a coixin promoter, U.S. patent application Serial No. 09/757,089 which discloses a maize chloroplast aldolase promoter, and U.S. patent application Serial No. 10/739,565 which discloses water-deficit inducible promoters, all of which are incorporated herein by reference. These and numerous other promoters that function in plant cells are known to those skilled in the art and available for use in recombinant polynucleotides of the present invention to provide for expression of desired genes in transgenic plant cells.

Furthermore, the promoters may be altered to contain multiple "enhancer sequences" to assist in elevating gene expression. Such enhancers are known in the art. By including an enhancer sequence with such constructs, the expression of the selected protein may be enhanced. These enhancers often are found 5' to the start of transcription in a promoter that functions in eukaryotic cells, but can often be inserted in the forward or reverse orientation 5' or 3' to the coding sequence. In some instances, these 5' enhancing elements are introns. Deemed to be particularly useful as enhancers are the 5' introns of the rice actin 1 and rice actin 2 genes. Examples of other enhancers that can be used in accordance with the invention include elements from the CaMV 35S promoter, octopine synthase genes, the maize alcohol dehydrogenase gene, the maize shrunken 1 gene and promoters from non-plant eukaryotes.

In some aspects of the invention it is preferred that the promoter element in the DNA construct be capable of causing sufficient expression to result in the production of an effective amount of a polypeptide in water deficit conditions. Such promoters can be identified and isolated from the regulatory region of plant genes that are over expressed in water deficit conditions. Specific water-deficit-inducible promoters for use in this invention are derived from the 5' regulatory region of genes identified as a heat shock protein 17.5 gene (*HSP17.5),* an HVA22 gene *(HVA22),* a Rab17 gene and a cinnamic acid 4-hydroxylase (CA4H) gene *(CA4H) of Zea maize.* Such water-deficit-inducible promoters are disclosed in U.S. application Serial No.10/739,565 ,, incorporated herein by reference.

In other aspects of the invention, sufficient expression in plant seed tissues is desired to effect improvements in seed composition. Exemplary promoters for use for seed composition modification include promoters from seed genes such as napin (U.S. Patent 5,420,034), maize L3 oleosin (U.S. Patent 6,433,252), zein Z27 (Russell et al. (1997) Transgenic Res. 6(2):157-166), globulin 1 (Belanger et al (1991) Genetics 129:863-872), glutelin 1 (Russell (1997) *supra),* and peroxiredoxin antioxidant (Per1) (Stacy et al. (1996) Plant Mol Biol. 31(6):1205-1216).

In still other aspects of the invention, preferential expression in plant green tissues is desired. Promoters of interest for such uses include those from genes such as SSU (Fischhoff et al. (1992) Plant Mol Biol. 20:81-93), aldolase and pyruvate orthophosphate dikinase (PPDK) (Taniguchi et al. (2000) Plant Cell Physiol. 41(1):42-48).

### Gene overexpression

"Gene overexpression" used herein in reference to a polynucleotide or polypeptide indicates that the expression level of a target protein, in a transgenic plant or in a host cell of the transgenic plant, exceeds levels of expression in a non-transgenic plant. In a preferred embodiment of the present invention, a recombinant DNA construct comprises the polynucleotide of interest in the sense orientation relative to the promoter to achieve gene overexpression, which is identified as such in Table 1.

### Gene suppression

Gene suppression includes any of the well-known methods for suppressing transcription of a gene or the accumulation of the mRNA corresponding to that gene thereby preventing translation of the transcript into protein. Posttranscriptional gene suppression is mediated by transcription of integrated recombinant DNA to form double-stranded RNA (dsRNA) having homology to a gene targeted for suppression. This formation of dsRNA most commonly results from transcription of an integrated inverted repeat of the target gene, and is a common feature of gene suppression methods known as anti-sense suppression, co-suppression and RNA interference (RNAi). Transcriptional suppression can be mediated by a transcribed dsRNA having homology to a promoter DNA sequence to effect what is called promoter *trans* suppression.

More particularly, posttranscriptional gene suppression by inserting a recombinant DNA construct with anti-sense oriented DNA to regulate gene expression in plant cells is disclosed in U.S. Patent 5,107,065 (Shewmaker et al.) and US Patent 5,759,829 (Shewmaker et al.)*.* Transgenic plants transformed using such anti-sense oriented DNA constructs for gene suppression can comprise integrated DNA arranged as an inverted repeats that result from insertion of the DNA construct into plants by *Agrobacterium-mediated* transformation, as disclosed by Redenbaugh et al. in "Safety Assessment of Genetically Engineered Flavr Savr™ Tomato, CRC Press, Inc. (1992). Inverted repeat insertions can comprises a part or all of the T-DNA construct, e.g. an inverted repeat of a complete transcription unit or an invetred repeat of transcription terminator sequence. Screening for inserted DNA comprising inverted repeat elements can improve the efficiency of identifying transformation events effective for gene silencing whether the transformation construct is a simple anti-sense DNA construct which must be inserted in multiple copies or a complex inverted repeat DNA construct (e.g. an RNAi construct) which can be inserted as a single copy.

Posttranscriptional gene suppression by inserting a recombinant DNA construct with sense-oriented DNA to regulate gene expression in plants is disclosed in U.S. Patent 5,283,184 (Jorgensen et al.) and U.S. Patent 5,231,020 (Jorgensen et al.*).* Inserted T-DNA providing gene suppression in plants transformed with such sense constructs by *Agrobacterium* is organized predominately in inverted repeat structures, as disclosed by Jorgensen et al., Mol. Gen. Genet., 207:471-477 (1987). See also Stam et al., The Plant Journal,, 12(1), 63-82 (1997) who used segregation studies to support Jorgensen's finding that gene silencing is mediated by multimeric transgene T-DNA loci in which the T-DNAs are arranged in inverted repeats. Screening for inserted DNA comprising inverted repeat elements can improve the gene silencing efficiency when transforming with simple sense-orientated DNA constructs. Gene silencing efficiency can also be improved by screening for single insertion events when transforming with an RNAi construct containing inverted repeat elements

As disclosed by Redenbaugh *et al.* gene suppression can be avhieved by inserting into a plant genome recombinant DNA that transcribes dsRNA. Such a DNA insert can be transcribed to an RNA element having the 3' region as a double stranded RNA. RNAi constructs are also disclosed in EP 0426195 A1 (Goldbach et al. - 1991) where recombinant DNA constructs for transcription into hairpin dsRNA for providing transgenic plants with resistance to tobacco spotted wilt virus. Double-stranded RNAs were also disclosed in WO 94/01550 (Agrawal et al.) where anti-sense RNA was stabilized with a self-complementary 3' segment. Agrawal *et al.* referred to U.S. Patent 5,107,065 for using such self-stablized anti-sense RNAs for regulating gene expression in plant cells; see International Publication No. 94/01550. Other double-stranded hairpin-forming elements in transcribed RNA are disclosed in International Publication No. 98/05770 (Werner et al.*)* where the anti-sense RNA is stabilized by hairpin forming repeats of poly(CG) nucleotides. See also U.S. Patent Application Publication No. 2003/0175965 A1 (Lowe et al.) which discloses gene suppression using and RNAi construct comprising a gene coding sequence preceded by inverted repeats of 5'UTR. See also U.S. Patent Application Publication No. 2002/0048814 A1 (Oeller) where RNAi constructs are transcribed to sense or anti-sense RNA which is stabilized by a poly(T)-poly(A) tail. See also U.S. Patent Application Publication No. 2003/0018993 A1 (Gutterson et al.) where sense or anti-sense RNA is stabilized by an inverted repeat of a of the 3' untranslated region of the NOS gene. See also U.S. Patent Application Publication No. 2003/0036197 A1 (Glassman et al.) where RNA having homology to a target is stabilized by two complementary RNA regions.

Gene silencing can also be effected by transcribing RNA from both a sense and an anti-sense oriented DNA, e.g. as disclosed by Shewmaker et al. in U.S. Patent 5,107,065 where in Example 1 a binary vector was prepared with both sense and anti-sense *aroA* genes. See also U.S. Patent 6,326,193 where gene targeted DNA is operably linked to opposing promoters.

Gene silencing can also be affected by transcribing from contiguous sense and anti-sense DNA. In this regard see Sijen et al., The Plant Cell, Vol. 8, 2277-2294 (1996)discloses the use of constructs carrying inverted repeats of a cowpea mosaic virus gene in transgenic plants to mediate virus resistance. Such constructs for posttranscriptional gene suppression in plants by double-stranded RNA are also disclosed in International Publication No. WO 99/53050 (Waterhouse et al.*),* International Publication No. WO 99/49029 (Graham et al.*),* U.S. Patent Application No.10/465,800 (Fillatti), U.S. Patent 6,506,559 (Fire et al.*).* See also U.S. Application Serial No. 10/393,347 (Shewmaker et al.) that discloses constructs and methods for simultaneously expressing one or more recombinant genes while simultaneously suppressing one or more native genes in a transgenic plant. See also U.S. Patent 6,448,473 (Mitsky et al.) that discloses multi-gene suppression vectors for use in plants. All of the above-described patents, applications and international publications disclosing materials and methods for posttranscriptional gene suppression in plants are incorporated herein by reference.

Transcriptional suppression such as promoter *trans* suppression can be affected by a expressing a DNA construct comprising a promoter operably linked to inverted repeats of promoter DNA for a target gene. Constructs useful for such gene suppression mediated by promoter *trans* suppression are disclosed by Mette et al., The EMBO Journal, Vol. 18, No. 1, pp. 241-148, 1999 and by Mette et al., The EMBO Journal, Vol. 19, No. 19, pp. 5194-5201-148, 2000, both of which are incorporated herein by reference.

Suppression can also be achieved by insertion mutations created by transposable elements may also prevent gene function. For example, in many dicot plants, transformation with the T-DNA of *Agrobacterium* may be readily achieved and large numbers of transformants can be rapidly obtained. Also, some species have lines with active transposable elements that can efficiently be used for the generation of large numbers of insertion mutations, while some other species lack such options. Mutant plants produced by *Agrobacterium* or transposon mutagenesis and having altered expression of a polypeptide of interest can be identified using the polynucleotides of the present invention. For example, a large population of mutated plants may be screened with polynucleotides encoding the polypeptide of interest to detect mutated plants having an insertion in the gene encoding the polypeptide of interest.

### Gene stacking

The present invention also contemplates that the trait-improving recombinant DNA provided herein can be used in combination with other recombinant DNA to create plants with a multiple desired traits. The combinations generated can include multiple copies of any one or more of the recombinant DNA constructs.

These stacked combinations can be created by any method, including but not limited to cross breeding of transgenic plants, or multiple genetic transformation.

### Plant Transformation Methods

Numerous methods for transforming plant cells with recombinant DNA are known in the art and may be used in the present invention. Two commonly used methods for plant transformation are *Agrobacterium-mediated* transformation and microprojectile bombardment. Microprojectile bombardment methods are illustrated in U.S. Patents 5,015,580 (soybean); 5,550,318 (corn); 5,538,880 (corn); 5,914,451 (soybean); 6,160,208 (corn); 6,399,861 (corn) and 6,153,812 (wheat) and *Agrobacterium*-mediated transformation is described in U.S. Patents 5,159,135 (cotton); 5,824,877 (soybean); 5,591,616 (corn); and 6,384,301 (soybean), all of which are incorporated herein by reference. For *Agrobacterium tumefaciens* based plant transformation system, additional elements present on transformation constructs will include T-DNA left and right border sequences to facilitate incorporation of the recombinant polynucleotide into the plant genome.

In general it is preferred to introduce heterologous DNA randomly, i.e. at a nonspecific location, in the genome of a target plant line. In special cases it may be useful to target heterologous DNA insertion in order to achieve site-specific integration, e.g. to replace an existing gene in the genome, to use an existing promoter in the plant genome, or to insert a recombinant polynucleotide at a predetermined site known to be active for gene expression. Several site specific recombination systems exist which are known to function implants include cre-lox as disclosed in U.S. Patent 4,959,317 and FLP-FRT as disclosed in U.S. Patent 5,527,695, both incorporated herein by reference.

Transformation methods of this invention are preferably practiced in tissue culture on media and in a controlled environment. "Media" refers to the numerous nutrient mixtures that are used to grow cells *in vitro,* that is, outside of the intact living organism. Recipient cell targets include, but are not limited to, meristem cells, callus, immature embryos and gametic cells such as microspores, pollen, sperm and egg cells. It is contemplated that any cell from which a fertile plant may be regenerated is useful as a recipient cell. Callus may be initiated from tissue sources including, but not limited to, immature embryos, seedling apical meristems, microspores and the like. Cells capable of proliferating as callus are also recipient cells for genetic transformation. Practical transformation methods and materials for making transgenic plants of this invention, e.g. various media and recipient target cells, transformation of immature embryos and subsequent regeneration of fertile transgenic plants are disclosed in U.S. Patents 6,194,636 and 6,232,526 and U.S. patent application Serial No. 09/757,089, which are incorporated herein by reference.

In practice DNA is introduced into only a small percentage of target cells in any one experiment. Marker genes are used to provide an efficient system for identification of those cells that are stably transformed by receiving and integrating a transgenic DNA construct into their genomes. Preferred marker genes provide selective markers that confer resistance to a selective agent, such as an antibiotic or herbicide. Potentially transformed cells are exposed to the selective agent. In the population of surviving cells will be those cells where, generally, the resistance-conferring gene has been integrated and expressed at sufficient levels to permit cell survival. Cells may be tested further to confirm stable integration of the exogenous DNA. Useful selective marker genes include those conferring resistance to antibiotics such as kanamycin (*nptII*), hygromycin B (*aph IV*) and gentamycin (*aac3* and *aacC*4) or resistance to herbicides such as glufosinate (*bar* or *pat*) and glyphosate (EPSPS). Examples of such selectable are illustrated in U.S. Patents 5,550,318; 5,633,435; 5,780,708 and 6,118,047, all of which are incorporated herein by reference. Screenable markers which provide an ability to visually identify transformants can also be employed, e.g., a gene expressing a colored or fluorescent protein such as a luciferase or green fluorescent protein (GFP) or a gene expressing a *beta*-glucuronidase or *uid*A gene (GUS) for which various chromogenic substrates are known. It is also contemplated that combinations of screenable and selectable markers will be useful for identification of transformed cells. See PCT publication WO 99/61129 which discloses use of a gene fusion between a selectable marker gene and a screenable marker gene, e.g. an NPTII gene and a GFP gene.

Cells that survive exposure to the selective agent, or cells that have been scored positive in a screening assay, may be cultured in regeneration media and allowed to mature into plants. Developing plantlets can be transferred to soil less plant growth mix, and hardened off, e.g., in an environmentally controlled chamber at about 85% relative humidity, 600 ppm CO₂, and 25-250 microeinsteins m⁻² s⁻¹ of light, prior to transfer to a greenhouse or growth chamber for maturation. Plants are preferably matured either in a growth chamber or greenhouse. Plants are regenerated from about 6 wk to 10 months after a transformant is identified, depending on the initial tissue. During regeneration, cells are grown to plants on solid media at about 19 to 28°C. After regenerating plants have reached the stage of shoot and root development, they may be transferred to a greenhouse for further growth and testing. Plants may be pollinated using conventional plant breeding methods known to those of skill in the art and seed produced.

Progeny may be recovered from transformed plants and tested for expression of the exogenous recombinant polynucleotide. Useful assays include, for example, "molecular biological" assays, such as Southern and Northern blotting and PCR; "biochemical" assays, such as detecting the presence of RNA, e.g. double stranded RNA, or a protein product, e.g., by immunological means (ELISAs and Western blots) or by enzymatic function; plant part assays, such as leaf or root assays; and also, by analyzing the phenotype of the whole regenerated plant.

### Discovery of Trait-improving Recombinant DNA

To identify recombinant DNA that confer improved traits to plants, *Arabidopsis thaliana* was transformed with a candidate recombinant DNA construct and screened for an improved trait.

*Arabidopsis thaliana* is used a model for genetics and metabolism in plants. *Arabidopsis* has a small genome, and well documented studies are available. It is easy to grow in large numbers and mutants defining important genetically controlled mechanisms are either available, or can readily be obtained. Various methods to introduce and express isolated homologous genes are available (see Koncz, et al., eds. Methods in Arabidopsis Research. et al. (1992), World Scientific, New Jersey, New Jersey, in "Preface").

A two-step screening process was employed which comprised two passes of trait characterization to ensure that the trait modification was dependent on expression of the recombinant DNA, but not due to the chromosomal location of the integration of the transgene. Twelve independent transgenic lines for each recombinant DNA construct were established and assayed for the transgene expression levels. Five transgenic lines with high transgene expression levels were used in the first pass screen to evaluate the transgene's function in T2 transgenic plants. Subsequently, three transgenic events, which had been shown to have one or more improved traits, were further evaluated in the second pass screen to confirm the transgene's ability to impart an improved trait. The following Table 3 summarizes the improved traits that have been confirmed as provided by a recombinant DNA construct .

In particular Table3 reports
"PEP SEQ ID NO" which is the amino acid sequence of the protein cognate to the DNA in the recombinant DNA construct corresponding to a protein sequence of a SEQ ID NO. in the Sequence Listing;
"construct_id" is an arbitrary name for the recombinant DNA describe more particularly in Table 1;
"annotation" refers to a description of the top hit protein obtained from an amino acid sequence query of each PEP SEQ ID NO to GenBank database of the National Center for Biotechnology Information (ncbi). More particularly, "gi" is the GenBank ID number for the top BLAST hit;
" description" refers to the description of the top BLAST hit ;
"e-value" provides the expectation value for the BLAST hit;
"identity" refers to the percentage of identically matched amino acid residues along the length of the portion of the sequences which is aligned by BLAST between the sequence of interest provided herein and the hit sequence in GenBank;
"traits" identifies by two letter code the confirmed improvement in a transgenic plant provided by the recombinant DNA. The codes for improved traits are:
"CK" which indicates cold tolerance improvement identified under a cold shock tolerance screen;
"CS" which indicates cold tolerance improvement identified by a cold germination tolerance screen ;
"SD" which indicates drought tolerance improvement identified by a drought stress tolerance screen ;
"PEG" which indicates osmotic stress tolerance improvement identified by a PEG induced osmotic stress tolerance screen;
"HS" which indicates heat stress tolerance improvement identified by a heat stress tolerance screen;
"SS" which indicates high salinity stress tolerance improvement identified by a salt stress tolerance screen;
"LN" which : indicates nitrogen use efficiency improvement identified by a low nitrogen tolerance screen.
"LL" which indicates attenuated shade avoidance response identified by a shade tolerance screen under a low light condition;
"PP" which indicates improved growth and development at early stages identified by an early plant growth and development screen;
"SP" which indicates improved growth and development at late stages identified by a late plant growth and development screen provided herein.

**Table 3**

| **PEP SEQ ID NO** | **construct_id** | **Annotation** | | | | **traits** | | | | | |
|---|---|---|---|---|---|---|---|---|---|---|---|
| | | **e-value** | **identity** | **gi** | **Description** | | | | | | |
| 240 | 19867 | 2.00E-72 | 47 | 15226242 | (NM_128336) hypothetical protein [Arabidopsis thaliana] | CK | CS | | | | |
| 241 | 74518 | 0 | 100 | 17980436 | bacteriophytochrome [Pseudomonas fluorescens] | DS | LN | PE G | PP | CK | CS |
| 242 | 15816 | 1.00E-101 | 100 | 18414706 | (NM_120565) expressed protein [Arabidopsis thaliana] dbj\|BAB08987.1 | CK | | | | | |
| 243 | 17918 | 5.00E-78 | 81 | 15232662 | (AB017071) zinc finger protein-like; Ser/Thr protein kinase-like protein [Arabidopsis thaliana] | SS | CK | | | | |
| 244 | 15306 | 1.00E-121 | 57 | 15227057 | (NM_126342) predicted by genefinder and genscan | CS | | | | | |
| 245 | 12038 | 2.00E-47 | 100 | 18413298 | auxin-regulated protein [Arabidopsis thaliana] gi\|30681325\|ref\|NP 849354.1 | PP | CS | | | | |
| 246 | 12046 | | | | | CS | | | | | |
| 247 | 13432 | 1.00E-79 | 53 | 18396732 | (NM_111270) expressed protein [Arabidopsis thaliana] gb\|AAF05858.1 | CS | | | | | |
| 248 | 13711 | 1.00E-33 | 84 | 15232724 | expressed protein [Arabidopsis thaliana] gi\|11280688\|pir\|\|T45643 hypothetical protein | CS | | | | | |
| 249 | 14809 | 1.00E-142 | 100 | 15230177 | AF488576_1 (AF488576) putative bHLH transcription factor [Arabidopsis thaliana] | CS | | | | | |
| 250 | 14951 | 1.00E-146 | 100 | 4056434 | (AC005990) Similar to OBP32pep protein gb\|U37698 from Arabidopsis thaliana | PP | CS | | | | |
| 251 | 15632 | 0 | 89 | 9758356 | (AB013396) eukaryotic initiation factor 4, eIF4-like protein [Arabidopsis thaliana] | CS | | | | | |
| 252 | 16147 | 0 | 100 | 11890406 | (AF197940)SAM:phosphoethanolamine N-methyltransferase [Arabidopsis thaliana] g | CS | | | | | |
| 253 | 16158 | 1.00E-68 | 100 | 18412355 | (NM_106587) expressed protein [Arabidopsis thaliana] | CS | | | | | |
| 254 | 16170 | 2.00E-82 | 91 | 15224757 | (NM_127488) putative small heat shock protein [Arabidopsis thaliana] | CS | | | | | |
| 255 | 16171 | 4.00E-92 | 72 | 18401372 | (NM_128284) expressed protein [Arabidopsis thaliana] | CS | | | | | |
| 256 | 16175 | 1.00E-157 | 85 | 15240715 | (NM_126137) putative protein [Arabidopsis thaliana] | CS | | | | | |
| 257 | 17430 | 1.00E-155 | 90 | 13878155 | (AF370340) putative mitochondrial dicarboxylate carrier protein [Arabidopsis thaliana] | CS | | | | | |
| 258 | 17819 | 1.00E-140 | 95 | 15236507 | (NM_116915) hypothetical protein [Arabidopsis thaliana] emb\|CAB77971.1 | SS | CS | | | | |
| 259 | 17921 | 1.00E-110 | 74 | 18415982 | (NM_118393) HSP associated protein like [Arabidopsis thaliana] | CS | | | | | |
| 260 | 17928 | 1.00E-74 | 100 | 15231105 | (NM_115730) transcriptional coactivator - like protein [Arabidopsis thaliana] | PP | CS | | | | |
| 261 | 18637 | 1.00E-128 | 87 | 15233509 | (NM_118226) putative protein [Arabidopsis thaliana] | CS | | | | | |
| 262 | 18816 | 0 | 94 | 18398254 | (NM_102942) expressed protein [Arabidopsis thaliana] | LL | CS | | | | |
| 263 | 19227 | 1.00E-164 | 96 | 15219482 | (NM_106009) MAP kinase, putative [Arabidopsis thaliana] | CS | | | | | |
| 264 | 19429 | 0 | 100 | 15237038 | (NM_118860) GH3 like protein [Arabidopsis thaliana] | CS | | | | | |
| 265 | 70235 | 2.00E-94 | 100 | 15234243 | (NM_117229) phospholipid hydroperoxide glutathione peroxidase [Arabidopsis thaliana] | PP | CS | | | | |
| 266 | 72634 | 4.00E-52 | 86 | 30699033 | GAST1-related protein [Arabidopsis thaliana] | CS | | | | | |
| 267 | 72752 | 0 | 100 | 6319971 | (NC_001136) phosphotyrosine-specific protein phosphatase; Ptp1p [Saccharomyces cerevisiae] | PP | CS | | | | |
| 268 | 12007 | 1.00E-119 | 76 | 15236117 | (NM_118746) uncharacterized protein [Arabidopsis thaliana] | HS | | | | | |
| 269 | 12290 | 5.00E-66 | 92 | 18396460 | (NM_111186) expressed protein [Arabidopsis thaliana]] | HS | | | | | |
| 270 | 12343 | 1.00E-111 | 61 | 18414724 | (NM_120571) expressed protein [Arabidopsis thaliana] gb\|AAF61902.1\|AF208051_1 (AF208051) small heat shock-like protein | HS | | | | | |
| 271 | 14348 | 0 | 100 | 15234254 | (NM_118912) putative protein [Arabidopsis thaliana] pir\|\|T05878 isp4 protein homolog T29A15.220 | HS | | | | | |
| 272 | 15708 | 7.00E-71 | 87 | 18394214 | (NM_101391) expressed protein [Arabidopsis thaliana] | HS | | | | | |
| 273 | 17615 | 1.00E-108 | 85 | 18414711 | (NM_120567) expressed protein [Arabidopsis thaliana] | CS | HS | | | | |
| 274 | 17622 | 0 | 93 | 15238837 | (NM_121852) putative protein [Arabidopsis thaliana] | HS | | | | | |
| 275 | 70714 | 0 | 94 | 7446439 | probable serine/threonine-specific protein kinase (EC 2.7.1.-) F1715.140 - Arabidopsis thaliana emb\|CAA19877.1\| (AL031032) protein kinase-like protein [Arabidopsis thaliana] | HS | | | | | |
| 276 | 17925 | 1.00E-157 | 82 | 18405518 | (NM_129646) expressed protein [Arabidopsis thaliana] pir\|\|00747 RING-H2 finger protein RHC1a | HS | CK | | | | |
| 277 | 18541 | 1.00E-128 | 83 | 15237100 | (NM_119735) hypothetical protein [Arabidopsis thaliana] | HS | CS | | | | |
| 278 | 11425 | 1.00E-155 | 100 | 18405364 | (AB024028) 20S proteasome beta subunit; multicatalytic endopeptidase [Arabidopsis thaliana] | HS | | | | | |
| 279 | 12263 | 9.00E-39 | 100 | 15241279 | small zinc finger-related protein [Arabidopsis thaliana] gi\|12230183\|sp\|Q9XGY4\|IM08_AR ATH Mitochondrial import inner membrane translocase subunit Tim8 | HS | | | | | |
| 280 | 12288 | 0 | 97 | 7488126 | AAB01678.11 (U27590) Fe(II) transport protein [Arabidopsis thaliana] | HS | | | | | |
| 281 | 12910 | 0 | 90 | 17065456 | (AY062804) A6 anther-specific protein [Arabidopsis thaliana] | HS | | | | | |
| 282 | 14335 | 0 | 93 | 15229692 | (NM_111953) omega-3 fatty acid desaturase, chloroplast precursor [Arabidopsis thaliana] | LL | HS | | | | |
| 283 | 17427 | 0 | 100 | 13878127 | (AF370326) putative 2-nitropropane dioxygenase [Arabidopsis thaliana] | HS | | | | | |
| 284 | 19140 | 0 | 100 | 15223458 | (NM_104489) SAR DNA binding protein, putative [Arabidopsis thaliana] gb\|AAF02835.1\|AC009894_6 (AC009894) nucleolar protein [Arabidopsis thaliana] gb\|AAG40838.1\|AF302492_1 (AF302492) NOP56-like protein [Arabidopsis thaliana] | HS | | | | | |
| 285 | 19179 | 7.00E-97 | 92 | 18390408 | (NM_100335) expressed protein [Arabidopsis thaliana] gb\|AAB80630.1\| (AC002376) Strong similarity to Triticum ABA induced membrane protein (gb\|U80037) | PP | SS | HS | | | |
| 286 | 19251 | 0 | 100 | 21553584 | (AY085451) putative 3-isopropylmalate dehydrogenase [Arabidopsis thaliana] | HS | | | | | |
| 287 | 19443 | 1.00E-171 | 100 | 15220490 | (NM_102700) zinc finger protein, putative [Arabidopsis thaliana] gb\|AAG51745.1\|AC068667_24 (AC068667) zinc finger protein, putative; 86473-88078 [Arabidopsis thaliana] | HS | | | | | |
| 288 | 19607 | 0 | 90 | 15239867 | (NM_124313) xylosidase [Arabidopsis thaliana] | HS | | | | | |
| 289 | 19915 | 2.00E-87 | 50 | 15229221 | (NM_111278) NAM-like protein (no apical meristem) [Arabidopsis thaliana] | SP | HS | | | | |
| 290 | 70222 | 0 | 97 | 15240523 | (NM_124341) amino acid permease 6 (emb\|CAA65051.1) [Arabidopsis thaliana] | DS | PP | HS | | | |
| 291 | 70464 | 1.00E-106 | 92 | 15233481 | (NM_118221) putative protein [Arabidopsis thaliana] | HS | | | | | |
| 292 | 70474 | 1.00E-148 | 99 | 20127049 | (AF488587) putative bHLH transcription factor [Arabidopsis thaliana] | HS | | | | | |
| 293 | 70484 | 0 | 93 | 18418491 | (NM_119632) putative protein [Arabidopsis thaliana] | CS | PP | HS | | | |
| 294 | 72474 | 1.00E-129 | 81 | 14150732 | (AF374475) hypersensitive-induced response protein [Oryza sativa] | PP | HS | | | | |
| 295 | 13047 | 1.00E-170 | 86 | 15237573 | (NM_123481) purine permease-like protein [Arabidopsis thaliana] dbj\|BAB09718.1\| (AB010072) purine permease-like protein [Arabidopsis thaliana] | LL | | | | | |
| 296 | 13304 | 0 | 92 | 15227905 | (NM_127337) putative senescence-associated protein 12 [Arabidopsis thaliana] | LL | | | | | |
| 297 | 13474 | 0 | 97 | 2318131 | (AF014824) histone deacetylase [Arabidopsis thaliana] | LL | | | | | |
| 298 | 19252 | 3.00E-85 | 100 | 18397475 | (NM_111486) putative dual-specificity protein phosphatase [Arabidopsis thaliana] | PP | LL | SS | HS | CS | |
| 299 | 12612 | 7.00E-84 | 67 | 18397426 | (NM_111472) expressed protein [Arabidopsis thaliana] | LL | | | | | |
| 300 | 12926 | 2.00E-08 | 63 | 18407064 | expressed protein [Arabidopsis thaliana] gi\|25408990\|pir\|\| | LL | | | | | |
| 301 | 13230 | 3.00E-68 | 83 | 15233017 | (NM_111160) unknown protein [Arabidopsis thaliana] | LL | | | | | |
| 302 | 14235 | 1.00E-142 | 82 | 18402650 | (NM_103835) expressed protein [Arabidopsis thaliana] | LL | | | | | |
| 303 | 17305 | 0 | 100 | 15222179 | (NM_100550) sugar kinase, putative [Arabidopsis thaliana] | LL | | | | | |
| 304 | 17470 | 1.00E-138 | 76 | 15219110 | AAD17313.1\|(AF 123310) NAC domain protein NAM [Arabidopsis thaliana] | LL | | | | | |
| 305 | 17718 | 2.00E-91 | 91 | 15228362 | (NM_114694) putative protein [Arabidopsis thaliana] | LL | | | | | |
| 306 | 17904 | 0 | 97 | 18399578 | (NM_112070) expressed protein [Arabidopsis thaliana] | LL | | | | | |
| 307 | 18280 | 0 | 97 | 18398767 | AAM66940.1\| (AY088617) membrane-associated salt-inducible protein like [Arabidopsis thaliana] | DS | LL | | | | |
| 308 | 18287 | 1.00E-148 | 100 | 15223439 | (NM_100045) polyphosphoinositide binding protein, putative [Arabidopsis thaliana] | LL | | | | | |
| 309 | 18501 | 0 | 94 | 18418838 | (NM_121863) putative protein [Arabidopsis thaliana] gb\|AAG35778.1\|AF280057_1 (AF280057) tonneau 2 [Arabidopsis thaliana] | LL | | | | | |
| 310 | 18877 | 1.00E-85 | 100 | 18408502 | (NM_105311)calmodulin-related protein [Arabidopsis thaliana] | LL | | | | | |
| 311 | 19531 | 0 | 98 | 15241970 | (NM_125674) 1-deoxy-D-xylulose 5-phosphate reductoisomerase (DXR) [Arabidopsis thaliana] | LL | | | | | |
| 312 | 70405 | 0 | 85 | 18390592 | (NM_100475) expressed protein [Arabidopsis thaliana] | SS | LL | | | | |
| 313 | 72136 | 2.00E-37 | 66 | 123379 | HMG1/2-like protein (SB11 protein) gi\|99914\|pir\|\|22309 high mobility group protein HMG-1-soybean gi\|18645\|emb\|CAA41200.1\| HMG-1 like protein gene [Glycine max] | LL | | | | | |
| 314 | 72611 | 1.00E-102 | 82 | 6721504 | (AP001072) hypothetical protein [Oryza sativa (japonica cultivar-group)] | LL | | | | | |
| 315 | 12627 | 0 | 100 | 15236663 | (NM_118524) UDPglucose 4-epimerase - like protein [Arabidopsis thaliana] | LN | | | | | |
| 316 | 12813 | 0 | 96 | 2454184 | (U80186) pyruvate dehydrogenase E1 beta subunit [Arabidopsis thaliana] | LN | | | | | |
| 317 | 14945 | 1.00E-122 | 92 | 18400517 | (NM_112338) expressed protein [Arabidopsis thaliana] dbj\|BAB02642.1\| (AP002061) MtN3-like protein | LN | | | | | |
| 318 | 15345 | 0 | 97 | 15237392 | (NM_123987) ornithine aminotransferase [Arabidopsis thaliana] | LN | | | | | |
| 319 | 15348 | 0 | 81 | 18414239 | (NM_117530) expressed protein [Arabidopsis thaliana] | LN | | | | | |
| 320 | 16325 | 1.00E-105 | 100 | 15225174 | (NM_128763) putative alanine acetyl transferase [Arabidopsis thaliana] gb\|AAD15401.1 | LN | | | | | |
| 321 | 16702 | 0 | 77 | 18408943 | (NM_105480) expressed protein [Arabidopsis thaliana] sp\|Q9M647\|IAR1_ARATH IAA-alanine resistance protein 1 | LN | | | | | |
| 322 | 16836 | 0 | 100 | 11692854 | AF327534_1 (AF327534) putative adenosine triphosphatase [Arabidopsis thaliana] | LN | | | | | |
| 323 | 17002 | 1.00E-138 | 56 | 18414140 | (NM_117486) Expressed protein [Arabidopsis thaliana] gb\|AAK68800.1\| (AY042860) Unknown protein [Arabidopsis thaliana] | LN | | | | | |
| 324 | 17012 | 3.00E-79 | 100 | 18398187 | (NM_127222) actin depolymerizing factor 5 [Arabidopsis thaliana] | LN | | | | | |
| 325 | 17017 | 1.00E-155 | 86 | 11358585 | nuclear envelope membrane protein-like - Arabidopsis thaliana | LN | | | | | |
| 326 | 17344 | 3.00E-49 | 100 | 18424201 | SKP1 family [Arabidopsis thaliana] gi\|9759236\|dbj\|BAB09760.1 contains similarity to elongin C~gene_id:MNC17.5 [Arabidopsis thaliana] gi\|15028385\|gb\|AAK76669.1\| putative elongin protein] | PP | LN | | | | |
| 327 | 17426 | 0 | 95 | 15238801 | (NM_124151)farnesyl diphosphate synthase precursor (gb\|AAB49290.1) [Arabidopsis thaliana] | LN | | | | | |
| 328 | 17655 | 1.00E-129 | 86 | 15227472 | (NM_129758) putative C2H2-type zinc finger protein [Arabidopsis thaliana] | LN | | | | | |
| 329 | 17656 | 1.00E-135 | 74 | 15233081 | (NM_15995) putative DNA-binding protein [Arabidopsis thaliana] | LN | | | | | |
| 330 | 17906 | 1.00E-129 | 100 | 18378887 | (NM_100065) expressed protein [Arabidopsis thaliana] | PP | LN | | | | |
| 331 | 18278 | 0 | 95 | 15220147 | (NM_103617) Cyclin, putative [Arabidopsis thaliana] | LN | | | | | |
| 332 | 18822 | 0 | 92 | 15232759 | (NM_111813) putative protein kinase [Arabidopsis thaliana] | LN | | | | | |
| 333 | 18881 | 0 | 100 | 18401029 | (NM_112485) putative L-asparaginase [Arabidopsis thaliana] | LN | | | | | |
| 334 | 19213 | 2.00E-70 | 91 | 18408726 | (NM_105394) expressed protein [Arabidopsis thaliana] | LN | | | | | |
| 335 | 19239 | 1.00E-59 | 100 | 15235876 | DNA-directed RNA polymerase subunit-related [Arabidopsis thaliana] gi\|25313101\|pir\|\|A85078 | LN | | | | | |
| 336 | 19247 | 1.00E-81 | 53 | 9711883 | (AP002524) hypothetical protein-similar to Drosophila melanogaster chromosome 3L, CG10171 gene product [Oryza sativa (japonica cultivar-group)] | LN | | | | | |
| 337 | 19460 | 1.00E-146 | 80 | 15238816 | (NM_121850) AP2-domain DNA-binding protein -like [Arabidopsis thaliana] | LN | | | | | |
| 338 | 19512 | 0 | 85 | 15237502 | (NM_124046) bHLH protein-like [Arabidopsis thaliana] | LN | | | | | |
| 339 | 19533 | 0 | 99 | 18395911 | (NM_102409) expressed protein [Arabidopsis thaliana] | LN | | | | | |
| 340 | 19603 | 0 | 87 | 18403383 | (NM_113143) expressed protein [Arabidopsis thaliana] dbj\|BAB01784.1\| (AB022215) hydroxyproline-rich glycoprotein [Arabidopsis thaliana] | LN | | | | | |
| 341 | 72126 | 5.00E-78 | 53 | 12005328 | (AF239956) unknown [Hevea brasiliensis] | LN | | | | | |
| 342 | 72437 | 8.00E-89 | 92 | 11994756 | (AP001313) kinetechore (Skp1 p-like) protein-like [Arabidopsis thaliana] | LN | | | | | |
| 343 | 72441 | 5.00E-95 | 84 | 15218602 | (NM_100157) ribosomal protein L19, putative [Arabidopsis thaliana] | LN | | | | | |
| 344 | 72639 | 2.00E-73 | 91 | 18403896 | (NM_104101) expressed protein [Arabidopsis thaliana] | LN | | | | | |
| 345 | 14825 | 0 | 93 | 15242814 | (NM_120445) protein kinase -like protein [Arabidopsis thaliana] | PEG | | | | | |
| 346 | 17931 | 1.00E-136 | 68 | 15242003 | (NM_125688) Dof zinc finger protein - like [Arabidopsis thaliana] | PEG | CS | | | | |
| 347 | 18854 | 1.00E-164 | 79 | 18423918 | (NM_125077) nucleosome assembly protein [Arabidopsis thaliana] | PEG | HS | | | | |
| 348 | 12237 | 1.00E-21 | 76 | 18398176 | expressed protein [Arabidopsis thaliana] gi\|12322743\|gb\|AAG51367.1\|AC0 12562_28 | PEG | | | | | |
| 349 | 13414 | 2.00E-69 | 92 | 12324443 | (AC012329) unknown protein; 50647-51606 [Arabidopsis thaliana] | PEG | | | | | |
| 350 | 16160 | 1.00E-176 | 87 | 18415888 | (NM_118352) putative protein [Arabidopsis thaliana] | PEG | | | | | |
| 351 | 16226 | 1.00E-138 | 96 | 9294682 | (AP001305) contains similarity to RNA polymerase transcriptional regulation mediator~gene_id:MHC9.3 [Arabidopsis thaliana] | HS | PEG | | | | |
| 352 | 16803 | 1.00E-146 | 90 | 18394201 | (NM_101382) expressed protein [Arabidopsis thaliana] gb\|AAD39643.1\|AC007591_8 (AC007591) Contains a PF\|00175 Oxidoreductase FAD/NADH-binding domain. | PEG | | | | | |
| 353 | 18260 | 0 | 100 | 15219795 | (NM_100349) putative K+ channel, beta subunit [Arabidopsis thaliana] | PEG | | | | | |
| 354 | 18642 | 2.00E-68 | 71 | 15235819 | (NM_118411) predicted protein [Arabidopsis thaliana] | PP | PEG | | | | |
| 355 | 18721 | 3.00E-21 | 53 | 18408611 | glycine-rich protein [Arabidopsis thaliana] gi\|12597766\|gb\|AAG60079.1\|AC0 13288_13 | PEG | | | | | |
| 356 | 19254 | 0 | 96 | 18398480 | (NM_111769) expressed protein [Arabidopsis thaliana] | PEG | | | | | |
| 357 | 70247 | 0 | 95 | 15238559 | (NM_122954) glutamate-ammonia ligase (EC 6.3.1.2) precursor, chloroplast (clone lambdaAtgsl1) (pir\|\|S18600) [Arabidopsis thaliana] | CS | DS | HS | PP | PEG | |
| 358 | 70650 | 1.00E-83 | 57 | 18399283 | (NM_127582) expressed protein [Arabidopsis thaliana] | PP | PEG | | | | |
| 359 | 12635 | 0 | 97 | 15231953 | (NM_111700) putative non-phototropic hypocotyl [Arabidopsis thaliana] | HS | | | | | |
| 359 | 11787 | 0 | 97 | 15231953 | (NM_111700) putative non-phototropic hypocotyl [Arabidopsis thaliana] | PP | | | | | |
| 360 | 13641 | 1.00E-42 | 87 | 15218189 | dynein light chain -related [Arabidopsis thaliana] gi\|25405535\|pir\|\|E96562 | PP | | | | | |
| 361 | 14515 | 0 | 71 | 15128395 | (AP003255) contains ESTs AU100655(C11462),C26007(C114 62)-similar to Arabidopsis thaliana chromosome 3, F24B22.150-unknown protein [Oryza sativa (japonica cultivar-group)] | PP | | | | | |
| 362 | 14920 | 0 | 100 | 4239819 | (AB010875) PHR1 [Arabidopsis thaliana] | PP | | | | | |
| 363 | 15204 | 0 | 96 | 15230379 | (NM_112829) putative tyrosine phosphatase [Arabidopsis thaliana] | PP | | | | | |
| 364 | 19058 | 0 | 96 | 18396298 | (NM_102496) expressed protein [Arabidopsis thaliana] | LN | | | | | |
| 364 | 15216 | 0 | 96 | 18396298 | (NM_102496) expressed protein [Arabidopsis thaliana] | PP | | | | | |
| 365 | 15330 | 5.00E-68 | 59 | 18400296 | (NM_112272) expressed protein [Arabidopsis thaliana] | PP | | | | | |
| 366 | 19610 | 0 | 100 | 18409509 | (NM_115079) expressed protein [Arabidopsis thaliana] | PP | CS | | | | |
| 367 | 14338 | 0 | 100 | 15222967 | (NM_103926) sterol delta7 reductase [Arabidopsis thaliana] SP | PP | HS | CS | | | |
| 368 | 17809 | 0 | 100 | 15242240 | (NM_124576) sorbitol dehydrogenase-like protein [Arabidopsis thaliana] | PP | HS | | | | |
| 369 | 72471 | 3.00E-83 | 52 | 18395821 | (NM_111011) expressed protein [Arabidopsis thaliana] | DS | PP | HS | | | |
| 370 | 16403 | 1.00E-176 | 73 | 15237042 | (NM_117178) 98b like protein [Arabidopsis thaliana] p | PP | LL | LN | | | |
| 371 | 17737 | 0 | 86 | 15240924 | (NM_122624) RING-H2 zinc finger protein-like [Arabidopsis thaliana] | PP | LN | | | | |
| 372 | 18395 | 0 | 84 | 18401775 | (NM_128415) putative AP2 domain transcription factor [Arabidopsis thaliana] | SS | PP | LN | | | |
| 373 | 72772 | 0 | 100 | 15226228 | (NM_128328) putative cytochrome P450 [Arabidopsis thaliana] | HS | SP | PP | LN | | |
| 374 | 19441 | 0 | 85 | 9294477 | (AB018114) RING finger protein-like [Arabidopsis thaliana] | PP | PEG | | | | |
| 375 | 10486 | 1.00E-99 | 100 | 15237535 | (NM_120465) Terminal flower1 (TFL1) [Arabidopsis thaliana] | PP | | | | | |
| 376 | 11409 | 1.00E-133 | 100 | 68888 | trichome differentiation protein GL1 - Arabidopsis thaliana | CS | LN | SS | PP | | |
| 377 | 12104 | 1.00E-132 | 92 | 15230178 | AF488577_1 (AF488577) putative bHLH transcription factor [Arabidopsis thaliana] | PP | | | | | |
| 378 | 12258 | 1.00E-22 | 59 | 21554390 | arabinogalactan-protein [Arabidopsis thaliana] | PP | | | | | |
| 379 | 12909 | 0 | 100 | 18398696 | (NM_111831) expressed protein [Arabidopsis thaliana] | PP | | | | | |
| 380 | 14310 | 0 | 96 | 15226784 | (NM_129655) unknown protein [Arabidopsis thaliana] | PP | | | | | |
| 381 | 14317 | 0 | 100 | 18395560 | (NM_126399) expressed protein [Arabidopsis thaliana] | PP | | | | | |
| 382 | 14709 | 0 | 91 | 15219676 | (NM_100303) putative beta-ketoacyl-CoA synthase [Arabidopsis thaliana] pir\|\|T00951 probable 3-oxoacyl-[acyl-carrier-protein] synthase (EC 2.3.1.41) F20D22.1 | PP | | | | | |
| 383 | 15123 | 0 | 97 | 15238451 | (NM_120596) putative protein [Arabidopsis thaliana] | PP | | | | | |
| 384 | 16013 | 4.00E-91 | 85 | 15241799 | (NM_125629) ripening-related protein - like [Arabidopsis thaliana] | PP | | | | | |
| 385 | 16185 | 0 | 95 | 18420375 | (NM_120069) cysteine proteinase RD19A [Arabidopsis thaliana] PP | | | | | | |
| 386 | 16719 | 0 | 100 | 18401703 | (NM_103632) expressed protein [Arabidopsis thaliana] | PP | | | | | |
| 387 | 17490 | 8.00E-93 | 92 | 18405248 | (NM_104392) expressed protein [Arabidopsis thaliana] | PP | | | | | |
| 388 | 17905 | 8.00E-75 | 69 | 18404002 | (NM_113306) PHD-finger protein, putative [Arabidopsis thaliana] | PP | | | | | |
| 389 | 18385 | 1.00E-117 | 100 | 15223626 | (NM_104559) integral membrane protein, putative [Arabidopsis thaliana] | PP | | | | | |
| 390 | 18392 | 0 | 95 | 15227193 | (NM_127194) putative homeodomain transcription factor [Arabidopsis thaliana] | SS | PP | | | | |
| 392 | 18531 | 1.00E-142 | 100 | 15242792 | (NM_125746) putative protein [Arabidopsis thaliana] | PP | | | | | |
| 393 | 18603 | 0 | 94 | 18415840 | (NM_118332) alcohol dehydrogenase like protein [Arabidopsis thaliana] | PP | | | | | |
| 394 | 19530 | 0 | 96 | 15242217 | (NM_122138) Ruv DNA-helicase-like protein [Arabidopsis thaliana] | PP | | | | | |
| 395 | 70202 | 0 | 61 | 15241293 | (NM_121408) putative protein [Arabidopsis thaliana] | HS | PP | | | | |
| 396 | 72009 | 0 | 91 | 6319543 | (NC_001134) Amino acid transport protein for valine, leucine, isoleucine, and tyrosine; Tat1p [Saccharomyces cerevisiae] | PP | | | | | |
| 397 | 72119 | 3.00E-70 | 57 | 126078 | LATE EMBRYOGENESIS ABUNDANT PROTEIN D-34 (LEA D-34) | PP | | | | | |
| 398 | 10188 | 0 | 92 | 15228011 | (NM_129846) putative cytochrome P450 [Arabidopsis thaliana] | DS | | | | | |
| 399 | 10404 | 1.00E-151 | 94 | 99713 | homeotic protein agamous -Arabidopsis thaliana | DS | | | | | |
| 400 | 11333 | 1.00E-145 | 100 | 7207994 | (AF083220) proliferating cellular nuclear antigen [Arabidopsis thaliana] | DS | | | | | |
| 401 | 11719 | 0 | 87 | 15240257 | (NM_126126) cyclin D3-like protein [Arabidopsis thaliana] | DS | | | | | |
| 402 | 13663 | 1.00E-152 | 94 | 15227497 | (NM_129769) unknown protein [Arabidopsis thaliana] | DS | | | | | |
| 403 | 13958 | 0 | 96 | 15222885 | (NM_101226) aminoalcoholphosphotransferase [Arabidopsis thaliana] | SP | DS | | | | |
| 404 | 15214 | 0 | 92 | 15223772 | (NM_106341) Tub family protein, putative [Arabidopsis thaliana] | DS | | | | | |
| 405 | 10483 | 2.00E-86 | 100 | 15223944 | (NM_100757) superoxidase dismutase [Arabidopsis thaliana] | SP | DS | | | | |
| 406 | 11711 | 0 | 100 | 15234217 | (NM_19505) 2-dehydro-3-deoxyphosphoheptonate aldolase [Arabidopsis thaliana] | DS | | | | | |
| 407 | 11909 | 1.00E-126 | 88 | 99735 | L-ascorbate peroxidase (EC 1.11.1.11) precursor - Arabidopsis thaliana (fragment) | DS | | | | | |
| 408 | 12216 | 0 | 100 | 15236949 | (NM_118837) putative protein [Arabidopsis thaliana] | DS | | | | | |
| 409 | 12236 | 2.00E-55 | 100 | 15231278 | pollen specific protein -related [Arabidopsis thaliana] | DS | | | | | |
| 410 | 12256 | 0 | 100 | 2317731 | (AF013628) reversibly glycosylated polypeptide-2 [Arabidopsis thaliana] | DS | | | | | |
| 411 | 12806 | 1.00E-157 | 86 | 15235640 | (NM_119926) putative protein [Arabidopsis thaliana] | DS | | | | | |
| 412 | 12904 | 0 | 96 | 15239631 | BAA97512.1\|(AB026634)3'(2'), 5'-bisphosphate nucleotidase protein-like protein [Arabidopsis thaliana] | SP | DS | | | | |
| 413 | 13212 | 4.00E-74 | 93 | 15236917 | (AL161566) putative protein [Arabidopsis thaliana] | DS | | | | | |
| 414 | 13232 | 5.00E-30 | 60 | 15223263 | expressed protein [Arabidopsis thaliana] gi\|7485996\|pir\|\|T00711 | DS | | | | | |
| 415 | 13912 | 9.00E-68 | 100 | 18406846 | O64644\|SP18_ARATH Probable Sin3 associated polypeptide [Arabidopsis thaliana] | DS | | | | | |
| 416 | 14327 | 0 | 92 | 15221444 | (NM_102795) putative GTP-binding protein [Arabidopsis thaliana] | DS | | | | | |
| 417 | 14704 | 3.00E-71 | 46 | 17228240 | (NC_003272) hypothetical protein [Nostoc sp. PCC 7120] | DS | | | | | |
| 418 | 14714 | 0 | 94 | 15219541 | (NM_106032) ethylene-insensitive3-like3 (EIL3) [Arabidopsis thaliana] | DS | | | | | |
| 419 | 15142 | 0 | 74 | 15235217 | (NM_118107) putative protein [Arabidopsis thaliana] | SP | DS | | | | |
| 420 | 17450 | 1.00E-169 | 100 | 15232066 | AAF26152.1\|AC008261_9 (AC008261) putative homeobox-leucine zipper protein, HAT7 [Arabidopsis thaliana] | DS | | | | | |
| 421 | 18607 | 1.00E-151 | 94 | 15221373 | (NM_105503) putative transcription factor [Arabidopsis thaliana] | DS | | | | | |
| 422 | 19409 | 0 | 73 | 15241667 | (NM_120281) putative homeodomain protein [Arabidopsis thaliana] | DS | | | | | |
| 423 | 19412 | 0 | 96 | 15228826 | (NM_116132) putative protein [Arabidopsis thaliana] | DS | | | | | |
| 424 | 13005 | 0 | 99 | 15239405 | (NM_122447) cyclin 3a [Arabidopsis thaliana] gb\|AAC98445.1\| (AC006258) cyclin 3a [Arabidopsis thaliana] | SP | SS | PEG | | | |
| 425 | 10203 | 0 | 81 | 18405485 | (NM_104444) expressed protein [Arabidopsis thaliana] | SP | | | | | |
| 426 | 11327 | 0 | 92 | 12643807 | Protein farnesyltransferase alpha subunit (CAAX farnesyltransferase alpha subunit) (RAS proteins prenyltransferase alpha) (FTase-alpha)[Arabidopsis thaliana] | SP | | | | | |
| 427 | 12018 | 1.00E-175 | 96 | 18404664 | (NM_129374) expressed protein [Arabidopsis thaliana] | LL | | | | | |
| 427 | 11814 | 1.00E-175 | 96 | 18404664 | (NM_129374) expressed protein [Arabidopsis thaliana] | SP | | | | | |
| 428 | 13003 | 1.00E-169 | 85 | 18394319 | (NM_101474) expressed protein [Arabidopsis thaliana] | SP | | | | | |
| 429 | 13949 | 1.00E-160 | 85 | 18399097 | (NM_103124) expressed protein [Arabidopsis thaliana] | SP | | | | | |
| 430 | 16416 | 1.00E-161 | 94 | 15236283 | (NM_116570) putative chloroplast protein import component [Arabidopsis thaliana] | SP | | | | | |
| 431 | 16438 | 1.00E-167 | 78 | 18403775 | (AC004667) expressed protein [Arabidopsis thaliana] gb\|AAM62820.1\| (AY085599) zinc finger protein Glo3-like [Arabidopsis thaliana] | SP | | | | | |
| 432 | 17124 | 0 | 100 | 15221491 | (NM_104934) similar to flavin-containing monooxygenase (sp\|P36366); similar to ESTs gb\|R30018, gbIH36886, gbIN37822, and gb\|T88100 [Arabidopsis thaliana] | SP | | | | | |
| 433 | 19132 | 0 | 92 | 18396094 | (NM_111084) expressed protein [Arabidopsis thaliana] | SP | | | | | |
| 434 | 17922 | 1.00E-134 | 83 | 7485939 | AAC13593.1\| (AF058914) contains similarity to Arabidopsis thaliana DNA-damage-repair/tolerance resistance protein DRT111 (SW:P42698) | LL | SP | CS | | | |
| 435 | 19719 | 1.00E-141 | 72 | 6692816 | (AB036735) allyl alcohol dehydrogenase [Nicotiana tabacum] | PEG | SP | HS | | | |
| 436 | 14274 | 8.00E-90 | 100 | 18407428 | (NM_130339) expressed protein [Arabidopsis thaliana] | SP | | | | | |
| 436 | 17336 | 8.00E-90 | 100 | 18407428 | (NM_130339) expressed protein [Arabidopsis thaliana] | SP | LL | | | | |
| 437 | 17735 | 1.00E-108 | 91 | 18404601 | (NM_129353) expressed protein [Arabidopsis thaliana] | SP | LL | | | | |
| 438 | 19249 | 5.00E-43 | 100 | 21553354 | glycine-rich RNA binding protein 7 [Arabidopsis thaliana] | PP | SP | LN | | | |
| 439 | 18513 | 0 | 96 | 15226492 | (NM_130274) putative protein kinase [Arabidopsis thaliana] pir\|\|T02181 protein kinase homolog F14M4.11 | SP | PP | SS | | | |
| 440 | 11517 | 1.00E-153 | 85 | 18412044 | (NM_106509) expressed protein [Arabidopsis thaliana] | SP | | | | | |
| 441 | 12363 | 0 | 100 | 15242458 | (NM_123934) GDSL-motif lipase/hydrolase-like protein [Arabidopsis thaliana] | SP | | | | | |
| 442 | 12922 | 7.00E-81 | 100 | 18403216 | (NM_128881) expressed protein [Arabidopsis thaliana] | SP | | | | | |
| 443 | 15360 | 1.00E-152 | 89 | 18398108 | (NM_111674) expressed protein [Arabidopsis thaliana] | SP | | | | | |
| 444 | 16028 | 0 | 100 | 15232435 | (NM_115274) peptide transport -like protein [Arabidopsis thaliana] | SP | | | | | |
| 445 | 16648 | 1.00E-134 | 100 | 15891409 | NP_534027.1\| (NC_003305) 3-oxoacyl-(acyl-carrier-protein) reductase [Agrobacterium tumefaciens str. C58 (U. Washington)] | SP | | | | | |
| 446 | 16705 | 0 | 95 | 15236458 | (NM_116899) nodulin-like protein [Arabidopsis thaliana] | SP | | | | | |
| 447 | 16715 | 0 | 96 | 15238198 | (NM_120537) putative protein [Arabidopsis thaliana] | SP | | | | | |
| 448 | 17316 | 1.00E-124 | 95 | 18378907 | (NM_100079) expressed protein [Arabidopsis thaliana] | SP | | | | | |
| 449 | 17331 | 0 | 85 | 15220100 | (NM_106680) putative sulfate transporter [Arabidopsis thaliana] | SP | | | | | |
| 450 | 17339 | 2.00E-79 | 100 | 15228208 | (NM_114633) putative protein [Arabidopsis thaliana] | SP | | | | | |
| 451 | 17420 | 1.00E-124 | 94 | 15229782 | (NM_114248) glutathione transferase-like protein [Arabidopsis thaliana] | SP | | | | | |
| 452 | 17446 | 1.00E-144 | 88 | 15230344 | (NM_115620) AP2 transcription factor - like protein [Arabidopsis thaliana] | SP | | | | | |
| 453 | 17487 | 1.00E-71 | 78 | 15218649 | (NM_102603) ethylene-responsive element binding factor, putative [Arabidopsis thaliana] | SP | | | | | |
| 454 | 17740 | 0 | 97 | 15232593 | (NM_114527) scarecrow-like protein [Arabidopsis thaliana] | SP | | | | | |
| 455 | 17752 | 1.00E-176 | 94 | 9755372 | (AC000107) F17F8.3 [Arabidopsis thaliana] | SP | | | | | |
| 456 | 18021 | 0 | 96 | 7262677 | (AC012188) Contains similarity to MYB-Related Protein B from Gallus gallus [Arabidopsis thaliana] | SP | | | | | |
| 457 | 18245 | 1.00E-168 | 86 | 15239503 | (NM_122484) GATA transcription factor - like [Arabidopsis thaliana] | SP | | | | | |
| 458 | 18617 | 3.00E-69 | 94 | 18424873 | (NM_125879) expressed protein [Arabidopsis thaliana] | SP | | | | | |
| 459 | 18734 | 0 | 96 | 15237253 | (NM_121609) UVB-resistance protein-like [Arabidopsis thaliana] | SP | | | | | |
| 460 | 18823 | 0 | 82 | 15222227 | AAM62510.1\| (AY085278) homeodomain protein BELL1, putative [Arabidopsis thaliana] | SP | | | | | |
| 461 | 19222 | 0 | 100 | 18390636 | (NM_100509) expressed protein [Arabidopsis thaliana] | PP | SP | | | | |
| 462 | 19430 | 0 | 95 | 18405149 | (NM_129533) expressed protein [Arabidopsis thaliana] | SP | | | | | |
| 463 | 12332 | 1.00E-113 | 84 | 15221408 | (NM_106142) myb-related transcription activator, putative [Arabidopsis thaliana] | ss | | | | | |
| 464 | 13649 | 1.00E-127 | 92 | 11281134 | hypothetical protein F9G14.50 -Arabidopsis thaliana | SS | | | | | |
| 465 | 16113 | 0 | 97 | 15217485 | AAD18098.1\| (AC006416) Identical to gb\|Y10557 g5bf gene from Arabidopsis thaliana putative RNA-binding protein [Arabidopsis thaliana] | SS | | | | | |
| 466 | 12069 | 1.00E-63 | 100 | 18410081 | (NM_105902) expressed protein [Arabidopsis thaliana] | SS | | | | | |
| 467 | 12906 | 0 | 98 | 5915825 | Cytochrome P450 71B2 dbj\|BAA28537.1\| (D78605) cytochrome P450 monooxygenase [Arabidopsis thaliana] | SS | | | | | |
| 468 | 13443 | 1.00E-111 | 100 | 18409105 | (NM_114908) expressed protein [Arabidopsis thaliana] | SS | | | | | |
| 469 | 14707 | 0 | 96 | 13122288 | (AB047808) proteasel (pfpl)-like protein [Arabidopsis thaliana] | SS | | | | | |
| 470 | 15116 | 1.00E-167 | 100 | 15242465 | (NM_121002) inorganic pyrophosphatase - like protein [Arabidopsis thaliana] | SS | | | | | |
| 471 | 16117 | 1.00E-90 | 78 | 15227349 | (NM_129704) calmodulin-like protein [Arabidopsis thaliana] | SS | | | | | |
| 472 | 16136 | 1.00E-115 | 92 | 15222919 | (NM_101236) unknown protein [Arabidopsis thaliana] | SS | | | | | |
| 473 | 19077 | 8.00E-98 | 70 | 15221874 | (NM_101737) hypothetical protein [Arabidopsis thaliana] | HS | PP | SS | | | |
| 474 | 19178 | 0 | 95 | 6321456 | (NC_001139) gamma-aminobutyrate (GABA) transaminase (4-aminobutyrate aminotransferase); Uga1p [Saccharomyces cerevisiae] | CK | HS | PEG | PP | SS | |
| 475 | 70752 | 4.00E-46 | 100 | 15224299 | trypsin inhibitor -related [Arabidopsis thaliana] gi\|3287862\|sp\|O22867\|ITI5_ARAT H | SS | | | | | |
| 476 | 70753 | 4.00E-86 | 100 | 15231204 | (NM_112176) DnaJ protein, putative [Arabidopsis thaliana] | SS | | | | | |
| 477 | 70809 | 6.00E-70 | 48 | 20503004 | (AC098693) Hypothetical protein [Oryza sativa (japonica cultivar-group)] | LL | PP | SS | | | |
| 478 | 72091 | 1.00E-177 | 94 | 6322655 | (NC_001143) Interacts with and may be a positive regulator of GLC7 which encodes type1 protein phosphatase; Sds22p [Saccharomyces cerevisiae] | LL | LN | SS | | | |

### Trait Improvement Screens

SD- Improvement of drought tolerance identified by soil drought stress tolerance screen: Drought or water deficit conditions impose mainly osmotic stress on plants. Plants are particularly vulnerable to drought during the flowering stage. The drought condition in the screening process disclosed in Example 1B started from the flowering time and was sustained to the end of harvesting. The present invention provides recombinant DNA that can improve the plant survival rate under such sustained drought condition. Exemplary recombinant RNA for conferring such drought tolerance are identified as such in Table 3. Such recombinant RNA may find particular use in generating transgenic plants that are tolerant to the drought condition imposed during flowering time and in other stages of the plant life cycle. As demonstrated from the model plant screen, in some embodiments of transgenic plants with trait-improving recombinant DNA grown under such sustained drought condition can also have increased total seed weight per plant in addition to the increased survival rate within a transgenic population, providing a higher yield potential as compared to control plants.

PEG-Improvement of drought tolerance identified by PEG induced osmotic stress tolerance screen: Various drought levels can be artificially induced by using various concentrations of polyethylene glycol (PEG) to produce different osmotic potentials (Pilon-Smits et al. (1995) Plant Physiol. 107:125-130). Several physiological characteristics have been reported as being reliable indications for selection of plants possessing drought tolerance. These characteristics include the rate of seed germination and seedling growth. The traits can be assayed relatively easily by measuring the growth rate of seedling in PEG solution. Thus, a PEG-induced osmotic stress tolerance screen is a useful surrogate for drought tolerance screen As demonstrated from the model plant screen, embodiments of transgenic plants with trait-improving recombinant DNA identified in a PEG-induced osmotic stress tolerance screen can survive better drought conditions providing a higher yield potential as compared to control plants.

SS -Improvement of drought tolerance identified by high salinity stress tolerance screen: Three different factors are responsible for salt damages: (1) osmotic effects, (2) disturbances in the mineralization process, (3) toxic effects caused by the salt ions, e.g. inactivation of enzymes. While the first factor of salt stress results in the wilting of the plants that is similar to drought effect, the ionic aspect of salt stress is clearly distinct from drought. The present invention provides genes that help plants to maintain biomass, root growth, and/or plant development in high salinity conditions, which are identified as such in Table 3. Since osmotic effect is one of the major component of salt stress, which is common to the drought stress, trait-improving recombinant DNA identified in a high salinity stress tolerance screen can provide transgenic crops with improved drought tolerance..

HS-Improvement of drought tolerance identified by heat stress tolerance screen: Heat and drought stress often occur simultaneously, limiting plant growth. Heat stress can cause the reduction in photosynthesis rate, inhibition of leaf growth and osmotic potential in plants. Thus, genes identified by the present invention as heat stress tolerance conferring genes may also impart improved drought tolerance to plants.

CK and CS-Improvement of tolerance to cold stress: Low temperature may immediately result in mechanical constraints, changes in activities of macromolecules, and reduced osmotic potential. In the present invention, two screening conditions, i.e. cold shock tolerance screen (CK) and cold germination tolerance screen (CS), were set up to look for transgenic plants that display visual growth advantage at lower temperature. In cold germination tolerance screen, the transgenic *Arabidopsis* plants were exposed to a constant temperature of 8°C from planting until day 28 post planting. The recombinant nucleotides identified by such screen as cold stress tolerance conferring genes are particular useful for the production of transgenic plant that can germinate more robustly in a cold temperature as compared to the wild type plants. In cold shock tolerance screen, the transgenic plants were first grown under the normal growth temperature of 22°C until day 8 post planting, and subsequently were placed under 8°C until day 28 post planting. In some preferred embodiments, transgenic plants transformed with the recombinant DNA constructs comprising SEQ ID NO: 1 or SEQ ID NO: 2 display more robust growth in both cold tolerance screens.

Improvement of tolerance to multiple stresses: Different kinds of stresses often lead to identical or similar reaction in the plants. Genes that are activated or inactivated as a reaction to stress can either act directly in a way the genetic product reduces a specific stress, or they can act indirectly by activating other specific stress genes. By manipulating the activity of such regulatory genes, i.e. multiple stress tolerance genes, the plant can be enabled to react to different kinds of stresses. For examples, SEQ ID NO: 37, SEQ ID NO: 38 and SEQ ID NO: 128 can be used to improve both heat stress tolerance and cold stress tolerance in plants. Of particular interest, plants transformed with SEQ ID NO: 59 can resist heat stress, salt stress and cold stress. In addition to these multiple stress tolerance genes, the stress tolerance conferring genes provided by the present invention may be used in combinations to generate transgenic plants that can resist multiple stress conditions.

### PP-Improvement of early plant growth and development.

It has been known in the art that to minimize the impact of disease on crop profitability, it is important to start the season with healthy vigorous plants. This means avoiding seed and seedling diseases, leading to increased nutrient uptake and increased yield potential. Traditionally early planting and applying fertilizer are the methods used for promoting early seedling vigor. In early development stage, plant embryos establish only the basic root-shoot axis, a cotyledon storage organ(s), and stem cell populations, called the root and shoot apical meristems, that continuously generate new organs throughout post-embryonic development. "Early growth and development " used herein encompasses the stages of seed imbibition through the early vegetative phase. The present invention provides genes that are useful to produce transgenic plants that have advantages in one or more processes including, but not limited to, germination, seedling vigor, root growth and root morphology under non-stressed conditions. The transgenic plants starting from a more robust seedling are less susceptible to the fungal and bacterial pathogens that attach germinating seeds and seedling. Furthermore, seedlings with advantage in root growth are more resistant to drought stress due to extensive and deeper root architecture. Therefore, it can be recognized by those skilled in the art that genes conferring the growth advantage in early stages to plants may also be used to generate transgenic plants that are more resistant to various stress conditions due to improved early plant development. The present invention provides such exemplary genes that confer both the stress tolerance and growth advantages to plants, identified as such in Table 3, e.g. SEQ ID NO: 128 which can improve the plant early growth and development and impart heat and cold tolerance to plants.

### SP-Improvement of late plant growth and development

"Late growth and development" used herein encompasses the stages of leaf development, flower production, and seed maturity. In certain embodiments, transgenic plants produced using genes that confer growth advantages to plants provided by the present invention, identified as such in Table 3, exhibit at least one phenotypic characteristics including, but not limited to, increased rosette radius, increased rosette dry weight, seed dry weight, silique dry weight, and silique length. On one hand, the rosette radius and rosette dry weight are used as the indexes of photosynthesis capacity, and thereby plant source strength and yield potential of a plant. On the other hand, the seed dry weight, silique dry weight and silique length are used as the indexes for plant sink strength, which are considered as the direct determinants of yield.

### LL-Improvement of tolerance to shade stress

The effects of light on plant development are especially prominent at the seedling stage. Under normal light conditions with unobstructed direct light, a plant seeding develops according to a characteristic photomorphogenic pattern, in which plants have open and expanded cotyledons and short hypocotyls. Then the plant's energy is devoted to cotyledon and leaf development while longitudinal extension growth is minimized. Under low light condition where light quality and intensity are reduced by shading, obstruction or high population density, a seedling displays a shade-avoidance pattern, in which the seedling displays a reduced cotyledon expansion, and hypocotyls extension is greatly increased. As the result, a plant under low light condition increases significantly its stem length at the expanse of leaf, seed or fruit and storage organ development, thereby adversely affecting of yield. The present invention provides recombinant nucleotides that enable plants to have an attenuated shade avoidance response so that the source of plant can be contribute to reproductive growth efficiently, resulting higher yield as compared to the wild type plants. One skilled in the art can recognize that transgenic plants generated by the present invention may be suitable for a higher density planting, thereby resulting increased yield per unit area. In some preferred embodiments, the present invention provides transgenic plants that have attenuated low light response and advantage in the flower bud formation.

### LN-Improvement of tolerance to low nitrogen availability stress

Nitrogen is a key factor in plant growth and crop yield. The metabolism, growth and development of plants are profoundly affected by their nitrogen supply. Restricted nitrogen supply alters shoot to root ratio, root development, activity of enzymes of primary metabolism and the rate of senescence (death) of older leaves. All field crops have a fundamental dependence on inorganic nitrogenous fertilizer. Since fertilizer is rapidly depleted from most soil types, it must be supplied to growing crops two or three times during the growing season. Improved nitrogen use efficiency by plants should enable crops cultivated under low nitrogen availability stress condition resulted from low fertilizer input or poor soil quality.

According to the present invention, transgenic plants generated using the recombinant nucleotides, which confer improved nitrogen use efficiency, identified as such in Table 3, exhibit one or more desirable traits including, but not limited to, increased seedling weight, increased number of green leaves, increased number of rosette leaves, increased root length and advanced flower bud formation. One skilled in the art may recognize that the transgenic plants with improved nitrogen use efficiency, established by the present invention may also have altered amino acid or protein compositions, increased yield and/or better seed quality. The transgenic plants of the present invention may be productively cultivated under nitrogen nutrient deficient conditions, i.e. nitrogen-poor soils and low nitrogen fertilizer inputs, that would cause the growth of wild type plants to cease or to be so diminished as to make the wild type plants practically useless. The transgenic plants also may be advantageously used to achieve earlier maturing, faster growing, and/or higher yielding crops and/or produce more nutritious foods and animal feedstocks when cultivated using nitrogen non-limiting growth conditions.

### Stacked Traits

The present invention also encompasses transgenic plants with stacked engineered traits, e.g. a crop having an improved phenotype resulting from expression of a trait-improving recombinant DNA, in combination with herbicide and/or pest resistance traits. For example, genes of the current invention can be stacked with other traits of agronomic interest, such as a trait providing herbicide resistance, for example a RoundUp Ready trait, or insect resistance, such as using a gene from *Bacillus thuringensis* to provide resistance against lepidopteran, coliopteran, homopteran, hemiopteran, and other insects. Herbicides for which resistance is useful in a plant include glyphosate herbicides, phosphinothricin herbicides, oxynil herbicides, imidazolinone herbicides, dinitroaniline herbicides, pyridine herbicides, sulfonylurea herbicides, bialaphos herbicides, sulfonamide herbicides and gluphosinate herbicides. To illustrate that the production of transgenic plants with herbicide resistance is a capability of those of ordinary skill in the art, reference is made to U.S. patent application publications 2003/0106096A1 and 2002/0112260A1 and U.S. Patents 5,034,322; 5,776,760, 6,107,549 and 6,376,754, all of which are incorporated herein by reference. To illustrate that the production of transgenic plants with pest resistance is a capability of those of ordinary skill in the art reference is made to U.S. Patents 5,250,515 and 5,880,275 which disclose plants expressing an endotoxin of *Bacillus thuringiensis* bacteria, to U.S. Patent 6,506,599 which discloses control of invertebrates which feed on transgenic plants which express dsRNA for suppressing a target gene in the invertebrate, to U.S. Patent 5,986,175 which discloses the control of viral pests by transgenic plants which express viral replicase, and to U.S. Patent Application Publication 2003/0150017 A1 which discloses control of pests by a transgenic plant which express a dsRNA targeted to suppressing a gene in the pest, all of which are incorporated herein by reference.

Once one recombinant DNA has been identified as conferring an improved trait of interest in transgenic *Arabidopsis* plants, several methods are available for using the sequence of that recombinant DNA and knowledge about the protein it encodes to identify homologs of that sequence from the same plant or different plant species or other organisms, e.g. bacteria and yeast. Thus, in one aspect, the invention provides methods for identifying a homologous gene with a DNA sequence homologous to any of SEQ ID NO: 1 through SEQ ID NO: 151 and SEQ ID NO: 153 through SEQ ID NO: 239, or a homologous protein with an amino acid sequence homologous to any of SEQ ID NO: 240 through SEQ ID NO: 390 and SEQ ID NO: 392 through SEQ ID NO: 478. In another aspect, the present invention provides the protein sequences of identified homologs for a sequence listed as SEQ ID NO: 240 through SEQ ID NO: 390 and SEQ ID NO: 392 through SEQ ID NO: 478. In yet another aspect, the present invention also includes linking or associating one or more desired traits, or gene function with a homolog sequence provided herein.

The trait-improving recombinant DNA and methods of using such trait-improving recombinant DNA for generating transgenic plants with improved traits provided by the present invention are not limited to any particular plant species. Indeed, the plants according to the present invention may be of any plant species, i.e., may be monocotyledonous or dicotyledonous. Preferably, they will be agricultural useful plants, i.e., plants cultivated by man for purposes of food production or technical, particularly industrial applications. Of particular interest in the present invention are corn and soybean plants. The recombinant DNA constructs optimized for soybean transformation and corn transformation are provide by the present invention. Other plants of interest in the present invention for production of transgenic plants having improved traits include, without limitation, cotton, canola, wheat, sunflower, sorghum, alfalfa, barley, millet, rice, tobacco, fruit and vegetable crops, and turfgrass.

In certain embodiments, the present invention contemplates to use an orthologous gene in generating the transgenic plants with similarly improved traits as the transgenic *Arabidopsis* counterpart. Improved physiological properties in transgenic plants of the present invention may be confirmed in responses to stress conditions, for example in assays using imposed stress conditions to detect improved responses to drought stress, nitrogen deficiency, cold growing conditions, or alternatively, under naturally present stress conditions, for example under field conditions. Biomass measures may be made on greenhouse or field grown plants and may include such measurements as plant height, stem diameter, root and shoot dry weights, and, for corn plants, ear length and diameter.

Trait data on morphological changes may be collected by visual observation during the process of plant regeneration as well as in regenerated plants transferred to soil. Such trait data includes characteristics such as normal plants, bushy plants, taller plants, thicker stalks, narrow leaves, striped leaves, knotted phenotype, chlorosis, albino, anthocyanin production, or altered tassels, ears or roots. Other improved traits may be identified by measurements taken under field conditions, such as days to pollen shed, days to silking, leaf extension rate, chlorophyll content, leaf temperature, stand, seedling vigor, internode length, plant height, leaf number, leaf area, tillering, brace roots, stay green, stalk lodging, root lodging, plant health, barreness/prolificacy, green snap, and pest resistance. In addition, trait characteristics of harvested grain may be confirmed, including number of kernels per row on the ear, number of rows of kernels on the ear, kernel abortion, kernel weight, kernel size, kernel density and physical grain quality.

To confirm hybrid yield in transgenic corn plants expressing genes of the present invention, it may be desirable to test hybrids over multiple years at multiple locations in a geographical location where maize is conventionally grown, e.g. in Iowa, Illinois or other locations in the midwestem United States, under "normal" field conditions as well as under stress conditions, e.g. under drought or population density stress.

Transgenic plants can be used to provide plant parts according to the invention for regeneration or tissue culture of cells or tissues containing the constructs described herein. Plant parts for these purposes can include leaves, stems, roots, flowers, tissues, epicotyl, meristems, hypocotyls, cotyledons, pollen, ovaries, cells and protoplasts, or any other portion of the plant which can be used to regenerate additional transgenic plants, cells, protoplasts or tissue culture. Seeds of transgenic plants are provided by this invention can be used to propagate more plants containing the trait-improving recombinant DNA constructs of this invention. These descendants are intended to be included in the scope of this invention if they contain a trait-improving recombinant DNA construct of this invention, whether or not these plants are selfed or crossed with different varieties of plants.

The various aspects of the invention are illustrated by means of the following examples which are in no way intended to limit the full breath and scope of claims.

### EXAMPLES

### Example 1. Identification of recombinant DNA that confers improved trait(s) to plants A. Expression constructs for Arabidopsis plant transformation

Each gene of interest was amplified from a genomic or cDNA library using primer specific to sequences upstream and downstream of coding region. Transformation vectors were prepared to constitutively transcribe DNA in either sense orientation (for enhanced protein expression) or anti-sense orientation (for endogenous gene suppression) under the control of an enhanced Cauliflower Mosaic Virus 35S promoter (U.S. patent 5,359,142) directly or indirectly (Moore et al. PNAS 95:376-381, 1998; Guyer et al. Genetics 149: 633-639, 1998; International patent application NO. PCT/EP98/07577). The transformation vectors also contain a *bar* gene as a selectable marker for resistance to glufosinate herbicide. The transformation of *Arabidopsis* plants was carried out using the vacuum infiltration method known in the art (Bethtold et al. Methods Mol. Biol. 82:259-66, 1998). Seeds harvested from the plants, named as T 1 seeds, were subsequently were grown in a glufosinate-containing selective medium to select for plants which were actually transformed and which produced T2 transgenic seed.. For first pass screening T2 seeds from five independent transgenic lines of *Arabidopsis* were

### B. Soil Drought tolerance screen

This example describes a soil drought tolerance screen to identify *Arabidopsis* plants transformed with recombinant DNA that wilt less rapidly and/or produce higher seed yield when grown in soil under drought conditions

T2 seeds were sown in flats filled with Metro/Mix^{®} 200 (The Scotts^{®} Company, USA). Humidity domes were added to each flat and flats were assigned locations and placed in climate-controlled growth chambers. Plants were grown under a temperature regime of 22°C at day and 20°C at night, with a photoperiod of 16 hours and average light intensity of 170 µmol/m²/s. After the first true leaves appeared, humidity domes were removed. The plants were sprayed with glufosinate herbicide and put back in the growth chamber for 3 additional days. Flats were watered for 1 hour the week following the herbicide treatment. Watering was continued every seven days until the flower bud primordia became apparent, at which time plants were watered for the last time.

To identify drought tolerant plants, plants were evaluated for wilting response and seed yield. Beginning ten days after the last watering, plants were examined daily until 4 plants/line had wilted. In the next six days, plants were monitored for wilting response. Five drought scores were assigned according to the visual inspection of the phenotypes: 1 for healthy, 2 for dark green, 3 for wilting, 4 severe wilting, and 5 for dead. A score of 3 or higher was considered as wilted.

At the end of this assay, seed yield measured as seed weight per plant under the drought condition was characterized for the transgenic plants and their controls and analyzed as a quantitative response according to example 1M.
Two approaches were used for statistical analysis on the wilting response. First, the risk score was analyzed for wilting phenotype and treated as a qualitative response according to the example 1L. Alternatively, the survival analysis was carried out in which the proportions of wilted and non-wilted transgenic and control plants were compared over each of the six days under scoring and an overall log rank test was performed to compare the two survival curves using S-PLUS statistical software (S-PLUS 6, Guide to statistics, Insightful, Seattle, WA, USA). Table 4 provides a list of recombinant DNA constructs that improve drought tolerance in transgenic plants.

**Table 4**

| **Pep SEQ ID** | **Construct_id** | **Orientation** | **Wilt Response Risk score** | | | **Seed Weight/plant** | | | **Survival Anaysis of wilt response** | | |
|---|---|---|---|---|---|---|---|---|---|---|---|
| | | | **RS mean** | **p-value** | **c** | **delta** | **p-value** | **c** | **diff time to wilting** | **p-value** | **c** |
| 241 | 74518 | SENSE | -0.131 | 0.985 | / | 1.26 | 0 | S | 0 | 1 | / |
| 290 | 70222 | SENSE | -0.032 | 0.726 | / | 0.461 | 0.001 | S | -0.63 | 0.21 | / |
| 307 | 18280 | SENSE | -0.066 | 0.937 | / | 0.402 | 0.004 | S | 0 | 1 | / |
| 357 | 70247 | SENSE | 0.11 | 0.169 | T | 0.336 | 0.006 | S | -0.57 | 0.134 | / |
| 369 | 72471 | SENSE | 0.16 | 0.038 | S | -0.053 | 0.546 | / | 0.16 | 0.226 | / |
| 398 | 10188 | ANTI-SENSE | 0.133 | 0.004 | S | 0.68 | 0 | S | 0.24 | 0.297 | / |
| 399 | 10404 | ANTI-SENSE | 0.13 | 0.068 | T | 0.2 | 0.271 | / | 0.57 | 0.083 | T |
| 400 | 11333 | ANTI-SENSE | 0.266 | 0.007 | S | 0.291 | 0.293 | / | 0.77 | 0.131 | T |
| 401 | 11719 | ANTI-SENSE | 0.56 | 0.006 | S | -0.088 | 0.751 | / | 0 | 1 | / |
| 402 | 13663 | ANTI-SENSE | 0.123 | 0.024 | S | -0.198 | 0.763 | / | 0.04 | 0.852 | / |
| 403 | 13958 | ANTI-SENSE | 0.526 | 0.001 | S | 0.518 | 0.08 | T | 0 | 1 | / |
| 404 | 15214 | ANTI-SENSE | 0.018 | 0.208 | / | 0.19 | 0.243 | / | 0.06 | 0.815 | / |
| 405 | 10483 | SENSE | 0.313 | 0.012 | S | -0.095 | 0.795 | / | 0.28 | 0.358 | / |
| 406 | 11711 | SENSE | 0.346 | 0.005 | S | 0.218 | 0.009 | S | 0.3 | 0.371 | / |
| 407 | 11909 | SENSE | 0.094 | 0.021 | S | 0.002 | 0.493 | / | 0.26 | 0.767 | / |
| 408 | 12216 | SENSE | 0.623 | 0 | S | -0.195 | 0.714 | / | 2.55 | 0.007 | S |
| 409 | 12236 | SENSE | 0.233 | 0.019 | S | 0.32 | 0.026 | S | 0.29 | 0.124 | T |
| 410 | 12256 | SENSE | 0.254 | 0.001 | S | 0.133 | 0.245 | / | 0.09 | 0.869 | / |
| 411 | 12806 | SENSE | 0.198 | 0.016 | S | 0.689 | 0.018 | S | 0.16 | 0.696 | / |
| 412 | 12904 | SENSE | 0.292 | 0.033 | S | -1.195 | 0.992 | / | 0.81 | 0.023 | S |
| 413 | 13212 | SENSE | 0.24 | 0.006 | S | 0.676 | 0.01 | S | 0.25 | 0.559 | / |
| 414 | 13232 | SENSE | 0.166 | 0.134 | T | -0.044 | 0.568 | / | 0.81 | 0.105 | T |
| 415 | 13912 | SENSE | 0.3 | 0 | S | -0.084 | 0.74 | / | 0.91 | 0.054 | T |
| 416 | 14327 | SENSE | 0.181 | 0.008 | S | -0.423 | 0.831 | / | 0.75 | 0.021 | S |
| 417 | 14704 | SENSE | 0.174 | 0.01 | S | 0.26 | 0.003 | S | 0.92 | 0.538 | / |
| 418 | 14714 | SENSE | 0.313 | 0.007 | S | -0.283 | 0.987 | / | 0.3 | 0.702 | / |
| 419 | 15142 | SENSE | 0.28 | 0 | S | -0.498 | 0.871 | / | 0.29 | 0.196 | T |
| 420 | 17450 | SENSE | 0.117 | 0.102 | T | 0.238 | 0.079 | T | -0.05 | 0.834 | / |
| 421 | 18607 | SENSE | 0.161 | 0.013 | S | -0.167 | 0.782 | / | 0.64 | 0.034 | S |
| 422 | 19409 | SENSE | 0.177 | 0.032 | S | 0.33 | 0.119 | T | 0.36 | 0.298 | / |
| 423 | 19412 | SENSE | 0.501 | 0.001 | S | -0.006 | 0.515 | / | 0.15 | 0.84 | / |

| | | | | | | | | | | | |
|---|---|---|---|---|---|---|---|---|---|---|---|
| S: represents that the transgenic plants showed statistically significant trait improvement as compared to the reference (p<0.05, p value, of the delta of a quantitative response or of the risk score of a qualitative response, is the probability that the observed difference between the transgenic plants and the reference occur by chance) T: represents that the transgenic plants showed a trend of trait improvement as compared to the reference, preferably with p<0.2 , /: represents the transgenic plants didn't show any alteration or had unfavorable change in traits examined compared to the reference in the current dataset. | | | | | | | | | | | |

### C. Heat stress tolerance screen

Under high temperatures, *Arabidopsis* seedlings become chlorotic and root growth is inhibited. This example sets forth the heat stress tolerance screen to identity *Arabidopsis* plants transformed with the gene of interest that are more resistant to heat stress based on primarily their seedling weight and root growth under high temperature. T2 seeds were plated on 1/2 X MS salts, 1/% phytagel, with 10 µg/ml glufosinate (7 per plate with 2 control seeds; 9 seeds total per plate). Plates were placed at 4°C for 3 days to stratify seeds. Plates were then incubated at room temperature for 3 hours and then held vertically for 11 additional days at temperature of 34°C at day and 20°C at night. Photoperiod was 16 h. Average light intensity was ~140 µmol/m²/s. After 14 days of growth, plants were scored for glufosinate resistance, root length, final growth stage, visual color, and seedling fresh weight. A photograph of the whole plate was taken on day 14.

Visual assessment was carried out to evaluate the robustness of the growth based on the leave size and rosette size.

The seedling weight and root length were analyzed as quantitative responses according to example 1M. The final grow stage at day 14 was scored as success if 50% of the plants had reached 3 rosette leaves and size of leaves are greater than 1mm (Boyes et al. (2001) The Plant Cell 13, 1499-1510). The growth stage data was analyzed as a qualitative response according to example IL.Table 5 provides a list of recombinant DNA constructs that improve heat tolerance in transgenic plants.

**Table 5**

| **Pep SEQ ID** | **Construct_id** | **Orientation** | **seedling weight** | | | **Root Length** | | | **growth stage** | | |
|---|---|---|---|---|---|---|---|---|---|---|---|
| | | | **delta** | **p-value** | **c** | **delta** | **p-value** | **c** | **RS mean** | **p-value** | **c** |
| 268 | 12007 | ANTI-SENSE | 1.283 | 0 | S | 0.221 | 0.018 | S | 0.844 | 0.044 | S |
| 269 | 12290 | ANTI-SENSE | 0.92 | 0.002 | S | -0.09 | 0.683 | / | 0.4 | 0.14 | T |
| 270 | 12343 | ANTI-SENSE | 1.186 | 0 | S | 0.008 | 0.478 | / | -0.066 | 0.818 | / |
| 271 | 14348 | ANTI-SENSE | 0.917 | 0 | S | 0.047 | 0.352 | / | 0.034 | 0.314 | / |
| 272 | 15708 | ANTI-SENSE | 1.12 | 0 | S | 0.122 | 0.092 | T | 0.467 | 0.016 | S |
| 273 | 17615 | ANTI-SENSE | 1.134 | 0 | S | 0.176 | 0.084 | T | 0.541 | 0.102 | T |
| 274 | 17622 | ANTI-SENSE | 0.728 | 0 | S | -0.142 | 0.874 | / | 0.875 | 0.002 | S |
| 275 | 70714 | ANTI-SENSE | 1.029 | 0 | S | 0.032 | 0.355 | / | -0.003 | 0.515 | / |
| 276 | 17925 | SENSE | 0.969 | 0 | S | -0.027 | 0.588 | / | 0.22 | 0.215 | / |
| 277 | 18541 | SENSE | 0.977 | 0 | S | -0.012 | 0.559 | / | 0.982 | 0.028 | S |
| 278 | 11425 | SENSE | 1.255 | 0 | S | 0.152 | 0.096 | T | 0.516 | 0.005 | S |
| 279 | 12263 | SENSE | 0.869 | 0.003 | S | -0.023 | 0.552 | / | 0.481 | 0.113 | T |
| 280 | 12288 | ANTI-SENSE | 1.256 | 0 | S | 0.086 | 0.314 | / | 0.968 | 0.036 | S |
| 281 | 12910 | SENSE | 1.067 | 0.105 | T | 0.097 | 0.274 | / | -0.417 | 1 | / |
| 282 | 14335 | SENSE | 1.107 | 0 | S | 0.16 | 0 | S | -0.024 | 0.804 | / |
| 283 | 17427 | SENSE | 0.837 | 0 | S | -0.069 | 0.706 | / | 0.569 | 0.087 | T |
| 284 | 19140 | SENSE | 0.894 | 0 | S | 0.111 | 0.131 | T | 1.794 | 0 | S |
| 285 | 19179 | SENSE | 1.039 | 0 | S | -0.095 | 0.742 | / | 0.614 | 0.063 | T |
| 286 | 19251 | SENSE | 0.77 | 0 | S | -0.061 | 0.771 | / | 0.543 | 0.027 | S |
| 287 | 19443 | SENSE | 1.115 | 0 | S | 0.042 | 0.369 | / | 0.537 | 0.078 | T |
| 288 | 19607 | SENSE | 0.939 | 0 | S | 0.024 | 0.381 | / | 0.095 | 0.215 | / |
| 289 | 19915 | SENSE | 1.336 | 0.057 | T | 0.19 | 0.299 | / | 0.07 | 0 | S |
| 290 | 70222 | SENSE | 0.778 | 0.004 | S | -0.078 | 0.677 | / | 1.153 | 0.015 | S |
| 291 | 70464 | SENSE | 1.039 | 0 | S | 0.026 | 0.411 | / | 0.806 | 0.04 | S |
| 292 | 70474 | SENSE | 1.026 | 0 | S | 0.094 | 0.207 | / | 1.145 | 0.03 | S |
| 293 | 70484 | SENSE | 1.511 | 0 | S | 0.236 | 0.004 | S | 0.688 | 0.016 | S |
| 294 | 72474 | SENSE | 0.816 | 0 | S | 0.095 | 0.229 | / | 1.149 | 0.01 | S |
| 298 | 19252 | SENSE | 0.571 | 0.111 | T | 0.02 | 0.416 | / | 1.27 | 0.022 | S |
| 347 | 18854 | SENSE | 0.854 | 0 | S | -0.14 | 0.896 | / | 0.595 | 0.148 | T |
| 351 | 16226 | SENSE | 1.372 | 0 | S | 0.244 | 0.009 | S | 0.112 | 0.017 | S |
| 357 | 70247 | SENSE | 1.146 | 0 | S | 0.124 | 0.114 | T | 0.953 | 0.029 | S |
| 359 | 12635 | SENSE | 0.702 | 0.109 | T | 0.587 | 0.001 | S | 0.637 | 0.06 | T |
| 367 | 14338 | SENSE | 0.888 | 0 | S | 0.036 | 0.326 | / | 0.17 | 0.077 | T |
| 368 | 17809 | SENSE | 0.838 | 0.002 | S | 0.033 | 0.308 | / | 0.619 | 0.04 | S |
| 369 | 72471 | SENSE | 1.051 | 0.001 | S | 0.067 | 0.227 | / | 1.531 | 0.005 | S |
| 373 | 72772 | SENSE | 1.364 | 0 | S | 0.299 | 0.002 | S | 0.648 | 0.045 | S |
| 395 | 70202 | SENSE | 1.159 | 0 | S | -0.116 | 0.941 | / | 0.339 | 0.159 | T |
| 435 | 19719 | SENSE | 1.184 | 0 | S | 0.032 | 0.411 | / | 1.433 | 0.018 | S |
| 473 | 19077 | SENSE | 1.405 | 0 | S | 0.026 | 0.369 | / | 0.61 | 0.013 | S |
| 474 | 19178 | SENSE | 1.381 | 0 | S | 0.267 | 0.008 | S | 1.54 | 0.006 | S |

| | | | | | | | | | | | |
|---|---|---|---|---|---|---|---|---|---|---|---|
| S: represents the transgenic plants showed statistically significant trait improvement as compared to the reference (p<0.05) T: represents the transgenic plants showed a trend of trait improvement as compared to the reference, preferably with p<0.2 /: represents the transgenic plants didn't show any alteration or had unfavorable change in traits examined compared to the reference in the current dataset | | | | | | | | | | | |

### D. Salt stress tolerance screen

This example sets forth the high salinity stress screen to identify *Arabidopsis* plants transformed with the gene of interest that are tolerant to high levels of salt based on their rate of development, root growth and chlorophyll accumulation under high salt conditions.

T2 seeds were plated on glufosinate selection plates containing 90 mM NaCl and grown under standard light and temperature conditions. All seedlings used in the experiment were grown at a temperature of 22°C at day and 20°C at night, a 16-hour photoperiod, an average light intensity of approximately 120 umol/m². On day 11, plants were measured for primary root length. After 3 more days of growth (day 14), plants were scored for transgenic status, primary root length, growth stage, visual color, and the seedlings were pooled for fresh weight measurement. A photograph of the whole plate was also taken on day 14.

Visual assessment was carried out to evaluate the robustness of the growth based on the leave size and rosette size.

The seedling weight and root length were analyzed as quantitative responses according to example 1M. The final growth stage at day 14 was scored as success if 50% of the plants reached 3 rosette leaves and size of leaves are greater than 1mm (Boyes, D.C. et. al. (2001), The Plant Cell 13, 1499/1510). The growth stage data was analyzed as a qualitative response according to example 1L.

**Table 6: a list of recombinant nucleotides that improve high salinity tolerance in plants**

| **Pep SEQ ID** | **Construct id** | **Seedling Weight at day14** | | | **Root Length at day 11** | | | **Root Length at day 14** | | | **Growth Stage** | | |
|---|---|---|---|---|---|---|---|---|---|---|---|---|---|
| | | **delta** | **p-value** | **c** | **delta** | **p-value** | **c** | **delta** | **p-value** | **c** | **RS mean** | **p-vallue** | **c** |
| 243 | 17918 | 0.749 | 0.001 | S | 0.007 | 0.945 | / | 0.054 | 0.348 | / | 0.107 | 0.152 | T |
| 258 | 17819 | 0.713 | 0.026 | S | 0.281 | 0.09 | T | 0.29 | 0.01 | S | 1.565 | 0.025 | S |
| 285 | 19179 | 0.939 | 0 | S | 0.228 | 0.044 | S | 0.269 | 0.001 | S | 1.561 | 0.034 | S |
| 298 | 19252 | 0.831 | 0.003 | S | 0.327 | 0.016 | S | 0.334 | 0.001 | S | 1.5 | 0.028 | S |
| 312 | 70405 | 0.266 | 0.096 | T | 0.051 | 0.628 | / | 0.202 | 0.014 | S | -0.201 | 0.766 | / |
| 372 | 18395 | 0.506 | 0.008 | S | 0.27 | 0.033 | S | 0.24 | 0.007 | S | 0.653 | 0.016 | S |
| 376 | 11409 | 0.834 | 0.004 | S | 0.305 | 0.007 | S | 0.329 | 0.001 | S | 0.712 | 0.073 | T |
| 390 | 18392 | 0.767 | 0 | S | 0.325 | 0.005 | S | 0.181 | 0.034 | S | 0.698 | 0.109 | T |
| 424 | 13005 | 0.787 | 0.003 | S | 0.228 | 0.021 | S | 0.161 | 0.12 | T | 1.079 | 0.013 | S |
| 439 | 18513 | 0.779 | 0.019 | S | 0.377 | 0.013 | S | 0.298 | 0.002 | S | 0.679 | 0.069 | T |
| 463 | 12332 | 0.292 | 0.604 | / | 0.204 | 0.22 | / | 0.057 | 0.829 | / | 0.538 | 0.188 | T |
| 464 | 13649 | 0.418 | 0.03 | S | 0.062 | 0.493 | / | 0.147 | 0.1 | T | 1.39 | 0.028 | S |
| 465 | 16113 | 0.108 | 0.305 | / | 0.168 | 0.221 | / | 0.138 | 0.076 | T | 1.09 | 0.068 | T |
| 466 | 12069 | 0.708 | 0.043 | S | 0.162 | 0.091 | T | 0.165 | 0.354 | / | 0.306 | 0.074 | T |
| 467 | 12906 | 0.764 | 0.05 | T | 0.185 | 0.039 | S | 0.175 | 0.01 | S | 1.212 | 0.009 | S |
| 468 | 13443 | 0.113 | 0.629 | / | -0.061 | 0.542 | / | -0.001 | 0.993 | / | 0.658 | 0.096 | T |
| 469 | 14707 | 0.388 | 0.159 | T | 0.088 | 0.452 | / | -0.094 | 0.564 | / | 0.366 | 0.012 | S |
| 470 | 15116 | 0.576 | 0.02 | S | 0.362 | 0.01 | S | 0.221 | 0.063 | T | 1.414 | 0.027 | S |
| 471 | 16117 | 0.038 | 0.789 | / | 0.02 | 0.87 | / | -0.003 | 0.98 | / | 0.599 | 0.224 | / |
| 472 | 16136 | 0.465 | 0.001 | S | 0.297 | 0 | S | 0.172 | 0.007 | S | 1.911 | 0.005 | S |
| 473 | 19077 | 0.525 | 0.02 | S | 0.23 | 0.006 | S | 0.214 | 0 | S | 0.299 | 0.116 | T |
| 474 | 19178 | 0.398 | 0.22 | / | 0.231 | 0.106 | T | 0.219 | 0.046 | S | 0.456 | 0.213 | / |
| 475 | 70752 | 0.273 | 0.379 | / | 0.122 | 0.387 | / | 0.096 | 0.417 | / | -0.022 | 0.519 | / |
| 476 | 70753 | 0.21 | 0.459 | / | -0.015 | 0.864 | / | 0.116 | 0.286 | / | 0.489 | 0.128 | T |
| 477 | 70809 | 0.802 | 0.007 | S | 0.233 | 0 | S | 0.348 | 0 | S | 2.24 | 0.009 | S |
| 478 | 72091 | 0.608 | 0.014 | S | 0.115 | 0.249 | / | 0.152 | 0.091 | T | 0.398 | 0.267 | / |

| | | | | | | | | | | | | | |
|---|---|---|---|---|---|---|---|---|---|---|---|---|---|
| S: represents the transgenic plants showed statistically significant trait improvement as compared to the reference (p<0.05) T: represents the transgenic plants showed a trend of trait improvement as compared to the reference, preferably with p<0.2 /: represents the transgenic plants didn't show any alteration or had unfavorable change in traits examined compared to the reference in the current dataset | | | | | | | | | | | | | |

### E. Polyethylene Glycol (PEG) induced osmotic stress tolerance screen

There are numerous factors, which can influence seed germination and subsequent seedling growth, one being the availability of water. Genes, which can directly affect the success rate of germination and early seedling growth, are potentially useful agronomic traits for improving the germination and growth of crop plants under drought stress. In this assay, PEG was used to induce osmotic stress on germinating transgenic lines of *Arabidopsis thaliana* seeds in order to screen for osmotically resistant seed lines.

T2 seeds were plated on glufosinate selection plates containing 3% PEG and grown under standard light and temperature conditions. Seeds were plated on each plate containing 3% PEG, 1/2 X MS salts, 1% phytagel, and 10 µg/ml glufosinate. Plates were placed at 4°C for 3 days to stratify seeds. On day 11, plants were measured for primary root length. After 3 more days of growth, i.e. at day 14, plants were scored for transgenic status, primary root length, growth stage, visual color, and the seedlings were pooled for fresh weight measurement. A photograph of the whole plate was taken on day14. Visual assessment was carried out to evaluate the robustness of the growth based on the leave size and rosette size.

Seedling weight and root length were analyzed as quantitative responses according to example 1M. The final growth stage at day 14 was scored as success or failure based on whether the plants reached 3 rosette leaves and size of leaves are greater than 1mm. The growth stage data was analyzed as a qualitative response according to example 1L.

**Table 7: a list of recombinant nucleotides that improve osmotic stress tolerance in plants**

| **Pep SE Q ID** | **Construct** | **Seedling Weight at day14** | | | **Root Length at day 11** | | | **Root Length at day14** | | | **Growth Stage** | | |
|---|---|---|---|---|---|---|---|---|---|---|---|---|---|
| | | **delta** | **p-value** | **c** | **delta** | **p-value** | **c** | **delta** | **p-value** | **c** | **RS mean** | **p-value** | **c** |
| 241 | 74518 | 0.53 | 0.018 | S | 0.217 | 0.024 | S | 0.51 | 0.001 | S | 4 | 0 | S |
| 345 | 14825 | 0.414 | 0.181 | T | 0.271 | 0.053 | T | 0.102 | 0.34 | / | 2.105 | 0.01 | S |
| 346 | 17931 | 0.295 | 0.425 | / | 0.124 | 0.574 | / | 0.116 | 0.454 | / | 2.069 | 0.085 | T |
| 347 | 18854 | 0.484 | 0.015 | S | 0.342 | 0.011 | S | 0.159 | 0.073 | T | 1.023 | 0.238 | / |
| 348 | 12237 | 0.371 | 0.165 | T | 0.325 | 0.001 | S | 0.297 | 0.003 | S | 2.191 | 0.017 | S |
| 349 | 13414 | 0.137 | 0.311 | / | 0.241 | 0.069 | T | 0.265 | 0.045 | S | 2.461 | 0.027 | S |
| 350 | 16160 | 0.303 | 0.044 | S | 0.242 | 0.035 | S | 0.077 | 0.512 | / | 3.381 | 0.001 | S |
| 351 | 16226 | 0.367 | 0.047 | S | 0.132 | 0.224 | / | 0.097 | 0.276 | / | 4 | 0 | S |
| 352 | 16803 | 0.382 | 0.036 | S | 0.125 | 0.489 | / | 0.224 | 0.023 | S | 4 | 0 | S |
| 353 | 18260 | 0.183 | 0.315 | / | 0.125 | 0.315 | / | 0.146 | 0.143 | T | 3.362 | 0.002 | S |
| 354 | 18642 | 0.076 | 0.674 | / | 0.199 | 0.09 | T | 0.199 | 0.029 | S | 3.056 | 0.002 | S |
| 355 | 18721 | 0.336 | 0.104 | T | 0.177 | 0.145 | T | 0.109 | 0.228 | / | 2.281 | 0.02 | S |
| 356 | 19254 | 0.334 | 0.242 | / | 0.155 | 0.227 | / | 0.153 | 0.183 | T | 0.905 | 0.129 | T |
| 357 | 70247 | 0.45 | 0.138 | T | 0.334 | 0.008 | S | 0.169 | 0.07 | T | 2.692 | 0.013 | S |
| 358 | 70650 | 0.215 | 0.121 | T | 0.105 | 0.114 | T | 0.092 | 0.255 | / | 2.749 | 0.011 | S |
| 374 | 19441 | 0.413 | 0.017 | S | 0.256 | 0.003 | S | 0.098 | 0.085 | T | 2.324 | 0.04 | S |
| 424 | 13005 | 0.685 | 0.008 | S | 0.395 | 0.002 | S | 0.226 | 0.013 | S | 3.787 | 0 | S |
| 435 | 19719 | 0.306 | 0.04 | S | 0.135 | 0.051 | T | -0.028 | 0.426 | / | -0.338 | 0.598 | / |
| 474 | 19178 | 0.515 | 0.02 | S | 0.21 | 0.059 | T | 0.169 | 0.08 | T | 3.53 | 0 | S |

| | | | | | | | | | | | | | |
|---|---|---|---|---|---|---|---|---|---|---|---|---|---|
| S: represents the transgenic plants showed statistically significant trait improvement as compared to the reference (p<0.05) T: represents the transgenic plants showed a trend of trait improvement compared to the reference, preferably with p<0.2 /: represents the transgenic plants didn't show any alteration or had unfavorable change in traits examined compared to the reference in the current dataset | | | | | | | | | | | | | |

### F. Cold shock tolerance screen

This example set forth a screen to identify *Arabidopsis* plants transformed with the genes of interest that are more tolerant to cold stress subjected during day 8 to day 28 after seed planting. During these crucial early stages, seedling growth and leaf area increase were measured to assess tolerance when *Arabidopsis* seedlings were exposed to low temperatures. Using this screen, genetic alterations can be found that enable plants to germinate and grow better than wild type plants under sudden exposure to low temperatures.

T2 seeds were tested. Eleven seedlings from each line plus one control line were plated together on a plate containing ½ X Gamborg Salts with 0.8 Phytagel^{™}, 1% Phytagel, and 0.3% Sucrose. Plates were then oriented horizontally and stratified for three days at 4°C. At day three, plates were removed from stratification and exposed to standard conditions (16 hr photoperiod, 22°C at day and 20°C at night) until day 8. At day eight, plates were removed from standard conditions and exposed to cold shock conditions (24 hr photoperiod, 8°C at both day and night) until the final day of the assay, i.e. day 28. Rosette areas were measured at day 8 and day28, which were analyzed as quantitative responses according to example 1M.

**Table 8: a list of recombinant nucleotides that improve cold shock stress tolerance in plants**

| **Pep SEQ ID** | **Construct_id** | **Orientation** | **rosette area at day8** | | | **rosette area at day28** | | | **difference in rosette area between day 28 and day 8** | | |
|---|---|---|---|---|---|---|---|---|---|---|---|
| | | | **delta** | **p-value** | **c** | **delta** | **p-value** | **c** | **delta** | **p-value** | **c** |
| 240 | 19867 | SENSE | -0.032 | 0.603 | / | 0.15 | 0.309 | / | 0.426 | 0.064 | T |
| 241 | 74518 | SENSE | 0.184 | 0.192 | T | 0.653 | 0.001 | S | 0.796 | 0 | S |
| 242 | 15816 | SENSE | 0.366 | 0.027 | S | 0.592 | 0.002 | S | 0.666 | 0.004 | S |
| 243 | 17918 | SENSE | 0.594 | 0 | S | 0.982 | 0 | S | 1.325 | 0 | S |
| 276 | 17925 | SENSE | 0.479 | 0.001 | S | 0.693 | 0 | S | 0.872 | 0 | S |
| 474 | 19178 | SENSE | 0.23 | 0.083 | T | 0.435 | 0.026 | S | 0.435 | 0.103 | T |

| | | | | | | | | | | | |
|---|---|---|---|---|---|---|---|---|---|---|---|
| S: represents the transgenic plants showed statistically significant trait improvement as compared to the reference (p<0.05) T: represents the transgenic plants showed a trend of trait improvement compared to the reference, preferably with p<0.2 /: represents the transgenic plants didn't show any alteration or had unfavorable change in traits examined compared to the reference in the current dataset. | | | | | | | | | | | |

### G. Cold germination tolerance screen

This example sets forth a screen to identify *Arabidopsis* plants transformed with the genes of interests are resistant to cold stress based on their rate of development, root growth and chlorophyll accumulation under low temperature conditions.

T2 seeds were plated and all seedlings used in the experiment were grown at 8°C. Seeds were first surface disinfested using chlorine gas and then seeded on assay plates containing an aqueous solution of 1/2 X Gamborg's B/5 Basal Salt Mixture (Sigma/Aldrich Corp., St. Louis, MO, USA G/5788), 1% Phytagel^{™} (Sigma-Aldrich, P-8169), and 10 ug/ml BASTA^{™} (Bayer Crop Science, Frankfort, Germany), with the final pH adjusted to 5.8 using KOH. Test plates were held vertically for 28 days at a constant temperature of 8°C, a photoperiod of 16 hr, and average light intensity of approximately 100 umol/m²/s. At 28 days post planting, root length was measured, growth stage was observed, the visual color was assessed, and a whole plate photograph was taken.

Visual assessment was carried out to evaluate the robustness of the growth based on the leave size and rosette size.

The root length at day 28 was analyzed as a quantitative response according to example 1M. The growth stage at day 7 was analyzed as a qualitative response according to example 1L.

**Table 9: a list of recombinant nucleotides that improve cold stress tolerance in plants**

| **Pep SEQ ID** | **Construct_id** | **Orientation** | **Root Length at day 28** | | | **Growth Stage at day 28** | | |
|---|---|---|---|---|---|---|---|---|
| | | | **delta** | **p-value** | **c** | **RS mean** | **p-value** | **c** |
| 240 | 19867 | SENSE | 0.071 | 0.292 | / | 1.954 | 0.103 | T |
| 241 | 74518 | SENSE | 0.278 | 0.031 | S | 4 | 0 | S |
| 244 | 15306 | ANTI-SENSE | 0.176 | 0.142 | T | 1.582 | 0.067 | T |
| 245 | 12038 | SENSE | 0.045 | 0.188 | T | 2.271 | 0.022 | S |
| 246 | 12046 | SENSE | 0.177 | 0.125 | T | 3.513 | 0 | S |
| 247 | 13432 | SENSE | 0.182 | 0.015 | S | 1.108 | 0.078 | T |
| 248 | 13711 | SENSE | 0.15 | 0.022 | S | 2.357 | 0.012 | S |
| 249 | 14809 | SENSE | -0.034 | 0.631 | / | 1.95 | 0.047 | S |
| 250 | 14951 | SENSE | 0.237 | 0.053 | T | 3.387 | 0.001 | S |
| 251 | 15632 | SENSE | 0.003 | 0.481 | / | 0.49 | 0.275 | / |
| 252 | 16147 | SENSE | 0.176 | 0.016 | S | 3.284 | 0.003 | S |
| 253 | 16158 | SENSE | 0.084 | 0.235 | / | 1.432 | 0.138 | T |
| 254 | 16170 | SENSE | 0.066 | 0.354 | / | 1.995 | 0.088 | T |
| 255 | 16171 | SENSE | -0.178 | 0.842 | / | -0.671 | 0.732 | / |
| 256 | 16175 | SENSE | -0.054 | 0.7 | / | 1.231 | 0.184 | T |
| 257 | 17430 | SENSE | 0.254 | 0.135 | T | 2.776 | 0.009 | S |
| 258 | 17819 | SENSE | 0.221 | 0.028 | S | -0.475 | 0.922 | / |
| 259 | 17921 | SENSE | -0.151 | 0.912 | / | 1.291 | 0.179 | T |
| 260 | 17928 | SENSE | 0.368 | 0.028 | S | 2.599 | 0.003 | S |
| 261 | 18637 | SENSE | 0.158 | 0.225 | / | 1.143 | 0.164 | T |
| 262 | 18816 | SENSE | 0.206 | 0.075 | T | 3.038 | 0.002 | S |
| 263 | 19227 | SENSE | 0.198 | 0.058 | T | 3.068 | 0.002 | S |
| 264 | 19429 | SENSE | 0.258 | 0.062 | T | 2.582 | 0.006 | S |
| 265 | 70235 | SENSE | 0.175 | 0.065 | T | 2.584 | 0.006 | S |
| 266 | 72634 | SENSE | 0.169 | 0.064 | T | 2.835 | 0.001 | S |
| 267 | 72752 | SENSE | 0.292 | 0.019 | S | 2.816 | 0.002 | S |
| 273 | 17615 | ANTI-SENSE | 0.317 | 0.006 | S | 2.239 | 0.022 | S |
| 277 | 18541 | SENSE | 0.321 | 0.072 | T | 2.631 | 0.014 | S |
| 293 | 70484 | SENSE | 0.2 | 0.018 | S | 2.61 | 0.016 | S |
| 298 | 19252 | SENSE | 0.391 | 0.002 | S | 1.041 | 0.084 | T |
| 346 | 17931 | SENSE | 0.096 | 0.059 | T | 1.213 | 0.142 | T |
| 357 | 70247 | SENSE | 0.299 | 0.006 | S | 2.607 | 0.005 | S |
| 366 | 19610 | SENSE | 0.33 | 0.079 | T | 4 | 0 | S |
| 367 | 14338 | SENSE | 0.223 | 0.071 | T | 1.125 | 0.087 | T |
| 376 | 11409 | SENSE | 0.193 | 0 | S | 1.831 | 0.024 | S |
| 434 | 17922 | SENSE | 0.238 | 0.029 | S | 3.109 | 0.002 | S |

| | | | | | | | | |
|---|---|---|---|---|---|---|---|---|
| S: represents the transgenic plants showed statistically significant trait improvement as compared to the reference (p<0.05) T: represents the transgenic plants showed a trend of trait improvement as compared to the reference, preferably with p<0.2 /: represents the transgenic plants didn't show any alteration or had unfavorable change in traits examined compared to the reference in the current dataset | | | | | | | | |

### H. shade tolerance-low light screen

Plants undergo a characteristic morphological response in shade that includes the elongation of the petiole, a change in the leaf angle, and a reduction in chlorophyll content. While these changes may confer a competitive advantage to individuals, in a monoculture the shade avoidance response is thought to reduce the overall biomass of the population. Thus, genetic alterations that prevent the shade avoidance response may be associated with higher yields. Genes that favor growth under low light conditions may also promote yield, as inadequate light levels frequently limit yield. This protocol describes a screen to look for *Arabidopsis* plants that show an attenuated shade avoidance response and/or grow better than control plants under low light intensity. Of particular interest, we were looking for plants that didn't extend their petiole length, had an increase in seedling weight relative to the reference and had leaves that were more close to parallel with the plate surface.

T2 seeds were plated on glufosinate selection plates with ½ MS medium. Seeds were sown on 1/2 X MS salts, 1% Phytagel, 10 ug/ml BASTA. Plants were grown on vertical plates at a temperature of 22°C at day, 20°C at night and under low light (approximately 30 uE/m²/s, far/red ratio (655/665/725/735) ~0.35 using PLAQ lights with GAM color filter #680). Twenty-three days after seedlings were sown, measurements were recorded including seedling status, number of rosette leaves, status of flower bud, petiole leaf angle, petiole length, and pooled fresh weights. A digital image of the whole plate was taken on the measurement day. Seedling weight and petiole length were analyzed as quantitative responses according to example 1M. The number of rosette leaves, flowering bud formation and leaf angel were analyzed as qualitative responses according to example 1L.

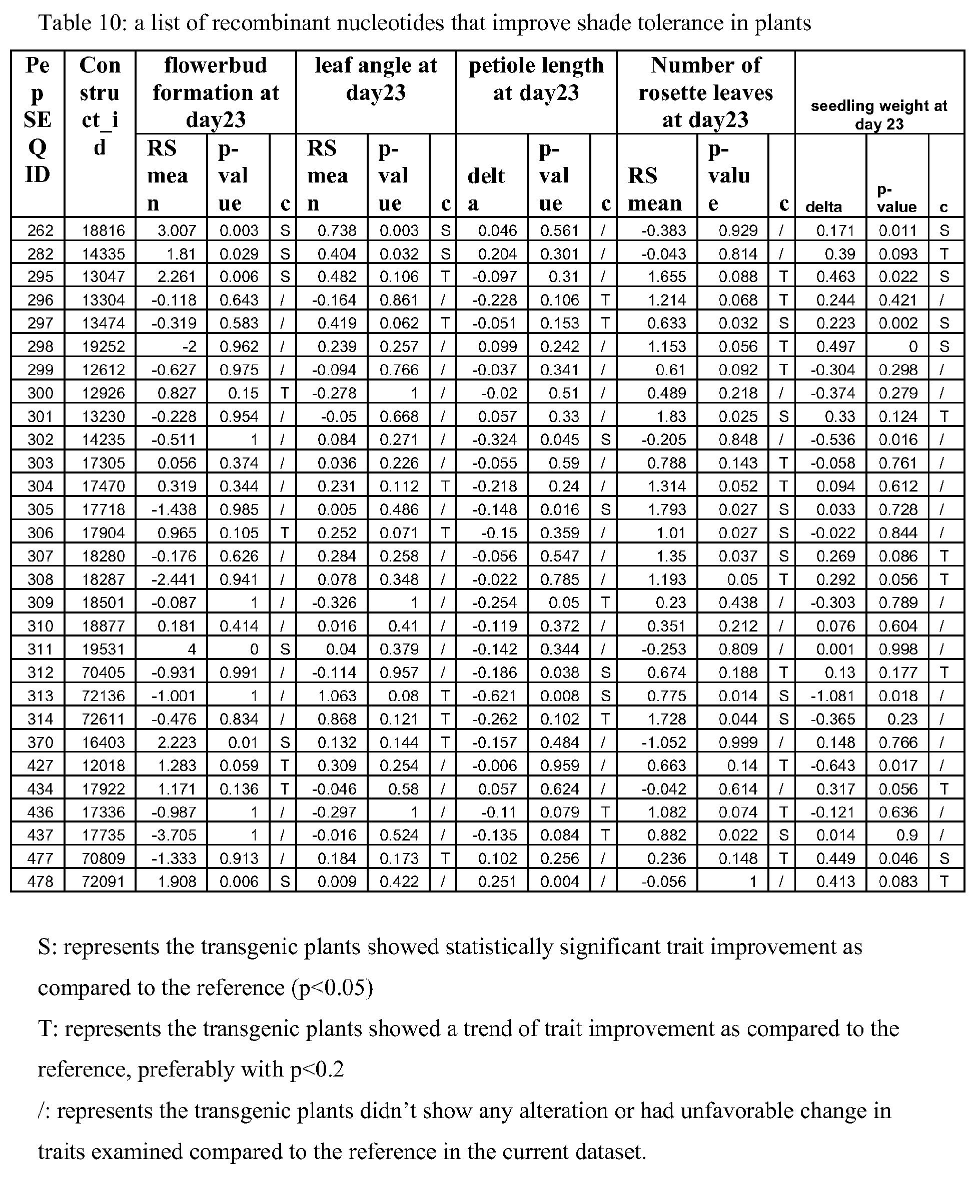

### I. early plant growth and development screen

This example sets forth a plate based phenotypic analysis platform for the rapid detection of phenotypes that are evident during the first two weeks of growth. In this screen, we were looking for genes that confer advantages in the processes of germination, seedling vigor, root growth and root morphology under non-stressed growth conditions to plants. The transgenic plants with advantages in seedling growth and development were determined by the seedling weight and root length at day 14 after seed planting.

T2 seeds were plated on glufosinate selection plates and grown under standard conditions (~100 uE/m²/s, 16 h photoperiod, 22°C at day, 20°C at night). Seeds were stratified for 3 days at 4°C. Seedlings were grown vertically (at a temperature of 22°C at day 20°C at night). Observations were taken on day 10 and day 14. Both seedling weight and root length at day 14 were analyzed as quantitative responses according to example 1M.

**Table 11: a list recombinant nucleotides that improve early plant growth and development**

| **Pep SEQ ID** | **Construct_id** | **Orientation** | **Root Length at day14** | | | **Seedling Weight at day14** | | |
|---|---|---|---|---|---|---|---|---|
| | | | **delta** | **p-value** | **c** | **delta** | **p-value** | **c** |
| 241 | 74518 | SENSE | 0.216 | 0.01 | S | 0.454 | 0.049 | S |
| 245 | 12038 | SENSE | 0.101 | 0.046 | S | 0.629 | 0.003 | S |
| 250 | 14951 | SENSE | 0.15 | 0.072 | T | 0.138 | 0.378 | / |
| 260 | 17928 | SENSE | 0.062 | 0.22 | / | 0.246 | 0.069 | T |
| 265 | 70235 | SENSE | 0.079 | 0.519 | / | 0.414 | 0.026 | S |
| 267 | 72752 | SENSE | 0.301 | 0.001 | S | 0.789 | 0.002 | S |
| 285 | 19179 | SENSE | 0.216 | 0.024 | S | 0.603 | 0.01 | S |
| 290 | 70222 | SENSE | 0.047 | 0.468 | / | 0.394 | 0.014 | S |
| 293 | 70484 | SENSE | 0.068 | 0.364 | / | 0.444 | 0.024 | S |
| 294 | 72474 | SENSE | 0.241 | 0.051 | T | 0.183 | 0.564 | / |
| 298 | 19252 | SENSE | 0.065 | 0.392 | / | 0.316 | 0.054 | T |
| 326 | 17344 | SENSE | 0.042 | 0.565 | / | 0.223 | 0.066 | T |
| 330 | 17906 | SENSE | 0.11 | 0.152 | T | 0.419 | 0.011 | S |
| 354 | 18642 | SENSE | 0.1 | 0.247 | / | 0.257 | 0.043 | S |
| 357 | 70247 | SENSE | -0.04 | 0.842 | / | 0.237 | 0.134 | T |
| 358 | 70650 | SENSE | 0.121 | 0.077 | T | 0.135 | 0.442 | / |
| 359 | 11787 | ANTI-SENSE | -0.083 | 0.784 | / | 0.167 | 0.365 | / |
| 360 | 13641 | ANTI-SENSE | 0.092 | 0.15 | T | 0.336 | 0.053 | T |
| 361 | 14515 | ANTI-SENSE | 0.051 | 0.616 | / | 0.351 | 0.038 | S |
| 362 | 14920 | ANTI-SENSE | 0.08 | 0.358 | / | 0.101 | 0.739 | / |
| 363 | 15204 | ANTI-SENSE | 0.203 | 0.015 | S | 0.076 | 0.811 | / |
| 364 | 15216 | ANTI-SENSE | 0.316 | 0.023 | S | 0.632 | 0.073 | T |
| 365 | 15330 | ANTI-SENSE | 0.084 | 0.428 | / | 0.435 | 0.002 | S |
| 366 | 19610 | SENSE | 0.192 | 0.011 | S | 0.523 | 0.008 | S |
| 367 | 14338 | SENSE | 0.145 | 0.155 | T | 0.589 | 0.072 | T |
| 368 | 17809 | SENSE | 0.014 | 0.928 | / | -0.121 | 0.753 | / |
| 369 | 72471 | SENSE | 0.07 | 0.023 | S | 0.407 | 0.048 | S |
| 370 | 16403 | SENSE | 0.199 | 0.027 | S | 0.6 | 0.003 | S |
| 371 | 17737 | SENSE | 0.049 | 0.472 | / | 0.242 | 0.073 | T |
| 372 | 18395 | SENSE | 0.219 | 0.001 | S | 0.58 | 0.002 | S |
| 373 | 72772 | SENSE | 0.224 | 0.023 | S | 0.442 | 0.106 | T |
| 374 | 19441 | SENSE | 0.271 | 0 | S | 0.482 | 0.019 | S |
| 375 | 10486 | SENSE | 0.191 | 0.03 | S | 0.343 | 0.052 | T |
| 376 | 11409 | SENSE | 0.258 | 0.034 | S | 0.468 | 0.006 | S |
| 377 | 12104 | SENSE | 0.1 | 0.379 | / | 0.489 | 0.009 | S |
| 378 | 12258 | SENSE | 0.16 | 0.05 | T | 0.392 | 0.137 | T |
| 379 | 12909 | SENSE | 0.139 | 0.267 | / | 0.322 | 0.261 | / |
| 380 | 14310 | SENSE | 0.544 | 0 | S | 0.764 | 0.026 | S |
| 381 | 14317 | SENSE | 0.134 | 0.18 | T | 0.117 | 0.64 | / |
| 382 | 14709 | SENSE | 0.206 | 0.009 | S | 0.389 | 0.117 | T |
| 383 | 15123 | SENSE | 0.026 | 0.872 | / | 0.27 | 0.348 | / |
| 384 | 16013 | SENSE | 0.046 | 0.622 | / | 0.464 | 0.01 | S |
| 385 | 16185 | SENSE | 0.191 | 0.045 | S | 0.145 | 0.596 | / |
| 386 | 16719 | SENSE | 0.019 | 0.872 | / | 0.424 | 0.088 | T |
| 387 | 17490 | SENSE | 0.186 | 0.026 | S | 0.272 | 0.102 | T |
| 388 | 17905 | SENSE | 0.239 | 0.004 | S | 0.346 | 0.196 | T |
| 389 | 18385 | SENSE | 0.287 | 0.003 | S | 0.687 | 0.003 | S |
| 390 | 18392 | SENSE | 0.088 | 0.338 | / | 0.504 | 0.012 | S |
| 392 | 18531 | SENSE | 0.313 | 0.015 | S | 0.627 | 0 | S |
| 393 | 18603 | SENSE | 0.212 | 0 | S | 0.165 | 0.187 | T |
| 394 | 19530 | SENSE | 0.106 | 0.137 | T | 0.342 | 0.025 | S |
| 395 | 70202 | SENSE | 0.218 | 0.056 | T | 0.279 | 0.223 | / |
| 396 | 72009 | SENSE | 0.191 | 0.054 | T | 0.328 | 0.043 | S |
| 397 | 72119 | SENSE | 0.236 | 0 | S | 0.259 | 0.008 | S |
| 438 | 19249 | SENSE | 0.054 | 0.375 | / | 0.222 | 0.048 | S |
| 439 | 18513 | SENSE | 0.204 | 0.044 | S | 0.193 | 0.322 | / |
| 461 | 19222 | SENSE | 0.255 | 0.072 | T | 0.622 | 0.034 | S |
| 473 | 19077 | SENSE | -0.049 | 0.669 | / | 0.2 | 0.227 | / |
| 474 | 19178 | SENSE | 0.303 | 0 | S | 0.592 | 0.001 | S |
| 477 | 70809 | SENSE | 0.128 | 0.093 | T | 0.224 | 0.185 | T |

| | | | | | | | | |
|---|---|---|---|---|---|---|---|---|
| For other responses: S: represents the transgenic plants showed statistically significant trait improvement as compared to the reference (p<0.05) T: represents the transgenic plants showed a trend of trait improvement as compared to the reference, preferably with p<0.2 /: represents the transgenic plants didn't show any alteration or had unfavorable change in traits examined compared to the reference in the current dataset | | | | | | | | |

### J. late plant growth and development screen

This example sets forth a soil based phenotypic platform to identify genes that confer advantages in the processes of leaf development, flowering production and seed maturity to plants.

*Arabidopsis* plants were grown on a commercial potting mixture (Metro Mix 360, Scotts Co., Marysville, OH) consisting of 30-40% medium grade horticultural vermiculite, 35-55% sphagnum peat moss, 10-20% processed bark ash, 1-15% pine bark and a starter nutrient charge. Soil was supplemented with Osmocote time-release fertilizer at a rate of 30 mg/ft³. T2 seeds were imbibed in 1% agarose solution for 3 days at 4 °C and then sown at a density of ~5 per 2 ½" pot. Thirty-two pots were ordered in a 4 by 8 grid in standard greenhouse flat. Plants were grown in environmentally controlled rooms under a 16 h day length with an average light intensity of ~200 µmoles/m²/s. Day and night temperature set points were 22 °C and 20 °C, respectively. Humidity was maintained at 65%. Plants were watered by sub-irrigation every two days on average until mid-flowering, at which point the plants were watered daily until flowering was complete.

Application of the herbicide glufosinate was performed to select T2 individuals containing the target transgene. A single application of glufosinate was applied when the first true leaves were visible. Each pot was thinned to leave a single glufosinate-resistant seedling ~3 days after the selection was applied.

The rosette radius was measured at day 25. The silique length was measured at day 40. The plant parts were harvested at day 49 for dry weight measurements if flowering production was stopped. Otherwise, the dry weights of rosette and silique were carried out at day 53. The seeds were harvested at day 58. All measurements were analyzed as quantitative responses according to example 1M.

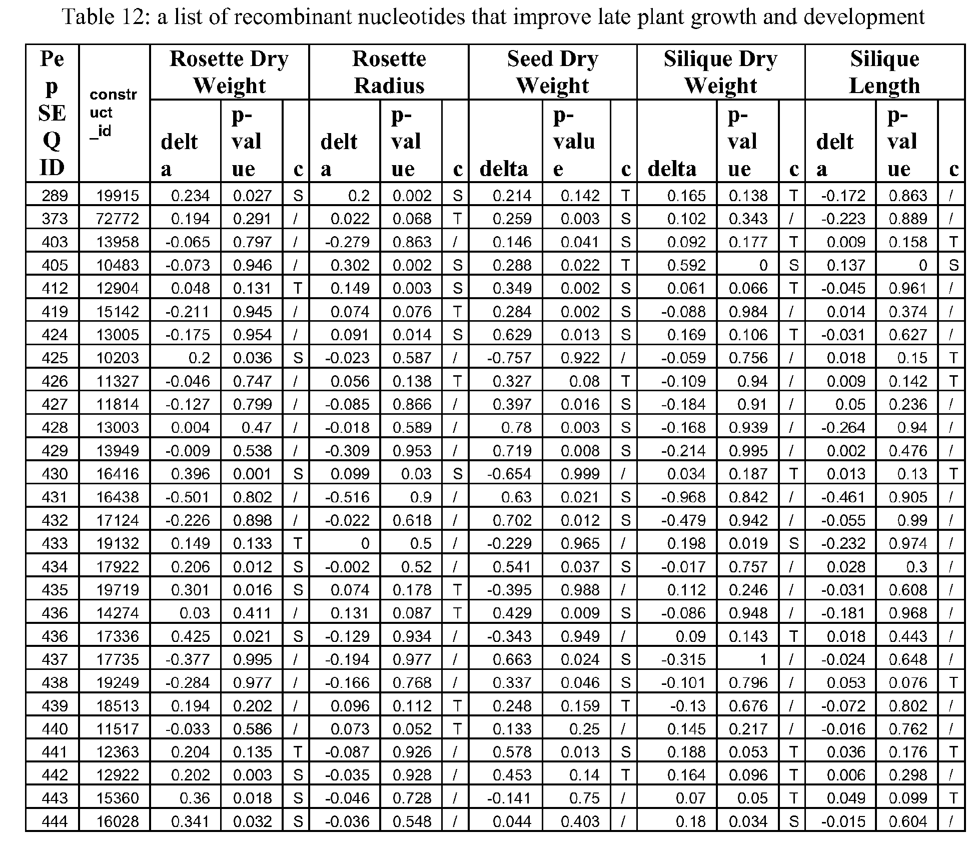

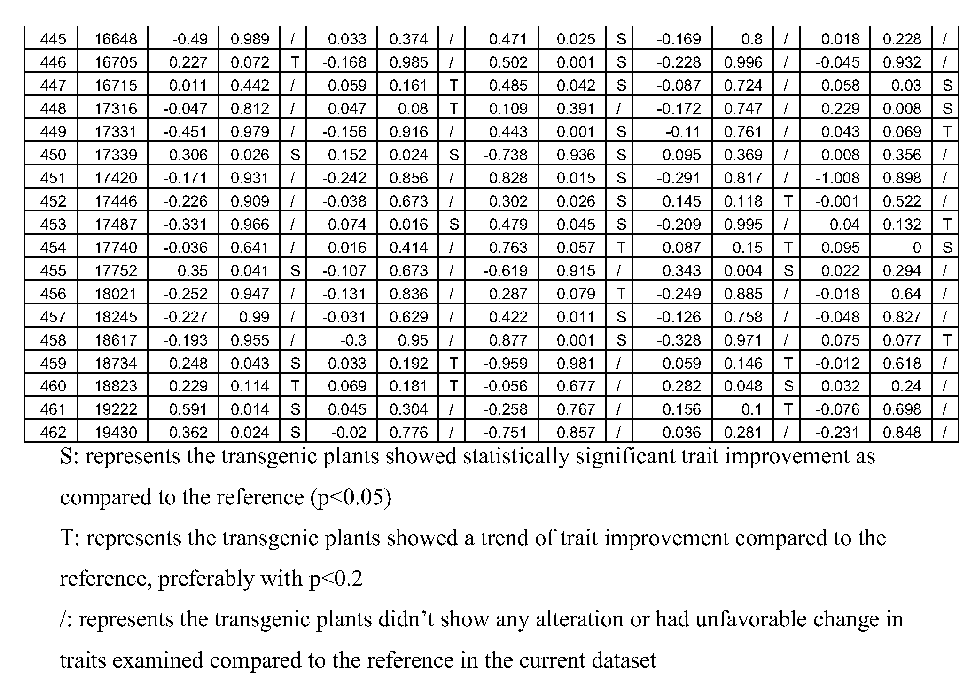

### K. Low nitrogen tolerance screen

Under low nitrogen conditions, *Arabidopsis* seedlings become chlorotic and have less biomass. This example sets forth the low nitrogen tolerance screen to identify *Arabidopsis* plants transformed with the gene of interest that are altered in their ability to accumulate biomass and/or retain chlorophyll under low nitrogen condition.

T2 seeds were plated on plates containing 0.5x N-Free Hoagland's T 0.1 mM NH₄NO₃ T 0.1 % sucrose T 1% phytagel media and grown under standard light and temperature conditions. At 12 days of growth, plants were scored for seedling status (i.e. viable or non-viable) and root length. After 21 days of growth, plants were scored for visual color, seedling weight, number of green leaves, number of rosette leaves, root length and formation of flowering buds. A photograph of each plant was also taken at this time point.

The seedling weight and root length were analyzed as quantitative responses according to example 1M. The number green leaves, the number of rosette leaves and the flowerbud formation were analyzed as qualitative responses according to example 1L. We considered that the transgenic plants grew better under the low nitrogen condition evidenced by having more green leaves or greener leaves, compared to the reference. In addition, the change in the root length in either direction, i.e. either increase or decrease will benefit plant growth. Transgenic plants with increased root length under a low nitrogen condition will enable plants to obtain nutrient from a farther distance, whereas transgenic plants with decreased root length while maintain a healthy growth evidenced by green leaves may have developed an intrinsic mechanism of using nitrogen source efficiently.

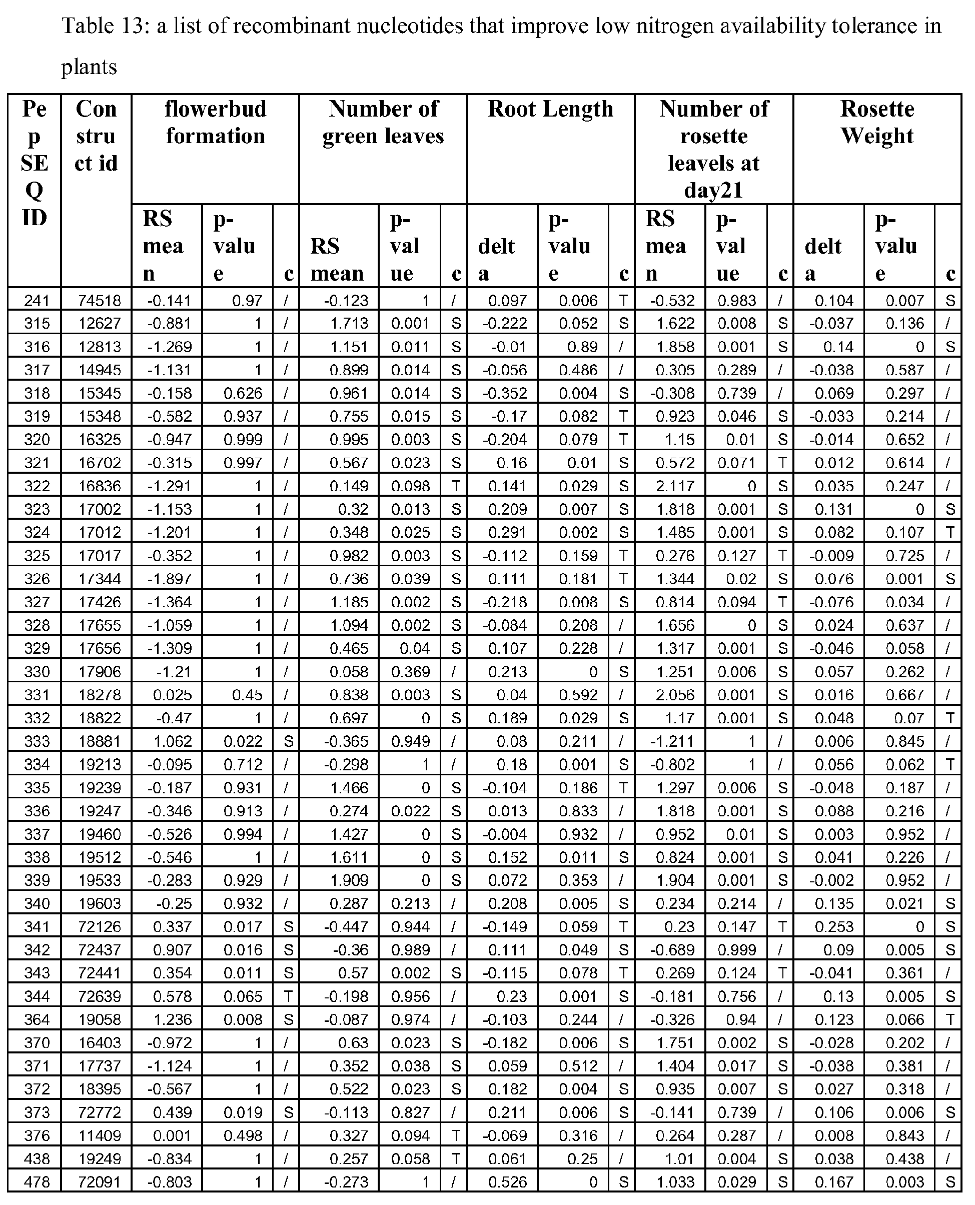

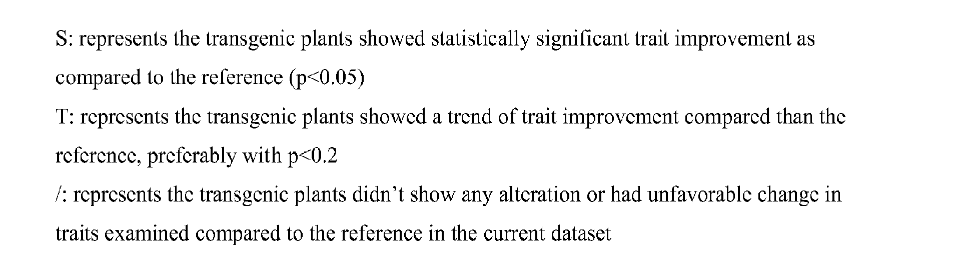

### L. Statistic analysis for qualitative responses

**Table 14: a list of responses analyzed as qualitative responses**

| **response** | **screen** | **categories (success vs. failure)** |
|---|---|---|
| wilting response risk score | drought tolerance screen | non-wilted vs. wilted |
| growth stage at day 14 | heat stress tolerance screen | 50% of plants reach stage1.03 vs. not |
| growth stage at day 14 | salt stress tolerance screen | 50% of plants reach stage1.03 vs. not |
| growth stage at day 14 | PEG induced osmotic stress tolerance screen | 50% of plants reach stage1.03 vs. not |
| growth stage at day 7 | cold germination stress tolerance screen | 50% of plants reach stage 0.5 vs. not |
| number of rosette leaves at day 23 | shade tolerance-low light screen | 5 leaves appeared vs. not |
| flower bud formation at day 23 | Shade tolerance-low light screen | flower buds appear vs. not |
| leaf angle at day 23 | Shade tolerance-low light screen | >60 degree vs. <60 degree |
| number of green leaves at day 21 | low nitrogen tolerance screen | 6 or 7 leaves appeared vs. not |
| number of rosette leaves at day 21 | low nitrogen tolerance screen | 6 or 7 leaves appeared vs. not |
| Flower bud formation at day 21 | low nitrogen tolerance screen | flower buds appear vs. not |

Plants were grouped into transgenic and reference groups and were scored as success or failure according to Table 14. First, the risk (R) was calculated, which is the proportion of plants that were scored as of failure plants within the group. Then the relative risk (RR) was calculated as the ratio of R (transgenic) to R (reference). Risk score (RS) was calculated as - log₂ ^{RR}. Subsequently the risk scores from multiple events for each transgene of interest were evaluated for statistical significance by t-test using S-PLUS statistical software (S-PLUS 6, Guide to statistics, Insightful, Seattle, WA, USA). RS with a value greater than 0 indicates that the transgenic plants perform better than the reference. RS with a value less than 0 indicates that the transgenic plants perform worse than the reference. The RS with a value equal to 0 indicates that the performance of the transgenic plants and the reference don't show any difference.

### M. Statistic analysis for quantitative responses

**Table 15: a list of responses analyzed as quantitative responses**

| **response** | **screen** |
|---|---|
| seed yield | drought stress tolerance screen |
| seedling weight at day 14 | heat stress tolerance screen |
| root length at day 14 | heat stress tolerance screen |
| seedling weight at day 14 | salt stress tolerance screen |
| root length at day 14 | salt stress tolerance screen |
| root length at day 11 | salt stress tolerance screen |
| seedling weight at day 14 | PEG induced osmotic stress tolerance screen |
| root length at day 11 | PEG induced osmotic stress tolerance screen |
| root length at day 14 | PEG induced osmotic stress tolerance screen |
| rosette area at day 8 | cold shock tolerance screen |
| rosette area at day28 | cold shock tolerance screen |
| difference in rosette area from day 8 to day 28 | cold shock tolerance screen |
| root length at day 28 | cold stress tolerance screen |
| seedling weight at day 23 | Shade tolerance-low light screen |
| petiole length at day 23 | Shade tolerance-low light screen |
| root length at day 14 | Early plant growth and development screen |
| seedling weight at day14 | Early plant growth and development screen |
| rosette radius at day 25 | Late plant growth and development screen |
| seed dry weight at day 58 | Late plant growth and development screen |
| silique dry weight at day 53 | Late plant growth and development screen |
| silique length at day 40 | Late plant growth and development screen |
| Seedling weight at day 21 | Low nitrogen tolerance screen |
| Root length at day 21 | Low nitrogen tolerance screen |

The measurements (M) of each plant were transformed by log₂ calculation. The Delta was calculated as log₂M(transgenic)- log₂M(reference). Subsequently the mean delta from multiple events of the transgene of interest was evaluated for statistical significance by t-test using S-PLUS statistical software (S-PLUS 6, Guide to statistics, Insightful, Seattle, WA, USA). The Delta with a value greater than 0 indicates that the transgenic plants perform better than the reference. The Delta with a value less than 0 indicates that the transgenic plants perform worse than the reference. The Delta with a value equal to 0 indicates that the performance of the transgenic plants and the reference don't show any difference.

### Example 2 Identification of Homologs

A BLAST searchable "All Protein Database" was constructed of known protein sequences using a proprietary sequence database and the National Center for Biotechnology Information (NCBI) non-redundant amino acid database (nr.aa). For each organism from which a DNA sequence provided herein was obtained, an "Organism Protein Database" was constructed of known protein sequences of the organism; the Organism Protein Database is a subset of the All Protein Database based on the NCBI taxonomy ID for the organism.. The All Protein Database was queried using amino acid sequence of cognate protein for gene DNA used in trait-improving recombinant DNA, i.e. sequences of SEQ ID NO: 240 through SEQ ID NO: 478 using "blastp" with E-value cutoff of 1e-8. Up to 1000 top hits were kept, and separated by organism names. For each organism other than that of the query sequence, a list was kept for hits from the query organism itself with a more significant E-value than the best hit of the organism. The list contains likely duplicated genes, and is referred to as the Core List. Another list was kept for all the hits from each organism, sorted by E-value, and referred to as the Hit List.

The Organism Protein Database was queried using amnno acid sequences of SEQ ID NO: 240 through SEQ ID NO: 478 using "blastp" with E-value cutoff of le-4. Up to 1000 top hits were kept. A BLAST searchable database was constructed based on these hits, and is referred to as "SubDB". SubDB was queried with each sequence in the Hit List using "blastp" with E-value cutoff of 1e-8. The hit with the best E-value was compared with the Core List from the corresponding organism. The hit is deemed a likely ortholog if it belongs to the Core List, otherwise it is deemed not a likely ortholog and there is no further search of sequences in the Hit List for the same organism. Likely orthologs from a large number of distinct organisms were identified and are reported by amino acid sequences of SEQ ID NO: 479 to SEQ ID NO: 12463. These orthologs are reported in Tables 2 as homologs to the proteins cognate to genes used in trait-improving recombinant DNA.

### Example 3 Consensus sequence build

ClustalW program was selected for multiple sequence alignments of the amino acid sequence of SEQ ID NO:439 and 25 homologs. Three major factors affecting the sequence alignments dramatically are (1) protein weight matrices; (2) gap open penalty; (3) gap extension penalty. Protein weight matrices available for ClustalW program include Blosum, Pam and Gonnet series. Those parameters with gap open penalty and gap extension penalty were extensively tested. On the basis of the test results, Blosum weight matrix, gap open penalty of 10 and gap extension penalty of 1 were chosen for multiple sequence alignment. Attached are the sequences of SEQ ID NO: 439, its homologs and the consensus sequence at the end. The symbols for consensus sequence are (1) uppercase letters for 100% identity in all positions of multiple sequence alignment output; (2) lowercase letters for >=70% identity; symbol; (3) "X" indicated <70% identity; (4) dashes "-" meaning that gaps were in >=70% sequences.

SEQ ID NO :
5211      :------------------------------------------------------------
12100     :------------------------------------------------------------
6033      :------------------------------------------------------------
5630      :------------------------------------------------------------
2801      :------------------------------------------------------------
11474     :------------------------------------------------------------
12365     :------------------------------------------------------------
9090      :------------------------------------------------------------
439       :------------------------------------------------------------
11419     :------------------------------------------------------------
11201     :------------------------------------------------------------
4683      :------------------------------------------------------------
1624      :------------------------------------------------------------
11490     :------------------------------------------------------------
9137      :------------------------------------------------------------
10769     :MIGTRVLAHSRVDPAIRWGVAARGRVVFAAIRWGAAARGRVVFAAVRWGAAARGTKREAG
2036      :------------------------------------------------------------
1472      :------------------------------------------------------------
2526      :------------------------------------------------------------
12153     :------------------------------------------------------------
2333      :------------------------------------------------------------
8918      :------------------------------------------------------------
12149     :------------------------------------------------------------
6330      :------------------------------------------------------------
11407     :------------------------------------------------------------
9050      :------------------------------------------------------------
consensus :------------------------------------------------------------
12464

-------------------MSCFACCGDEDTQ-VPDTRAQYPGHHPAR------------
-------------------MSCFACCGDEDTQ-VPDTRAQYPGHHPAR------------
-------------------MSCFACCGDEDTQ-VPDTRAQYPGHHPAR------------
-------------------MSCFACCGDEDTQ-VPDTRTQYPGHHPAR------------
-------------------MSCFACCGDEDTQGVPDNRNPYPGNHPAR------------
-------------------MSCLACCGGEDTQRTPDNGGPYPGGYPPR------------
-------------------MSCLACCGGEDTQRTPDNGGPYPGGYPPR------------
-------------------MSCFVCCGDEDTQRAPDNRNQYXKAIQQG------------
-------------------MSCFGCCGEDDDMHKTADYGGRHNQAKHFPPG---------
-------------------MSCFSCCDDDDMHRATDNGPFMAHNSAGN------------
-------------------MSCFSCCDDDDMHRATDNGPFMAHNSAGN------------
-------------------MGCFSCCGADDVGKKKKRDDPYVPIPDPG--G---------
-------------------MGFLCCSGKPSKRLESSSINENNSNIKRKDQTHVTSGSLKM
-------------------MGFLCFSGKSSKRSENSSIDENNSNIKRKDQTQLTSGSMKV
-------------------MGWIPCSGKSSGKTKKRSDSDENLSRNCSVSASERS-----
QETSTSETKKTKRKWGRGFCGMASHEVEEPLTSETKKTKRKWGRGFCGMASHEAEEPLTS
------------------MKILLGVGINGGLFGSCVSSRSKVDSSTSGISSHFEIKSTN-
------------------------------------------------------------

------------------------------------------------------------
------------------------------------------------------------
------------------------------------------------------------
------------------------------------------------------------
------------------------------------------------------------
------------------------------------------------------------
------------------------------------------------------------
----------------------MAAADTSRVFLILIIALVMVIVVLLGICWRFLGPGIMR
-------------------xxxxxxxxxxxxxxxxxxxxxxxxxxxxx--x---------

------------------ADAYRPSDQPPKGPQPVKMQPIAVPAIPVDEIREVTKGFGDE
------------------ADAYRPSDQPPKGPQPVKMQPIAVPAIPVDEIREVTKGFGDE
------------------ADAYRPSDQPPKGPQPVKMQPIAVPAIPVDEIREVTKGFGDE
------------------ADAYRPADQPPKGSQPVKMQPIAVPAIPVDELREVTKGFGDE
------------------SDAYRTADPTPRGPQPVKVQPIAVPIIPVDEIREVTKNFGDE
------------------DDAYRTADPTPRGAQPLKMQPITVPTIPVEEIREVTVAFGDE
------------------DDAYRTADPTPRGAQPLKMQPITVPTIPVEEIREVTVAFGDE
------------------NDAYRTADPTPKGPQPVKVQPIAVPTIPMDEIREKNCTGGDE
----------------NDARHHQASETAQKGPPVVKLQPIEVPIIPFSELKEATDDFGSN
------------------NGGQRATESAQRETQTVNIQPIAVPSIAVDELKDITDNFGSK
------------------NGGQRATESAQRETQTVNIQPIAVPSIAVDELKDITDNFGSK
----------------NYGRSKPGPPAPSRSPPTSRNLPIAVPAIPLDEIKGITKNFSSD
KPYVNNLSKEGESKDDQLSLDVKSLNMKDEISKDRRSNGKQAQTFTFEELAAATSNFRSD
KPYVNDSREEGASKDDQLSLDVKSLNLKDEISKDIRNNGNPAQTFTFEELVAATDNFRSD
----------------------KAKSSVSESRSRGSDNIVAQTFTFSELATATRNFRKE
ETKKKRKNVAASSEPDKKRWFKNKIWKKKKAKNEQLATLVKEISLATKLNSAMHVNINLS
-----------NVSKDQPTTSNSEHNLPTLTPEDELKVASRLRKFGFNDLKMATRNFRPE
----------------MGSKYSKATNSINDALNSSYLVPFESYRFPLVDLEEATNNFD--
----------------MGSKYSKATNSINDALSSSYLVPFESYRVPLVDLEEATNNFDDK
----------------MGSKYSKATNSISDASNSRYGVPFENYRVPLVDLEEATNNFDDN
----------------MGSKYSKATNSINDASNSSYRVPFESLRVPFVDLQEATNNFDDK
--------------------------------LNSSYRVPFESFRVPFVDLQEATNNFDEK
--------------------------------LNSSYRVPFESFRVPFVDLQEATNNFDEK
--------------------------------LNSSYRVPFESFRVPFVDLQEATNNFDEK
------------------MRSKDSKETTYISDTTSYRFPVESSQIPFAALQEATNNFNCN
---------------RLLRPRRCPSEVPEYFSGNMSGNLRTITYFDYVTLKKATKDFHQK
----------------xxxxxxxxxxxxxxxxxxxxxpxxxxxxxxxxxxxxtxxfxxx

ALIGEGSFGRVYLGVLRNG----------RSAAVKKLDS-NKQPDQEFLA-QVSMVSRLK
ALIGEGSFGRVYLGVLRNGX---------GVAAVKKLDS-NKQPDQEFLSXQVSMVSRLK
ALIGEGSFGRVYLGVLRNG----------RSAAVKKLDS-NKQPDQEFLA-QVSMVSRLK
ALIGEGSFGRVYLGVLRNGR---------SAXRVKKLDS-NKQPDQEFLXAQVSMVSRLK

ALIGEGSFGRVYFGVLRNG----------RSAAVKKLDS-SKQPDQEFLA-QVSMVSRLK
ALIGEGSFGRVYFGVLKNG----------RSAAIKKLDS-SKQPEQEFLA-QVSMVSRLK
ALIGEGSFGRVYFGVLKNG----------RSAAIKKLDS-SKQPEQEFLA-QVSMVSRLK
ALIGEGSFGRVYFGTLRNG----------RGAAIKKLDS-SKQPDQELLA-QVSMVSRLK
SLIGEGSYGRVYYGVLNND----------LPSAIKKLDS-NKQPDNEFLA-QVSMVSRLK
ALIGEGSYGRVYHGVLKSG----------RAAAIKKLDS-SKQPDREFLA-QVSMVSRLK
ALIGEGSYGRVYHGVLKSG----------RAAAIKKLDS-SKQPDREFLA-QVSMVSRLK
ALIGEGSYARVFFGVLRDG----------RRSAVKKLDS-SKQPDQEFLV-QVSAVSRLK
CFLGEGGFGKVYKGYLDK---------INQAVAIKQLDR-NGVQGIREFVVEVVTLSLAD
CFLGEGGFGKVYKGYLEK---------INQVVAIKQLDQ-NGLQGIREFVVEVLTLSLAD
CLIGEGGFGRVYKGYLAS---------TGQTAAIKQLDH-NGLQGNREFLVEVLMLSLLH
MNICPTQTYEEHSGTYLR---------NLAVIAVKQLDK-DGLQGNREFLVEVLMLSLLH
SLLGEGGFGCVFKGWIEENGTAPVKPGTGLTVAVKTLNH-DGLQGHKEWLAEVNFLGDLG
---GKGGFGKVYRGVLRDG----------TKVALKRHNR-DSGQSIEPFRTEIEILSRRS
FFIGAGVFGKVYKGVLRDG----------TKVALKRRKP-ESSQGIEEFETEIEILSFCS
FFIAEGGFGKVYRGVLRDG----------TKVALKRHNC-DSQQSIEEFRTEIEILSRRS
FLIGWGVFGKVYMGVLRNG----------TKVALKKHMP-ESSQGIEEFRTEIEILSLCS
FHIGLGGFGKVYRGVLRDG----------TKVALKRCKR-ESSQGIEEFRTEIEILSFCS

FHIGLGGFGKVYRGVLRDG----------TKVALKRCKR-ESSQGIEEFRTEIEILSFCS
FHIGLGGFGKVYRGVLRDG----------TKVALKRCKR-ESSQGIEEFQTEIEILSFCS
SLIGLGGFGTVYRGVLCDG----------TKVALKRCKL-ESSQGIEEFQTEIEMLSHFR
NQLGRGGFGPVYLGKLDDG----------RKVAVKQLSVGKSGQGESEFFMEVNMITSIQ
xxigxgxfgxvyxGvlxxg----------xxxaxKxxxx-xxxxxxexxxxxxxxxsxxx

HENVVELLGYCADGTLRVLAYEFATMGSLHDMLHGRKGVKG-AQPGPVLSWSQRVKIAVG
HENXVELLGYCXDGTLRVLAYEFATMGSLHDMLHGRKGVKG-AQPGPVLXWSQRXKIAVG
HENVVELLGYCADGTLRVLAYEFATMGSLHDMLHGRKGVKG-AQPGPVLSWSQRVKIAVG
HENVVELLGYCADGTLRVLAYEFATMGSLHDMLHGRKGVKG-AQPGPVLSWLQRVKIAVG
HEHVVELLGYCVDGNLRVLAYEFATMGSLHDMLHGRKGVKG-AQPGPVLSWAQRVKIAVG
HGNVVELLGYCVDGNTRILAYEFATMGSLHDMLHGRKGVKG-AQPGPVLSWTQRVKIAVG
HGNVVELLGYCVDGNTRILAYEFATMGSLHDMLHGRKGVKG-AQPGPVLSWTQRVKIAVG
HENVVELLGYCLDGNTRVLAYEFATMGSLHDMLHGRKGVKG-AQPGPVLSWIQRVKIAVG
HDNFVQLLGYCVDGNSRILSYEFANNGSLHDILHGRKGVKG-AQPGPVLSWYQRVKIAVG
DENVVELLGYCVDGGFRVLAYEYAPNGSLHDILHGRKGVKG-AQPGPVLSWAQRVKIAVG
DENVVELLGYCVDGGFRVLAYEYAPNGSLHDILHGRKGVKG-AQPGPVLSWAQRVKIAVG
HENIIQLIGYCAGGSIRVLAYEYAPRGSLHDILHGKKGVKG-AQPGPALSWMQRVKIALS
HPNLVKLIGFCAEGDQRLLVYEYMPLGSLENHLHDIP------PNRQPLDWNTRMKIAAG
NPNLVKLIGFCAEGDQRLLVYEYMPLGSLENHLHDIP------PNRQPLDWNARMKIAAG
HPNLVNLIGYCADGDQRLLVYEYMPLGSLEDHLHDIS------PSKQPLDWNTRMKIAAG
HPNLVTLLGYCTECDQKILVYEYMPLGSLQDHLLDLT------PKSQPLSWHTRMKIAVD
NPNLVKLIGYCIEDDQRLLVYEFLPRGSLENHLFRR---------SLPLPWSIRMKIALG
HPHLVSLIGFCDERNEMILIYDYMENGNLKSHLYG--------SDLPTMSWEQRLEICIG
HPHLVSLIGFCDERNEMILIYKYMENGNLKSHLYG--------SDLPSMSWEQRLEICIG
HPHLVSLIGYCDGRNEMILIYDYMENGNLKSHLYG--------SDLPSMSWEQRLEICIG
HPHLVSLIGYCDERNEMILIYEYMENGNLRSHLYG--------SDLPAMSWEQRLEICIG
HPHLVSLIGYCDETNEMILVYDYIENGNLRSHLYG--------PDLPTMSWEQRLEICIG
HPHLVSLIGYCDETNVMILVYDYIENGNLRSHLYG--------PDLPTMSWEQGLEICIG
HPHLVSLIGYCDERNEMILVYDYIENGNLRSHLYG--------SDLPSMSWEQRLEICIG
HPYLVSLIGYCDENNVTILIFKYMENGSLSSHLYG--------SYLPTMTWEQRLEICIG
HKNLVRLVGCCSEGTERLLVYEYMKNKSLDKILFAAADAPAPASAPPFLNWRTRHQIIIG
hxxxvxLxGyCxxxxxxxLxyexxxxgsLxxxLxgxxxxxx-xxxxpxxsWxqrxxIxxg

AAKGLEYLHEKAQPHIIHRDIKSSNVLLFDDDVAKIADFDLSNQ-APDMAARLHSTRVLG
AAKGLEYLHEKAQPHIIHRDIKSSNVLSFDDDVAKIADFDLSNQXAPDMAARLHSTRVLG
AAKGLEYLHEKAQPHIIHRDIKSSNVLLFDDDVAKIADFDLSNQ-APDMAARLHSTRVLG
AAKGLEYLHEKAQPHIMHRDIKSSNVLLFDDDVAKIADFDLSNQ-APDMAARLHSTRVLG
AAKGLEYLHEKAQPHIIHRDIKSSNVLLFDDDVAKIADFDLSNQ-APDMAARLHSTRVLG
AAKGLEYLHEKAQPHIIHRDIKSSNVLLFDDDVSKIADFDLSNQ-APDMAARLHSTRVLG
AAKGLEYLHEKAQPHIIHRDIKSSNVLLFDDDVSKIADFDLSNQ-APDMAARLHSTRVLG
AAKGLEYLHEKAQPHVIHRDIKSSNVLLFDDDVAKIADFDLSNQ-APDMAARLHSTRVLG
AARGLEYLHEKANPHIIHRDIKSSNVLLFEDDVAKIADFDLSNQ-APDMAARLHSTRVLG
AAKGLEYLHEKAQPHIIHRDIKSSNILLFDDDVAKIADFDLSNQ-APDMAARLHSTRVLG
AAKGLEYLHEKAQPHIIHRDIKSSNILLFDDDVAKIADFDLSNQ-APDMAARLHSTRVLG
AAKGLEFLHEKAEPRVVHRDIKSSNIMLFDNDVAKVGDFDVSNQ-SPDMAARLHSTRVLG
AAKGLEYLHNEMKPPVIYRDLKCSNILLGEGYHPKLSDFGLAKV-GPSGDKTHVSTRVMG
AAKGLEYLHNEMAPPVIYRDLKCSNILLGEGYHPKLSDFGLAKV-GPSGDHTHVSTRVMG
AAKGLEYLHDKTMPPVIYRDLKCSNILLGDDYFPKLSDFGLAKL-GPVGDKSHVSTRVMG
AARGLEYLHEVANPPVVYRDLKASNILLDGNFSAKLADFGLAKL-GPVGDKTHVTTRVMG
AAKGLAFLHEEAKRPVIYRDFKTSNILLDAEYNAKLSDFGLAKD-GPEGDKTHISTRVMG
AARGLHYLHTS---AVIHRDVKSTNILLDENFVAKITDFGISKK-GTELDQTHVSTDVKG
AARGLYYLHTR---AVIHRDVKSINILLDENFVPKITDFGISKK-GTELDQTHLSTVVQG
AARGLHYLHTN---GVMHRDVKSSNILLDENFVPKITDFGLSKT-RPQLYQTHVSTDVKG
AARGLHYLHTS---AVIHRDVKSINILLDDNFVPKITDFGLSKT-GTELDQTHVSTAVKG
AARGLHYLHTS---AVIHRDVKSINILLDENFVAKITDFGISKK-GTELDQTHLSTLVQG
AARGLHYLHTS---AVIHRDVKSINILLDENFVAKITDFGISKK-GTELDQTHLSTLVQG
AARGLHYLHTS---AVIHRDVKSINMLLDENFVAKITDFGLSKK-GTELDQTHLSTLVQG
AARGLYYLHKN---AVIHRDVKSANILLDENFVAKTTDFGVSKT-RTELDQTHVSTVVKG
IGRGLQYLHEESNLRIVHRDIKASNILLDDKFQPKISDFGLAR--FFPEDQTYLSTAFAG

aaxGLxyLHxxxxxxxihRDxKssNxllxxxxvxKixDFxlsxx-xxxxxxxxxsTxvxG

TFGYHAPEYAMTGQLSSKSDVYSFGVVLLELLTGRKPVDHTLPRGQQSLVTWATPRLSED
TFGYHAPEYAMTGQLSSKSDVYSFGVVLLELLTGRKPVDHTLPRGQQSLVTWATPXLSED
TFGYHAPEYAMTGQLSSKSDVYSFGVVLLELLTGRKPVDHTLPRGQQSLVTWATPRLSED
TFGYHAPEYAMTGQLSSKSDVYSFGVVLLELLTGRKPVDHTLPRGQQSLVTWATPRLSED
TFGYHAPEYAMTGQLSSKSDVYSFGVVLLELLTGRKPVDHTLPRGQQSLVTWATPRLSED
TFGYHAPEYAMTGQLSSKSDVYSFGVVLLELLTGRKPVDHTLPRGQQSLVTWATPRLCED
TFGYHAPEYAMTGQLSSKSDVYSFGVVLLELLTGRKPVDHTLPRGQQSLVTWATPRLCED
TFGYHAPEYAMTGQLSSKSDVYSFGVVLLELLTGRKPVDHTLPRGQQSLVTWATPRLSED
TFGYHAPEYAMTGQLNAKSDVYSFGVVLLELLTGRKPVDHRLPRGQQSLVTWATPKLSED
TFGYHAPEYAMTGQLSSKSDVYSFGVVLLELLTGRKPVDHTLPRGQQSLVTWATPRLSED
TFGYHAPEYAMTGQLSSKSDVYSFGVVLLELLTGRKPVDHTLPRGNR-VCYLGNARLSED
TFGYHAPEYAMTGQLSTKSDVYSFGVVLLELLTGRKPVDHTLPRGQQSLVTWATPRLSED
TYGYCAPDYAMTGQLTFKSDIYSFGVVLLELITGRKAIDQRKERGEQNLVAWARPMFKDR
TYGYCAPDYAMTGQLTFKSDVYSFGVVLLELITGRKAIDQTKERSEQNLVAWARPMFKDR
TYGYCAPEYAMTGQLTLKSDVYSFGVVLLEIITGRKAIDNSRCTGEQNLVAWARPLFKDR
TYGYCAPEYAMSGKLTKMSDIYCFGVVLLELITGRRAIDTTKPTREQILVHWAAPLFKDK
TYGYAAPEYVMTGHLSSKSDVYSFGVVLLEMLTGRRSMDKKRPNGEHNLVEWARPHLGER
TFGYLDPEYFIKGRLTEKSDVYSFGVVLFEVLCARSAIVQSLPREMVNLAEWAVESHNNG
TLGYLDPEYFIKGRLTEKSDVYSFGVVLFEVLCARSAIVQSLPREMVNLAEWAVESHNNG
TFGYIDPEYFIKGRLTEKSDVYSFGVVLFEVLCARSAIVQSLPSEMVNLAEWAVESHNNG
TVGYLDPEYFIRGQLTEKSDVYSFGVVLFEVLCARPAIAHSHSREMISLAEWAVESHNNG
TIGYLDPEYFIRGQLTEKSDVYSFGVVLFEVLCARPAIVQSLPREMVNLAEWAVDSHNKG
TIGYLDPEYFLRGQLTEKSDVYSFGVVLFEVLFARPAIVQSLPREMVSLAEWAVDSHNKG
TIGYLDPEYFIRGQLTEKSDVYSFGVVLFEVLCARPAIVQSLPREMVNLAEWAVDSHNKG
TLGYLDPEYVIRGKLTEKSDVYSFGVVLFKVLCARSAIVHYISKGLVTLAAWAMDSHKKG
TLGYTAPEYAIRGELTVKADTYSFGVLVLEIISSRKNTDLNLPNEMQYLPEHAWRLYEQS
TxGYxxPeYxxxGqLxxksDvYsFGVvlxexlxxRxxxxxxlprxxxxlxxwaxxxxxxx

K-VRQCVDSRLGGD--YPPKAVAKFAAVAALCVQYEADFRPNMSIVVKALQPLLNAHAR-
K-VRQCVDSRLGGD--YPPKAVAKFAAVAALCVQYEADFRPNMSIVVKALQPLLNAACAG
K-VRQCVDSRLGGD--YPPKAVAKFAAVAALCVQYEADFRPNMSIVVKALQPLLNAHAR-
K-VRQCVDSRLGGD--YPPKAVAKFAAVAALCVQYEADFRPNMSIVVKALQPLLNAHARA
K-VRQCVDSRLGGD--YPPKAVAKFAAVAALCVQYEADFRPNMSIVVKALQPLLNARATN
K-VRQCVDSRLGVE--YPPKSVAKFAAVAALCVQYEADFRPNMSIVVKALQPLLNARASN
K-VRQCVDSRLGVE--YPPKSVAKFAAVAALCVQYEADFRPNMSIVVKALQPLLNARASN
K-VRQCVDSRLGGD--YPPKAVAKFAAVAALCVQYEADFRPNMSIVVKALQPLLNARAAH
K-VKQCVDARLGGD--YPPKAVAKLAAVAALCVQYEADFRPNMSIVVKALQPLLNARAVA
K-VKQCVDARLNTD--YPPKAIAKMAAVAALCVQYEADFRPNMSIVVKALQPLLPRPVPS
K-VKQCVDARLNTD--YPPKAIAKMAAVAALCVQYEADFRPNMSIVVKLFSLCCLDLYQV
K-VKQCVDPRLEGD--YPPKAVAKMAAVAALCVQYEADFRPNMSIVVKALNPLLNSRPNN
RNFSCMVDPLLQGQ--YPIRGLYQALAIAAMCVQEQPNMRPAVSDLVMALNYLASHKYDP
RNFSGMVDPFLQGQ--YPIKGLYQALAIAAMCVQEQPNMRPAVSDVVLALNYLASHKYDP
RKFSQMADPMIQGQ--YPPRGLYQALAVAAMCVQEQPNLRPVIADVVTALTYLASQRFDP
KKFTKMADPLLDSK--YPLKGLYQALAISSMCLQEEAISRPLISDVVTALTFLADPNYDP
RRFYRLIDPRLEGH--FSIKGAQKAAQLASRCLSRDPKARPLMSEVVDCLKPLPALKDMA
Q-LEQIVDPNLADK--IRPESLRKFGDTAVKCLALSSEDRPSMGDVLWKLEYALRLQESV
Q-LEQIIDPNLADK--ITPESLRKFGETAVKCLALSSEDRPSMGDVLWKLEYALRLQESV
Q-LEQIIDPNLAAK--IRPESLRKFGETAVKCLALSSEDRPSMGDVLWKLEYALRLQESV
Q-LEQIIAPNIAAK--IRPESLKKFGETVVKCLALSSEDRPSMGDVLWKLEYALRLQESV
H-LEQIIDPDLAAK--IRPESLRKFGETAVKCLALSSEDRPSMGDVL-------------
Q-LEQIVDPDLAAK--IRPESLRKFGETAVKCLALSSEDRPSMGDVL-------------
Q-LEQIIDLNLAAK--IRPESLRKFGETAVKCLALSSGDRPSMGDVL-------------
Q-LEQIVDPNLASK--IRPKYLNKFGETAVKCLADSGVDRPSVGDVL-------------
K-ILELVDGRVQGGEGFEEKEVMLVCQIALLCVQPYPNSRPAMSEVVRMLTMKTDQSIPA
x-xxqxxdxxlxxx--xxpxxxxkxxxxaxxCxxxxxxxRPxmxxvxxxlxxxxxxxxxx

ATNPGEHAGS--------------------------------------------------
RPNPGEHAGS--------------------------------------------------

ATNPGEHAGS----------------------------------------------------
TNP-----------------------------------------------------------
PGENAGS-------------------------------------------------------
NPG-----------------------------------------------------------
NPG-----------------------------------------------------------
PGAEHAGR------------------------------------------------------
PGEGVH--------------------------------------------------------
--------------------------------------------------------------
RHQACEFSPYPCLYVMK---------------------------------------------
RPASFTDAGERSGL------------------------------------------------
---QVHSVQDSRRSPSRPGLDKDRGQ------------------------------------
---QIHPFKDPRRRPSHPGLDKDNGRT-----------------------------------
---MSQPVQGSLFGPGTPPRSKRVV-------------------------------------
---PDDIEPLPISVPNYDKGISLREAEISLSGFEEKQVEDS---------------------
GPSYYLQTVQPERAGSSPDPNRTRVGSFSRNGSQHPRTLSIPNASPRHNQFLQDSPNPNGKQ
I-------------------------------------------------------------
I-------------------------------------------------------------
I-------------------------------------------------------------
I-------------------------------------------------------------
--------------------------------------------------------------
--------------------------------------------------------------
--------------------------------------------------------------
--------------------------------------------------------------
PAKPAFLDRKNLNGDRDAASSDTATMEMMRSPAGYWMMTPSPMLEVDRPYDMSFGK------
xxxxxxxxxxxxxx------------------------------------------------

### Example 4 corn transformation construct

GATEWAY^{™} destination vectors (available from Invitrogen Life Technologies, Carlsbad, CA) were constructed for insertion of trait-improving DNA for corn transformation. The elements of each destination vector are summarized in Table 16 below and include a selectable marker transcription region and a DNA insertion transcription region. The selectable marker transcription region comprises a Cauliflower Mosaic Virus 35S promoter operably linked to a gene encoding neomycin phosphotransferase II (*npt*II) followed by both the 3' region of the *Agrobacterium tumefaciense* nopaline synthase gene (*nos*) and the 3' region of the potato proteinase inhibitor II (*pin*II) gene. The DNA insertion transcription region comprises a rice actin 1 promoter, a rice actin 1 exon 1 intron1 enhancer, an *att-*flanked insertion site and the 3' region of the potato *pin*II gene. Following standard procedures provided by Invitrogen the *att*-flanked insertion region is replaced by recombination with trait-improving DNA, in a sense orientation for expression of a trait-improving protein and in a gene suppression orientation (i.e. either anti-sense orientation or in a sense-and anti-sense orientation) for a trait-improving suppression of a protein.. Although the vector with trait-improving DNA inserted at the *att*-flanked insertion region is useful for plant transformation by direct DNA delivery, such as microprojectile bombardment, it is preferable to bombard target plant tissue with tandem transcription units that have been cut from the vector. For *Agrobacterium-mediated* transformation of plants the vector also comprises T-DNA borders from *Agrobacterium* flanking the transcription units.
Vectors for *Agrobacterium-mediated* transformation are prepared with each of the trait-improving genes having a sequence of SEQ ID NO: 1 through SEQ ID NO: 151 and SEQ ID NO: 153 through SEQ ID NO: 239 with the DNA solely in sense orientation for expression of the cognate trait-improving protein and in a gene suppression orientation for suppression of the cognate protein. Each vector is transformed into corn callus which is propagated into a plant that is grown to produce transgenic seed. Progeny plants are self-pollinated to produce seed which is selected for homozygous seed. Homozygous seed is used for producing inbred plants, for introgressing the trait into elite lines, and for crossing to make hybrid seed. The progeny transgenic plants comprising the trait-improving DNA with a sequence of SEQ ID NO:1 through SEQ ID NO: 151 and SEQ ID NO: 153 through SEQ ID NO: 239 have one or more improved traits including, but not limited to increased yield and those disclosed in Table 3. Transgenic corn including inbred and hybrids are also produced with DNA from each of the identified homologs and provide seeds for plants with the improved trait of the cognate DNA of SEQ ID NO: 1 through SEQ ID NO: 151 and SEQ ID NO: 153 through SEQ ID NO: 239. Transgenic corn plants are also produced where the trait-improving DNA is transcribed by each of the promoters from the group selected from, a maize globulin 1 promoter, a maize oleosin promoter, a glutelin 1 promoter, an aldolase promoter, a zein Z27 promoter, a pyruvate orthophosphate dikinase (PPDK) promoter, a soybean 7S alpha promoter, a peroxiredoxin antioxidant (Per1) promoter and a CaMV 35S promoter.

Seed produced by the plants is provided to growers to enable production of corn crops with improved traits associated with the trait-improving DNA.

**Table 16: Elements of an exemplary corn transformation vector**

| **FUNCTION** | **ELEMENT** | **REFERENCE** |
|---|---|---|
| DNA insertion transcription region | Rice actin 1 promoter | U.S. Patent 5,641,876 |
| | Rice actin 1 exon 1, intron 1 enhancer | U.S. Patent 5,641,876 |
| DNA insertion transcription region (*att* -flanked insertin region) | *Att*R1 | GATEWAY^{™}Cloning Technology Instruction Manual |
| | CmR gene | GATEWAY^{™}Cloning Technology Instruction Manual |
| | *ccd*A, *ccd*B genes | GATEWAY^{™}Cloning Technology Instruction Manual |
| | *attR2* | GATEWAY^{™}Cloning Technology Instruction Manual |
| DNA insertion | Potato pinII 3' region transcription region | An et al. (1989) Plant Cell 1:115-122 |
| selectable marker transcription region | CaMV 35S promoter | U.S. Patent 5,858,742 |
| | nptII selectable marker | U.S. Patent 5,858,742 |
| | nos 3region | U.S. Patent 5,858,742 |
| | ***PinII 3' region*** | An et al. (1989) Plant Cell 1:115-122 |
| *E. coli* maintenance region | *ColE1* origin of replication | |
| | F1 origin of replication | |
| | *Bla* ampicillin resistance | |

### Example 5 soybean transformation construct

Constructs for use in transformation of soybean may be prepared by restriction enzyme based cloning into a common expression vector. Elements of an exemplary common expression vector are shown in Table 17 below and include a selectable marker expression cassette and a gene of interest expression cassette. The selectable marker expression cassette comprises *Arabidopsis* act 7 gene (AtAct7) promoter with intron and 5'UTR, the transit peptide of *Arabidopsis* EPSPS, the synthetic CP4 coding region with dicot preferred codon usage and a 3' UTR of the nopaline synthase gene. The gene of interest expression cassette comprises a Cauliflower Mosaic Virus 35S promoter operably linked to a trait-improving gene in a sense orientation for expression of a trait-improving protein and in a gene suppression orientation (i.e. either anti-sense orientation or in a sense- and anti-sense orientation for a trait-improving suppression of a protein.

Vectors similar to that described above may be constructed for use in *Agrobacterium* mediated soybean transformation systems, with each of the trait-improving DNA having a sequence of SEQ ID NO: 1 though SEQ ID NO: 151 and SEQ ID NO: 153 through SEQ ID NO: 239 with the DNA solely in sense orientation for expression of the cognate protein and in a gene suppression orientation for suppression of the cognate protein. Transgenic soybean plants are produced comprising the trait-improving DNA with a sequence of SEQ ID NO: 1 through SEQ ID NO: 151 and SEQ ID NO: 153 through SEQ ID NO: 239 have one or more improved traits including, but not limited to, those disclosed in Table 3 and increased yield. Transgenic soybean plants are also produced with DNA from each of the identified homologs and provide seeds for plants with improved trait of the cognate DNA of SEQ ID NO: 1 through SEQ ID NO: 151 and SEQ ID NO: 153 through SEQ ID NO: 239. Transgenic soybean plants are also produced where the trait-improving DNA is transcribed by a desirable promoter including, but not limited to, the enhanced 35S promoter, napin promoter and *Arabidopsis* SSU promoter.
Seed produced by the plants is provided to growers to enable production of soybean crops with improved traits associated with the trait-improving DNA.

**TABLE 17: Elements of an exemplary soybean transformation construct**

| **Function** | **Element** | **Reference** |
|---|---|---|
| Agro transformation | B-ARGtu.right border | Depicker, A. et al (1982) Mol Appl Genet 1:561-573 |
| Antibiotic resistance | CR-Ec.aadA-SPC/STR | |
| Repressor of primers from the ColE1 plasmid | CR-Ec.rop | |
| Origin of replication | OR-Ec.oriV-RK2 | |
| Agro transformation | B-ARGtu.left border | Barker, R.F. et al (1983) Plant Mol Biol 2:335-350 |
| Plant selectable marker expression cassette | *Arabidopsis* act 7 gene(AtAct7) promoter with intron and 5'UTR | McDowell et al. (1996) Plant Physiol. 111:699-711. |
| | 5' UTR of *Arabidopsis* act 7 gene | |
| | Intron in 5'UTR of AtAct7 | |
| | Transit peptide region of *Arabidopsis* EPSPS | Klee, H.J. et al (1987) MGG 210:437-442 |
| | Synthetic CP4 coding region with dicot preferred codon usage | |
| | A 3' UTR of the nopaline synthase gene of Agrobacterium tumefaciens Ti plasmid | U.S. Patent 5,858,742 |
| Plant gene of interest expression cassette | Promoter for 35S RNA from CaMV containing a duplication of the -90 to -350 region | U.S. Patent 5,322,938 |
| | Gene of interest insertion site | |
| | Cotton E6 3' end | GenBank accession U30508 |

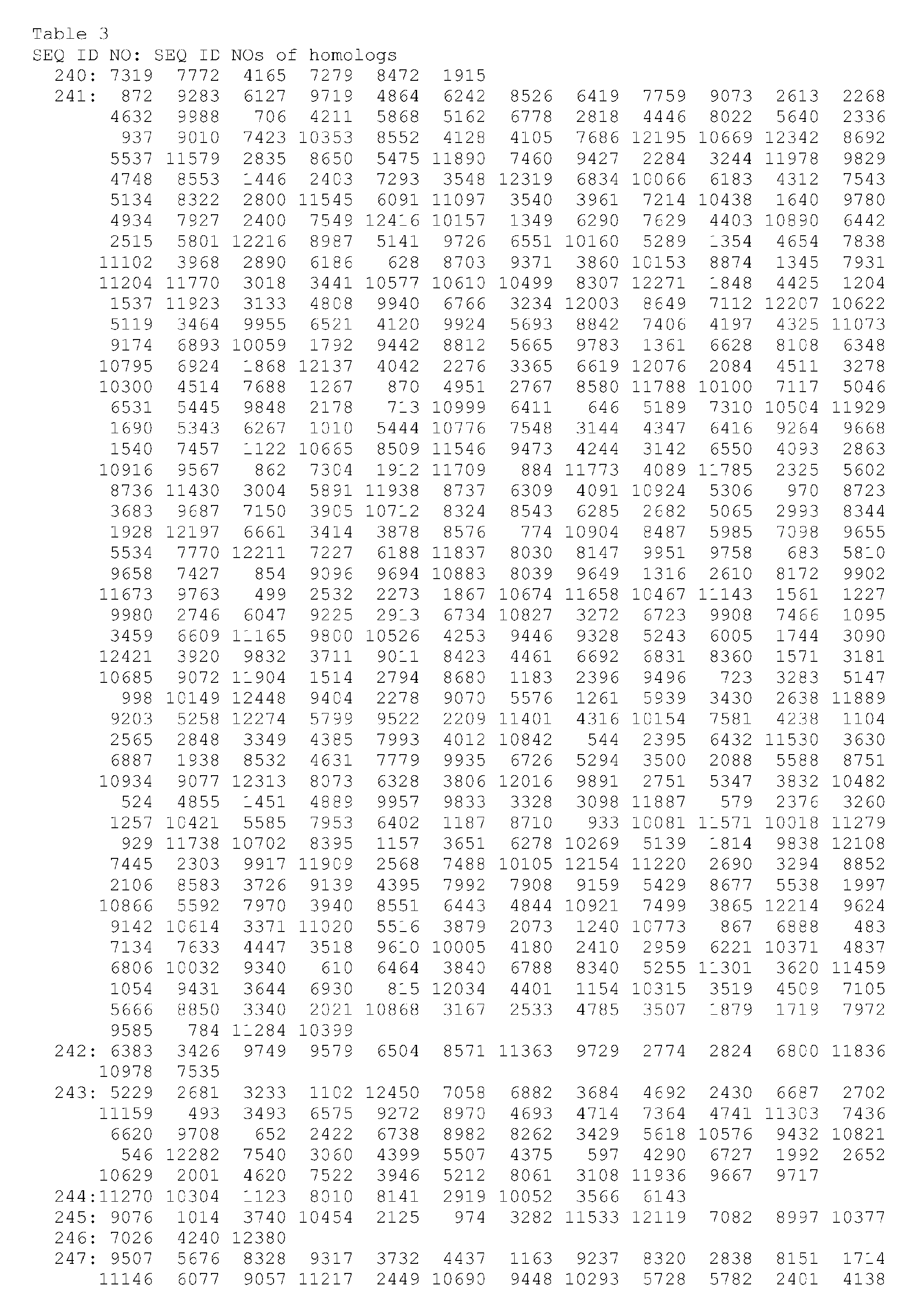

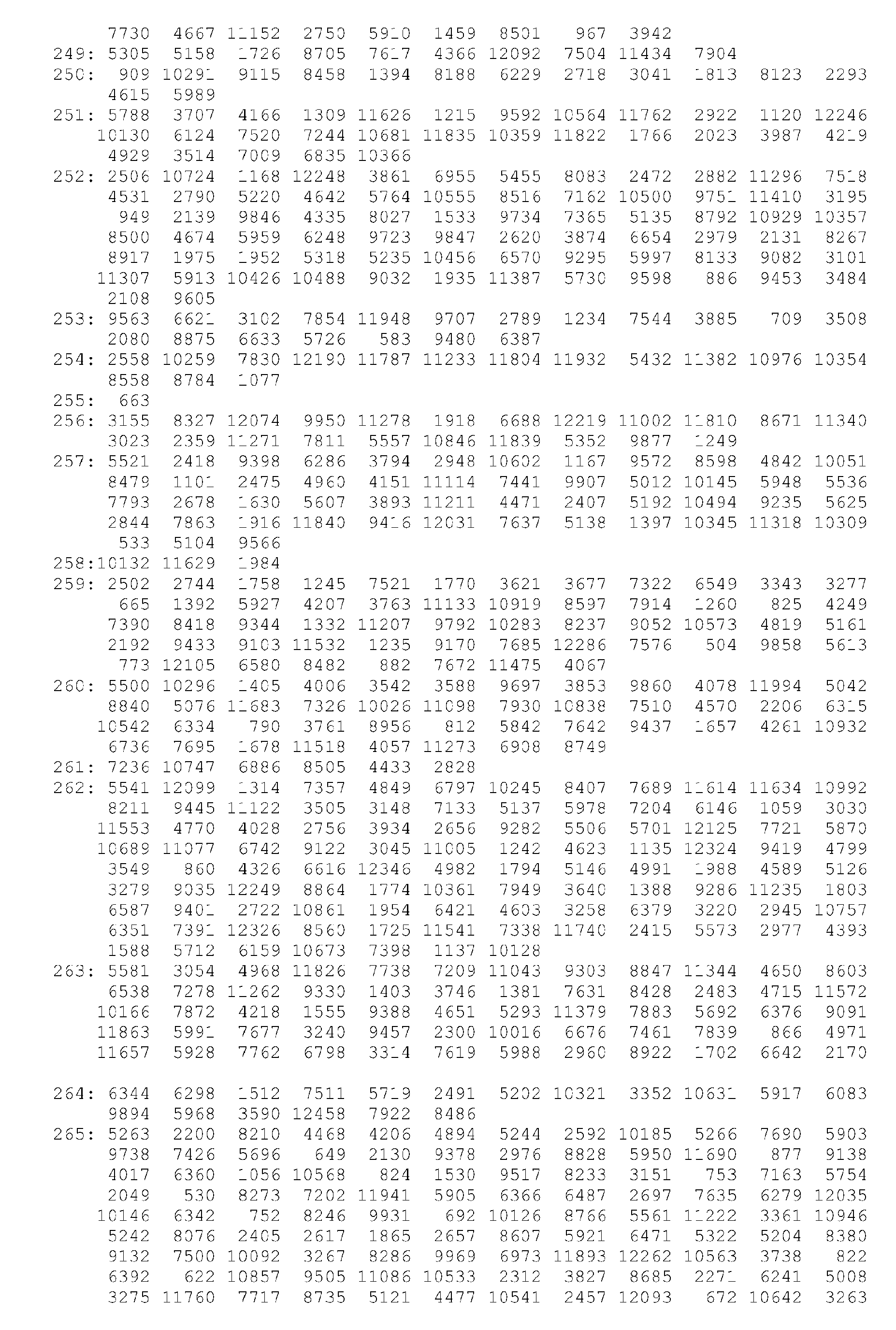

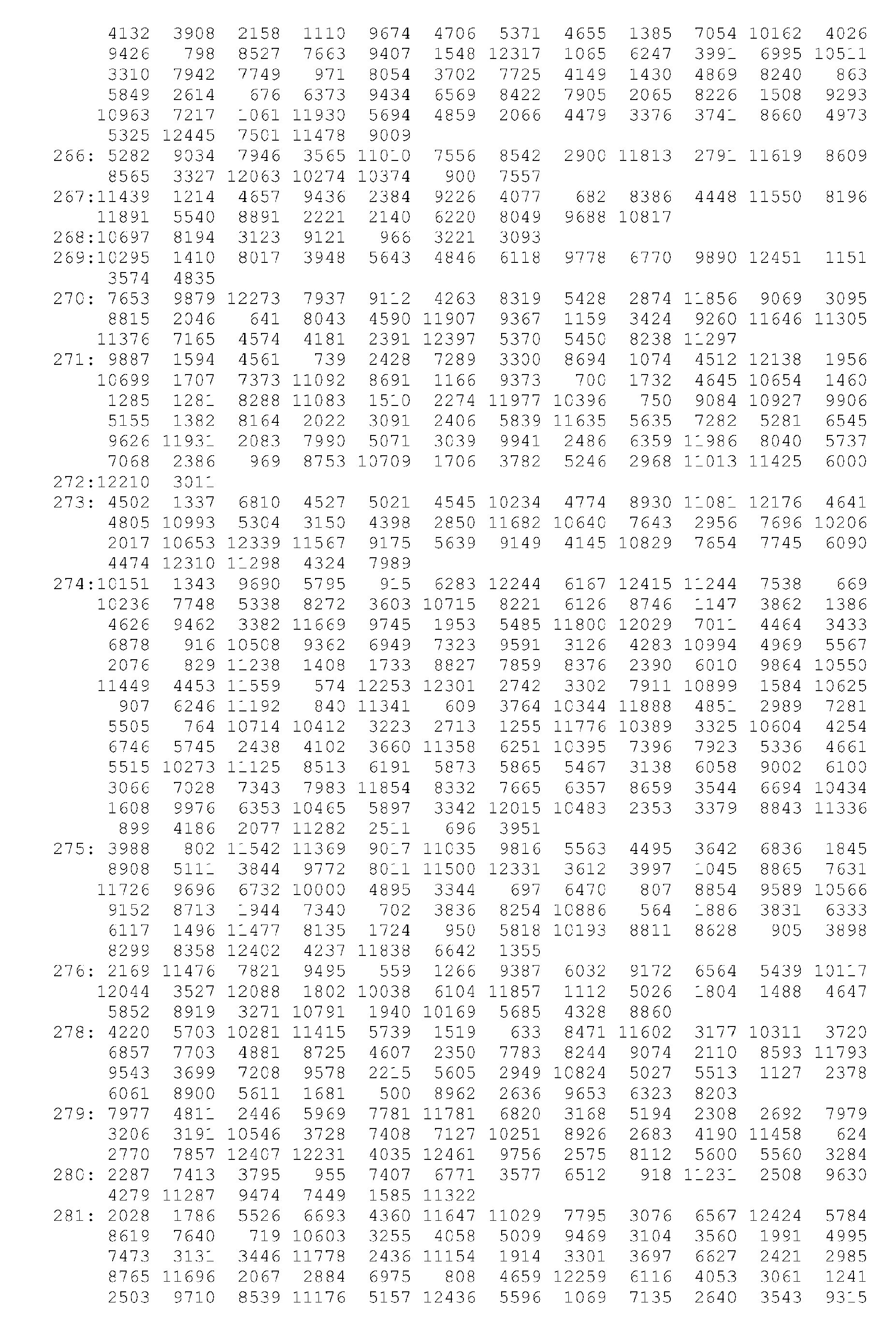

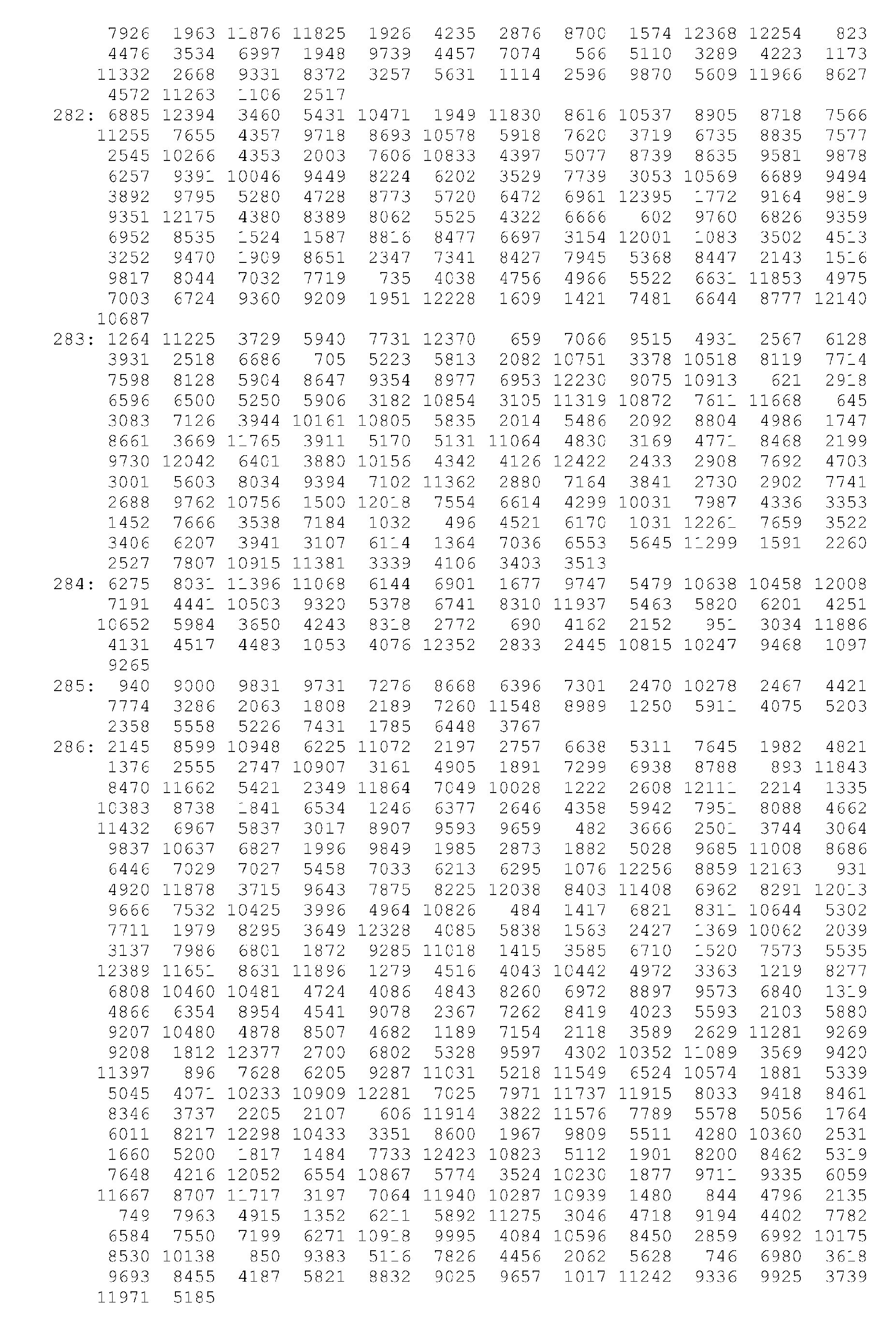

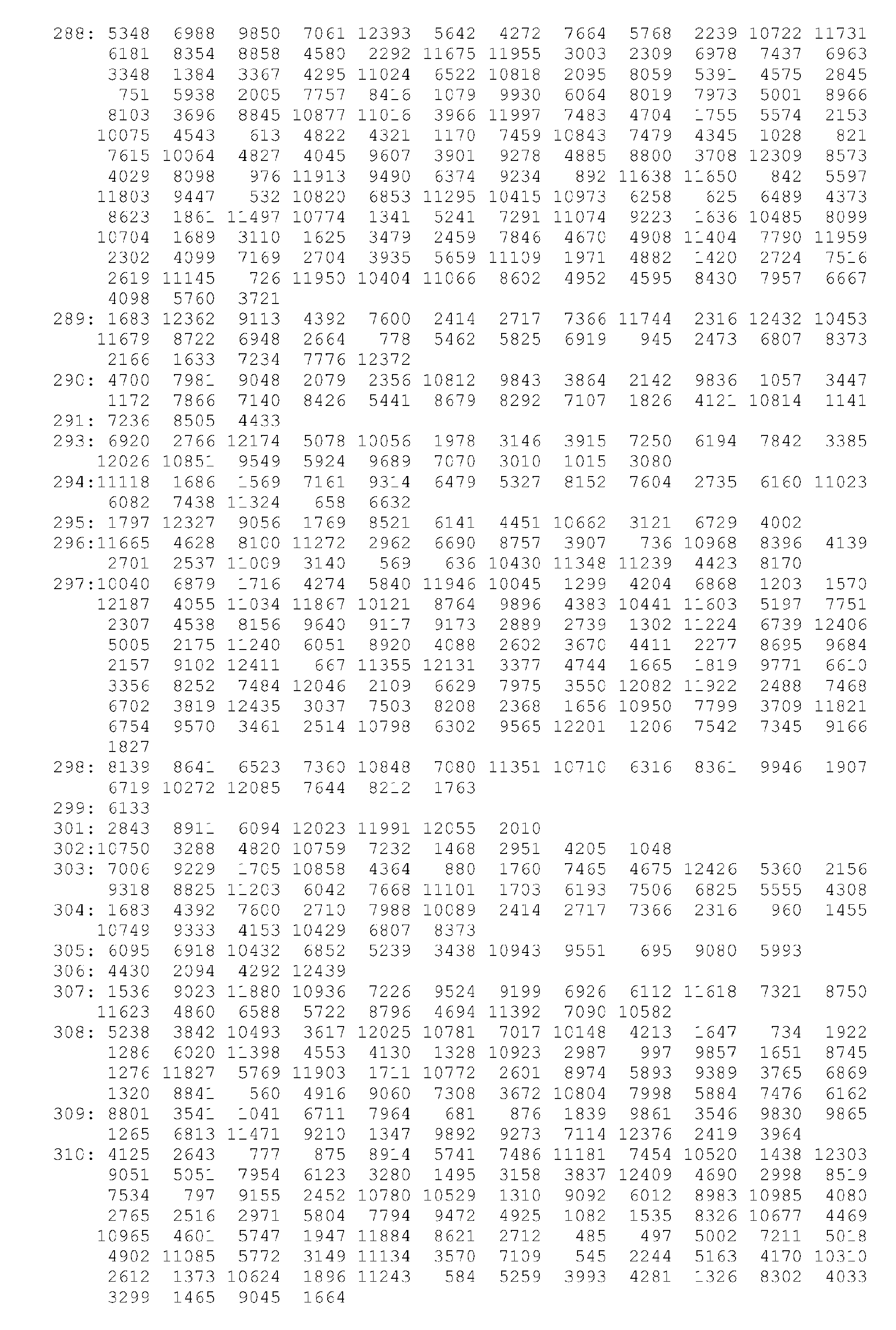

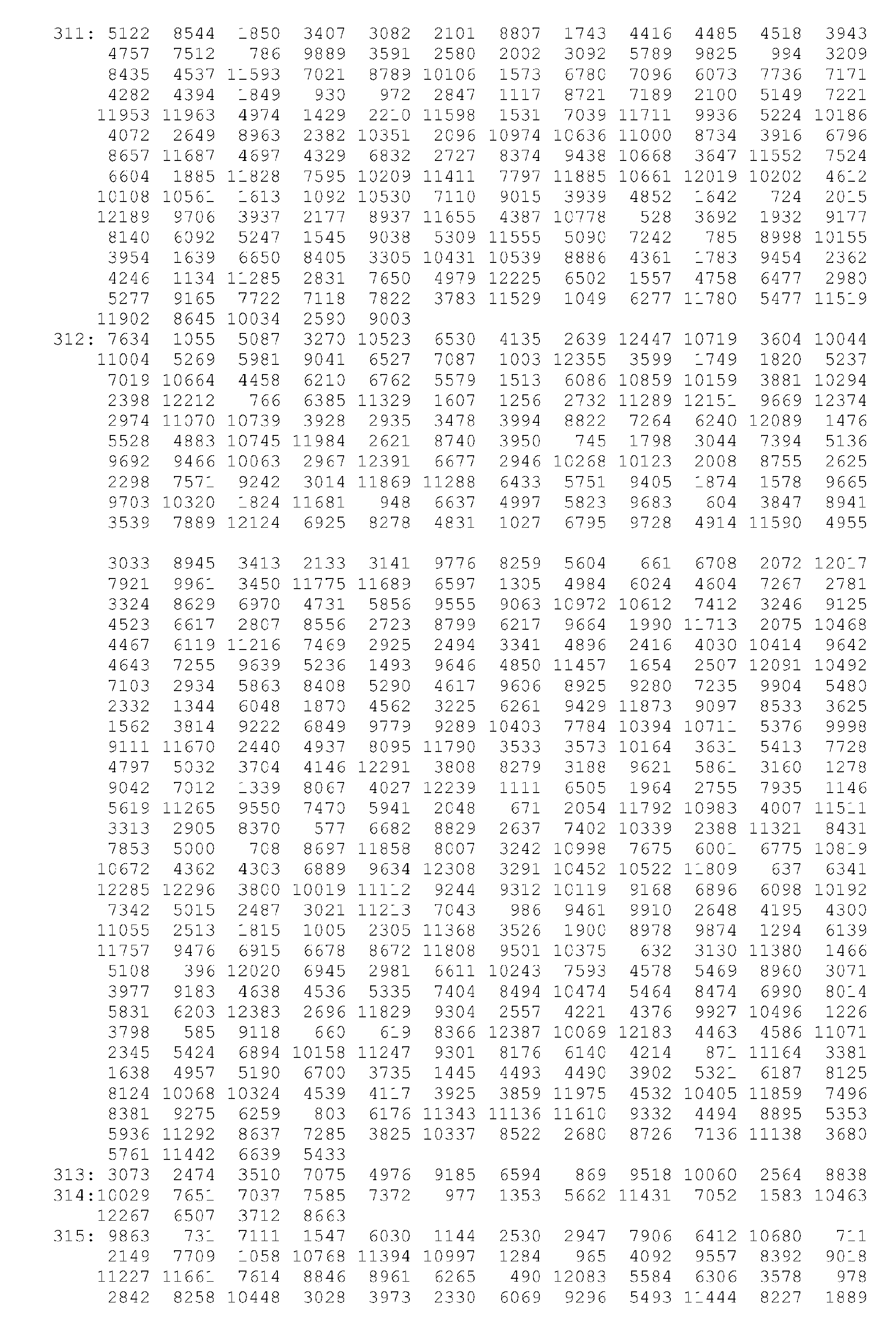

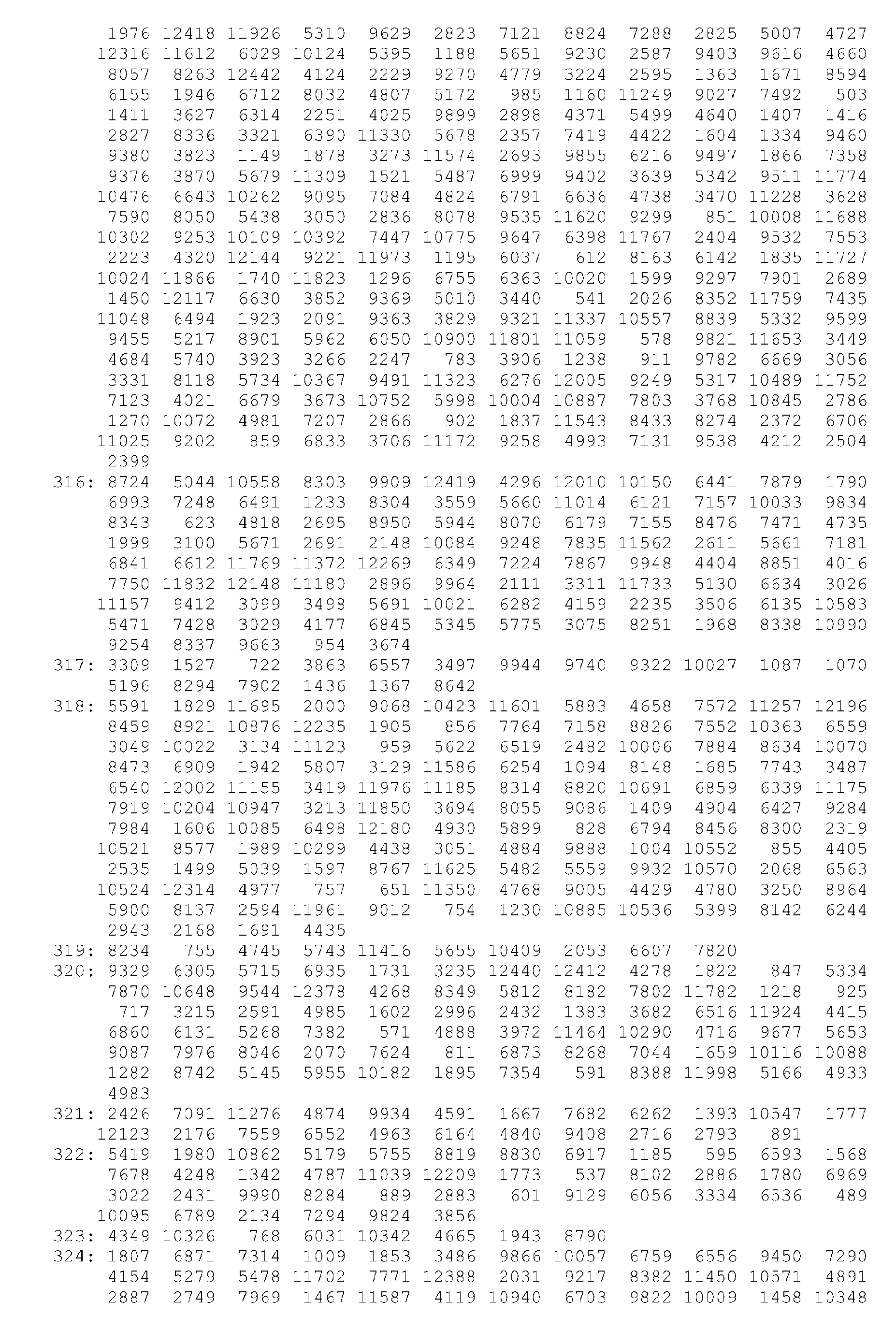

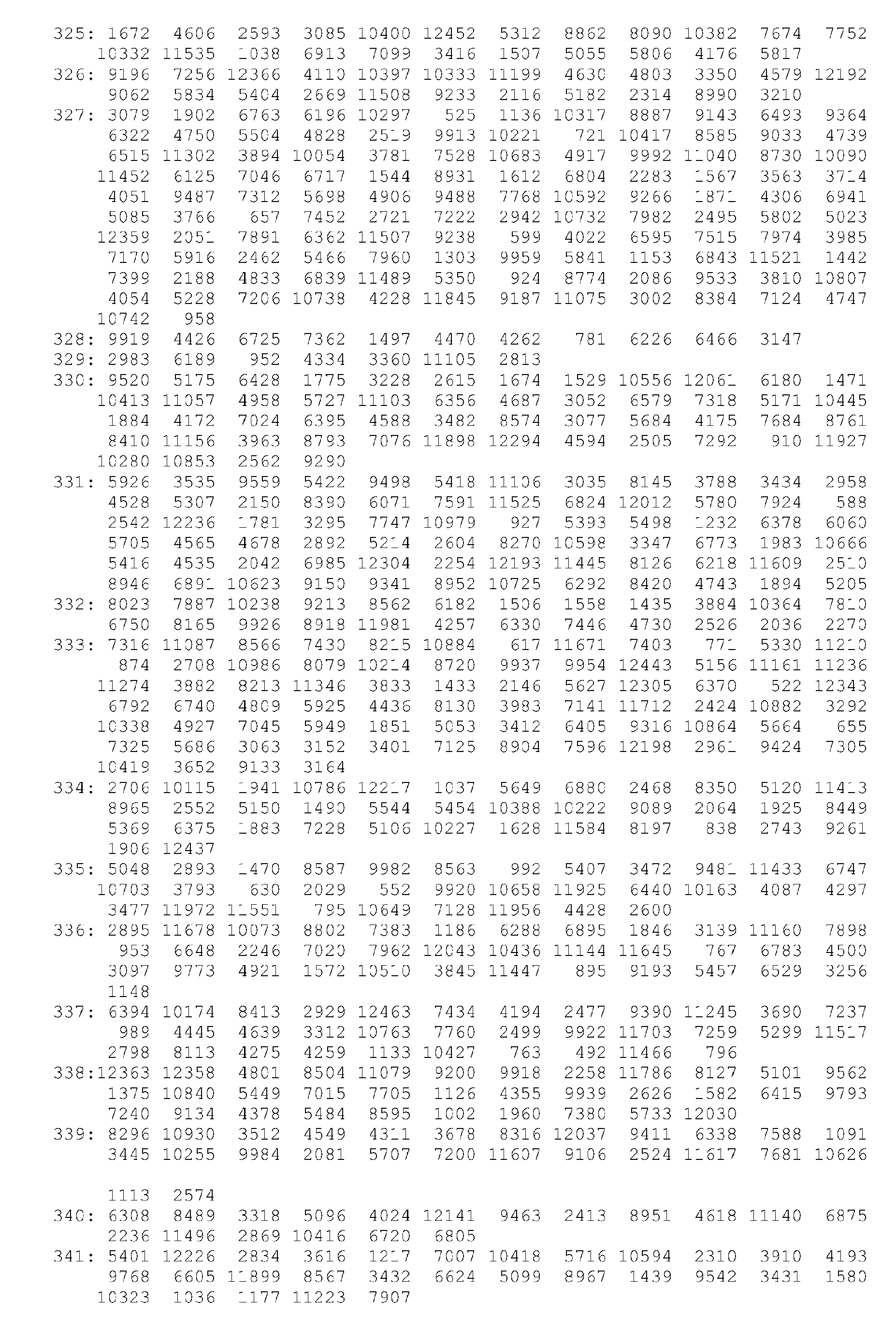

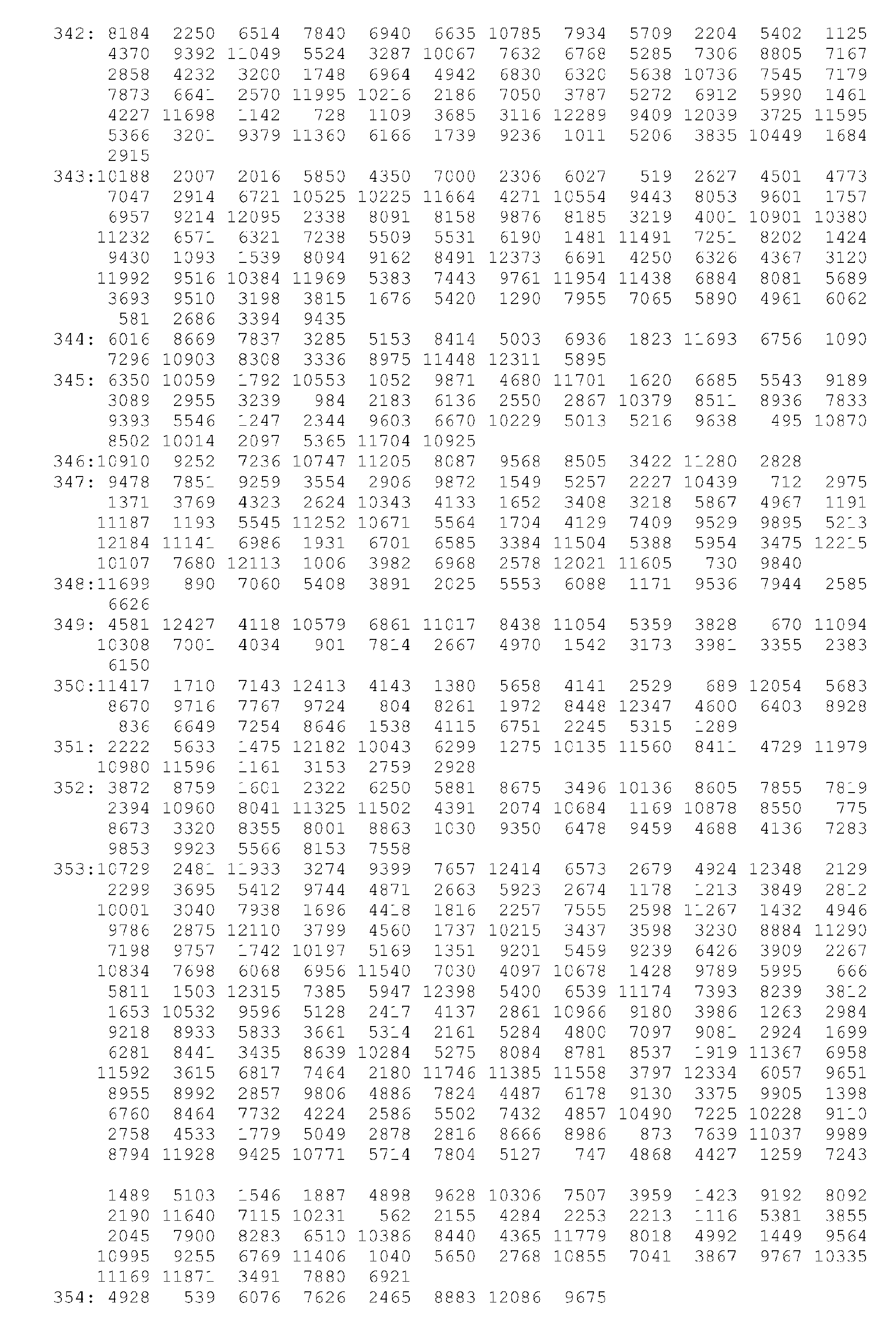

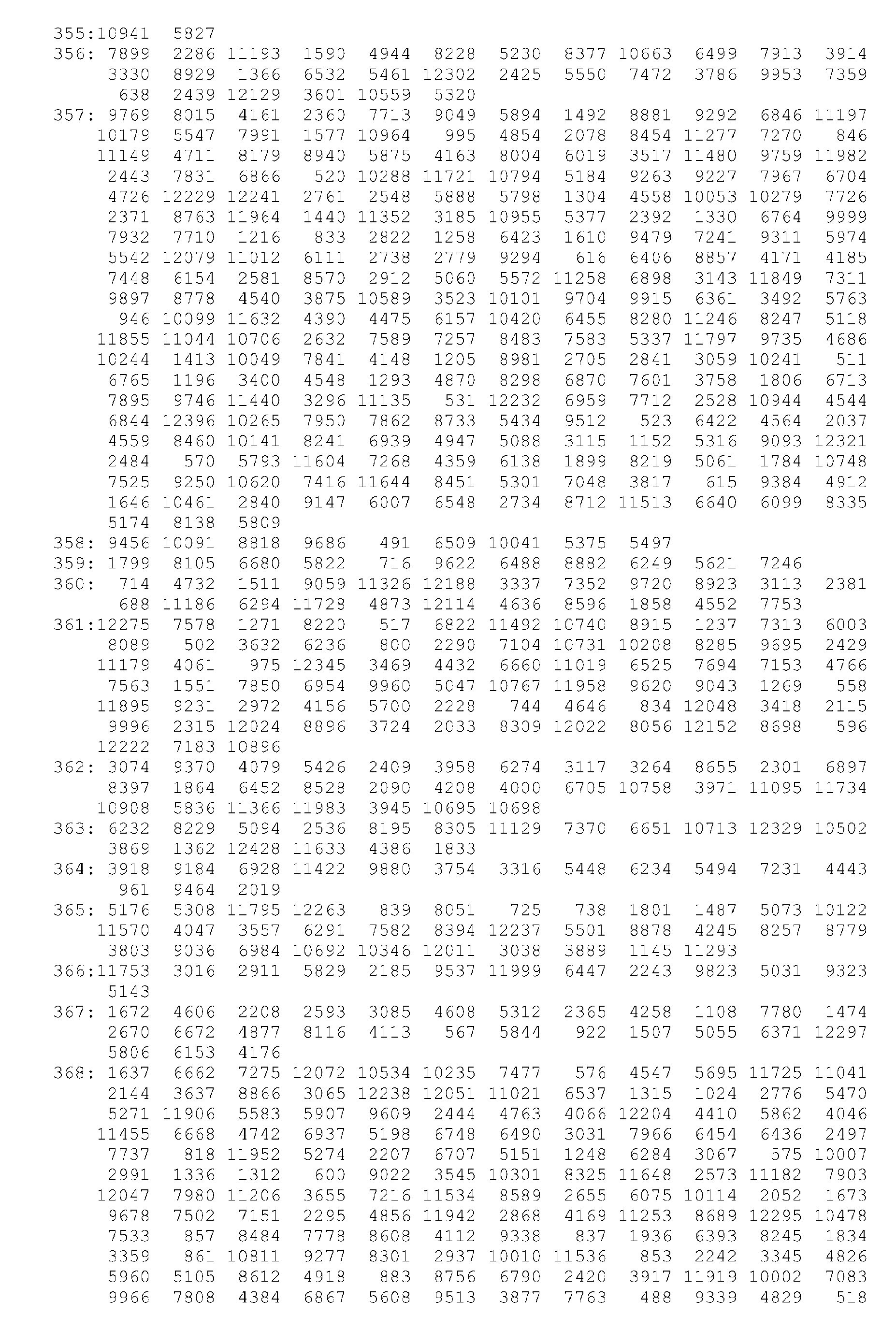

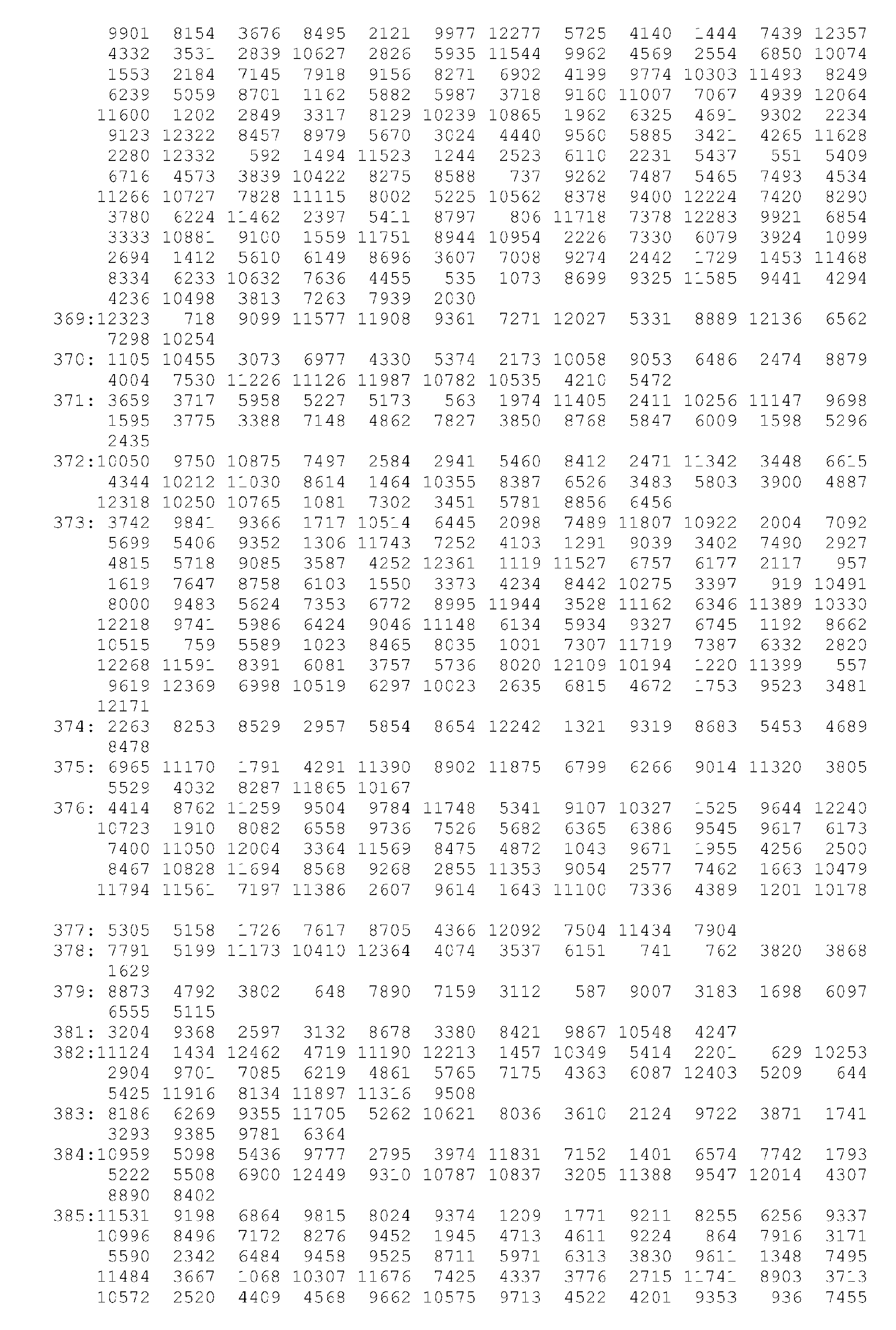

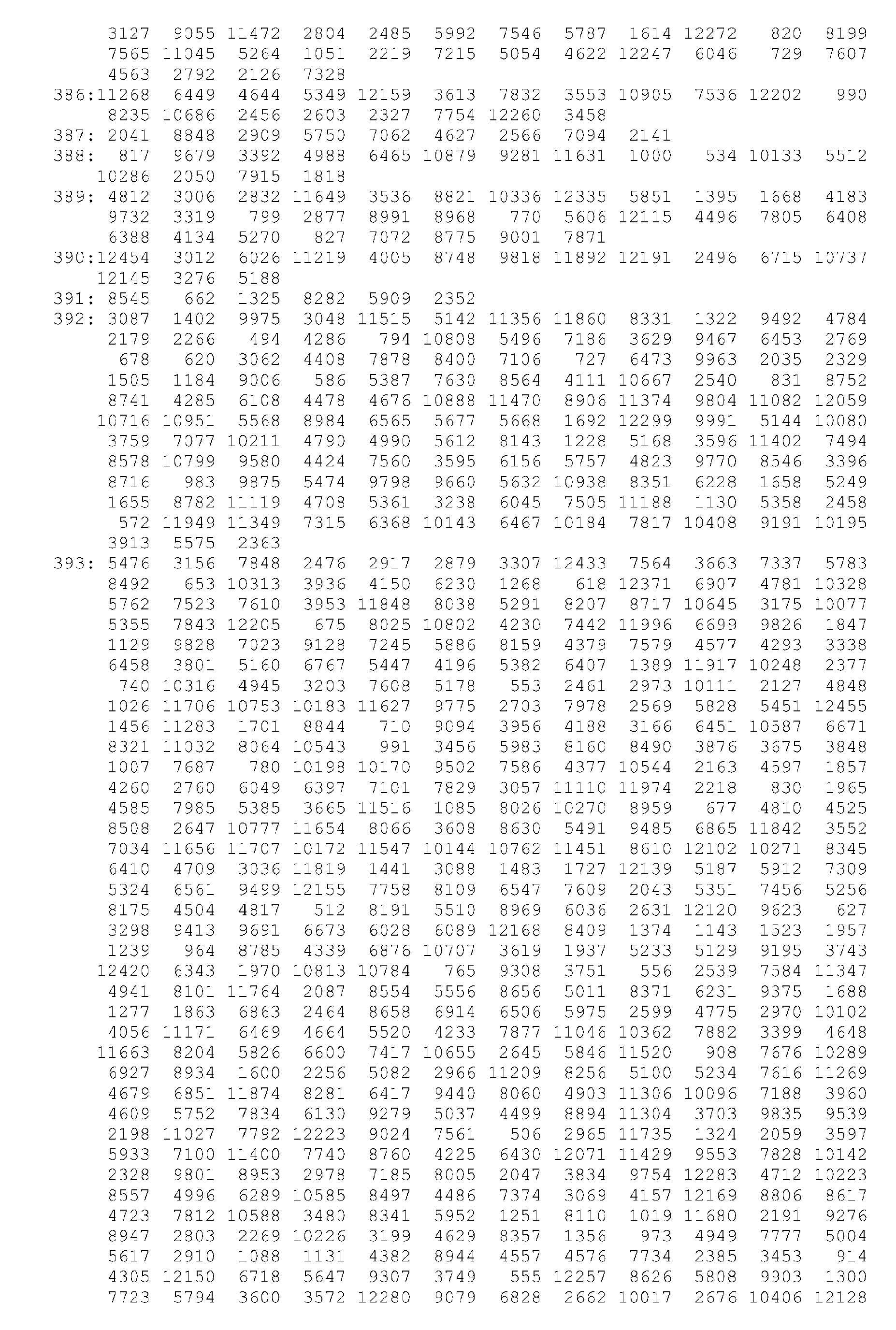

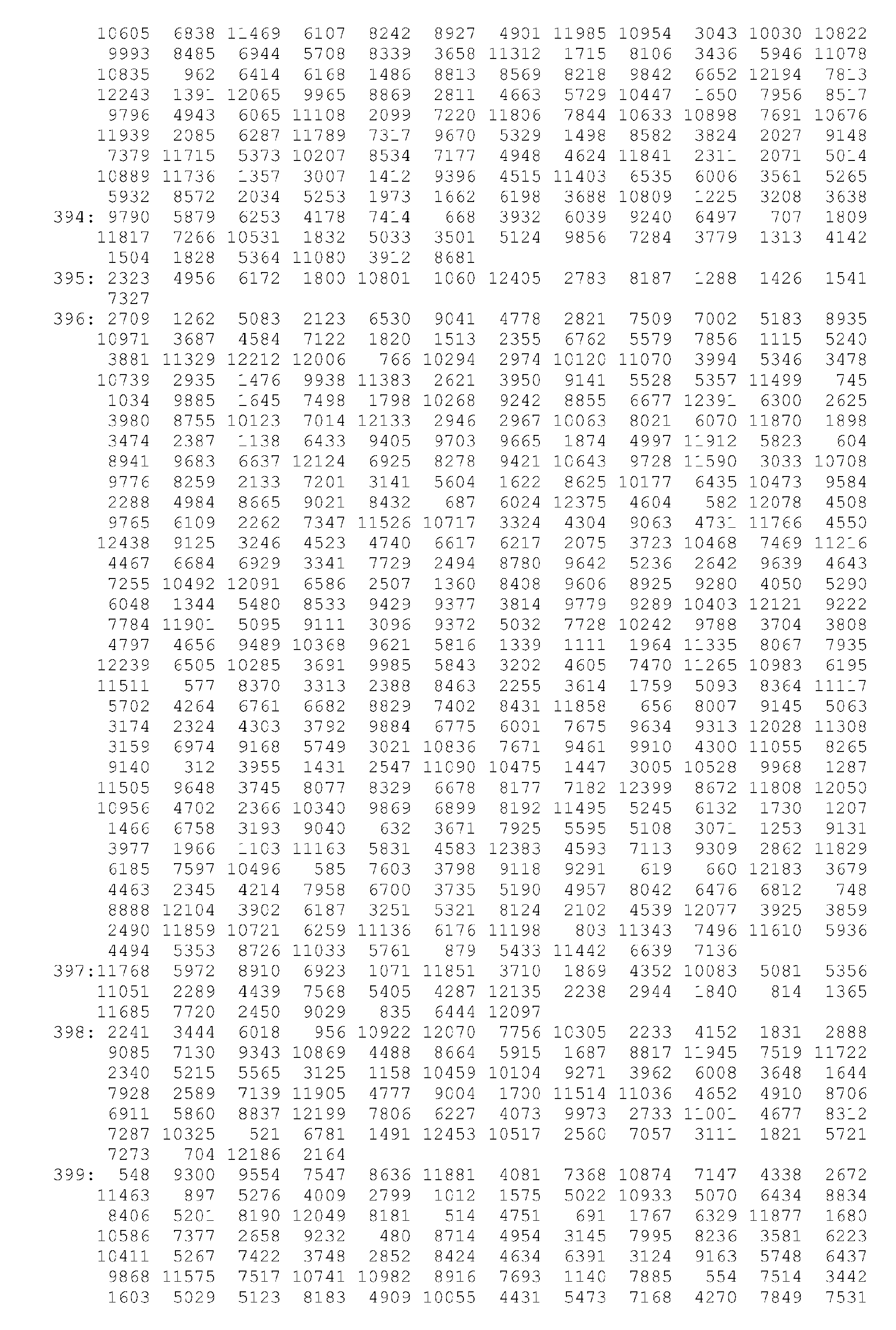

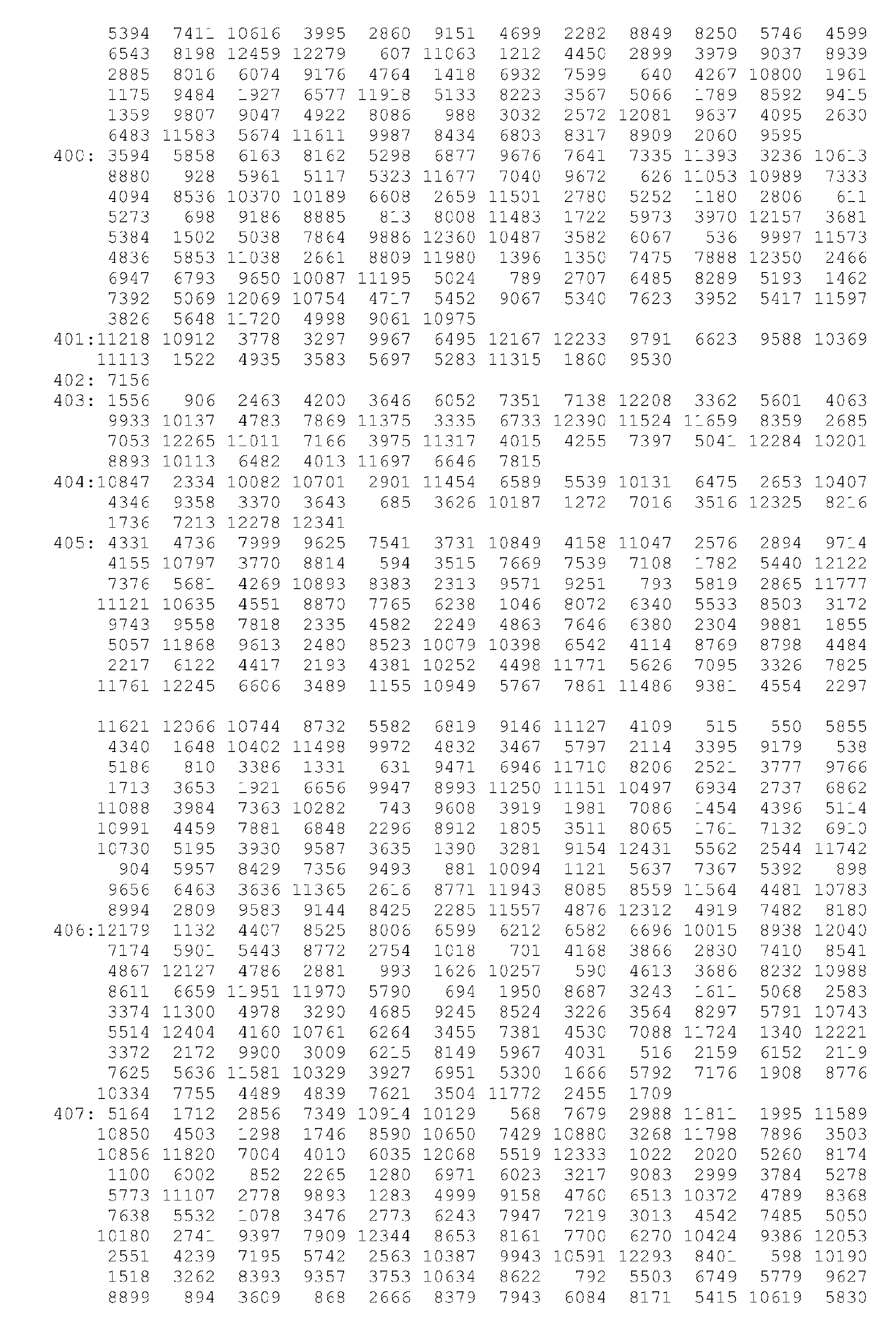

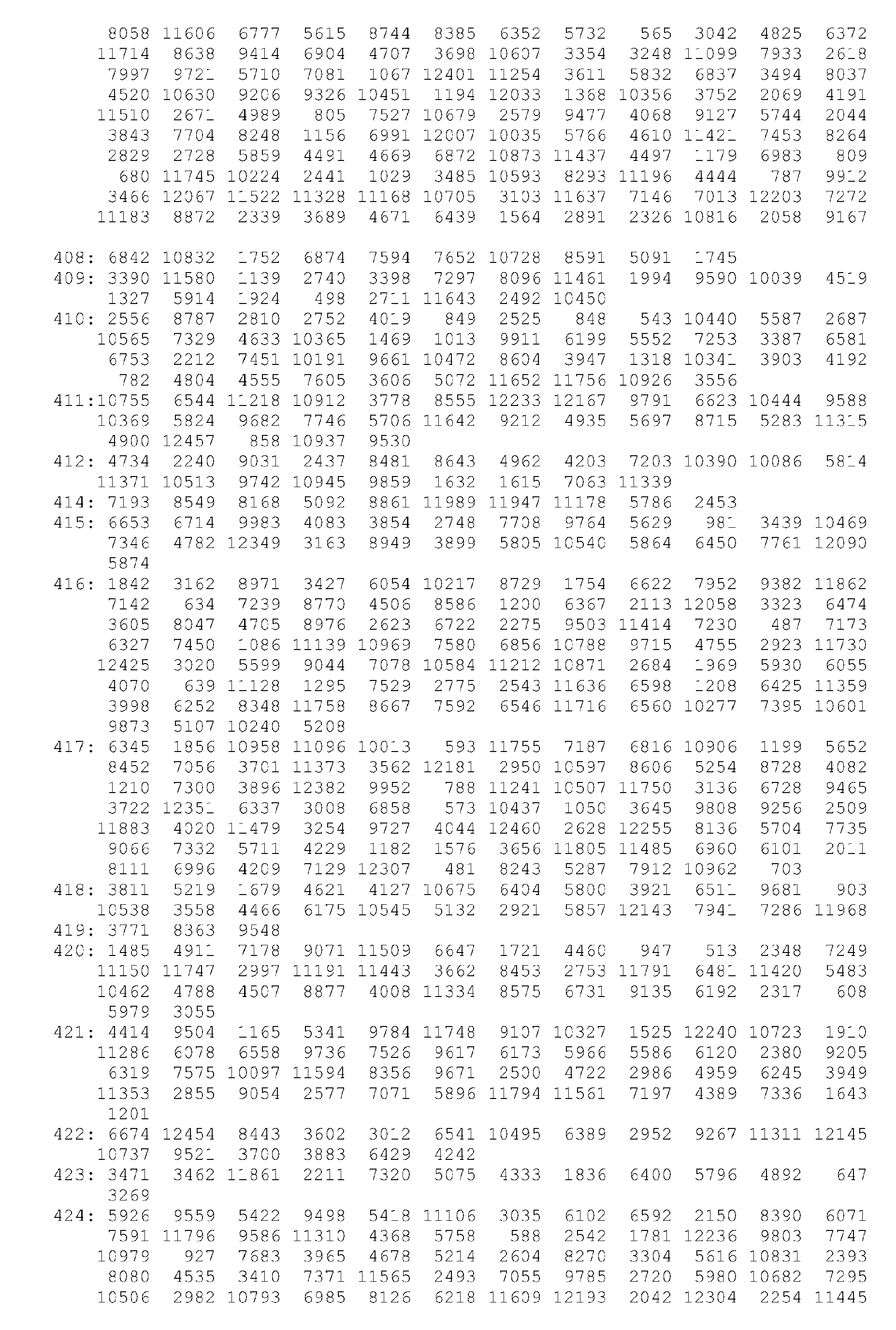

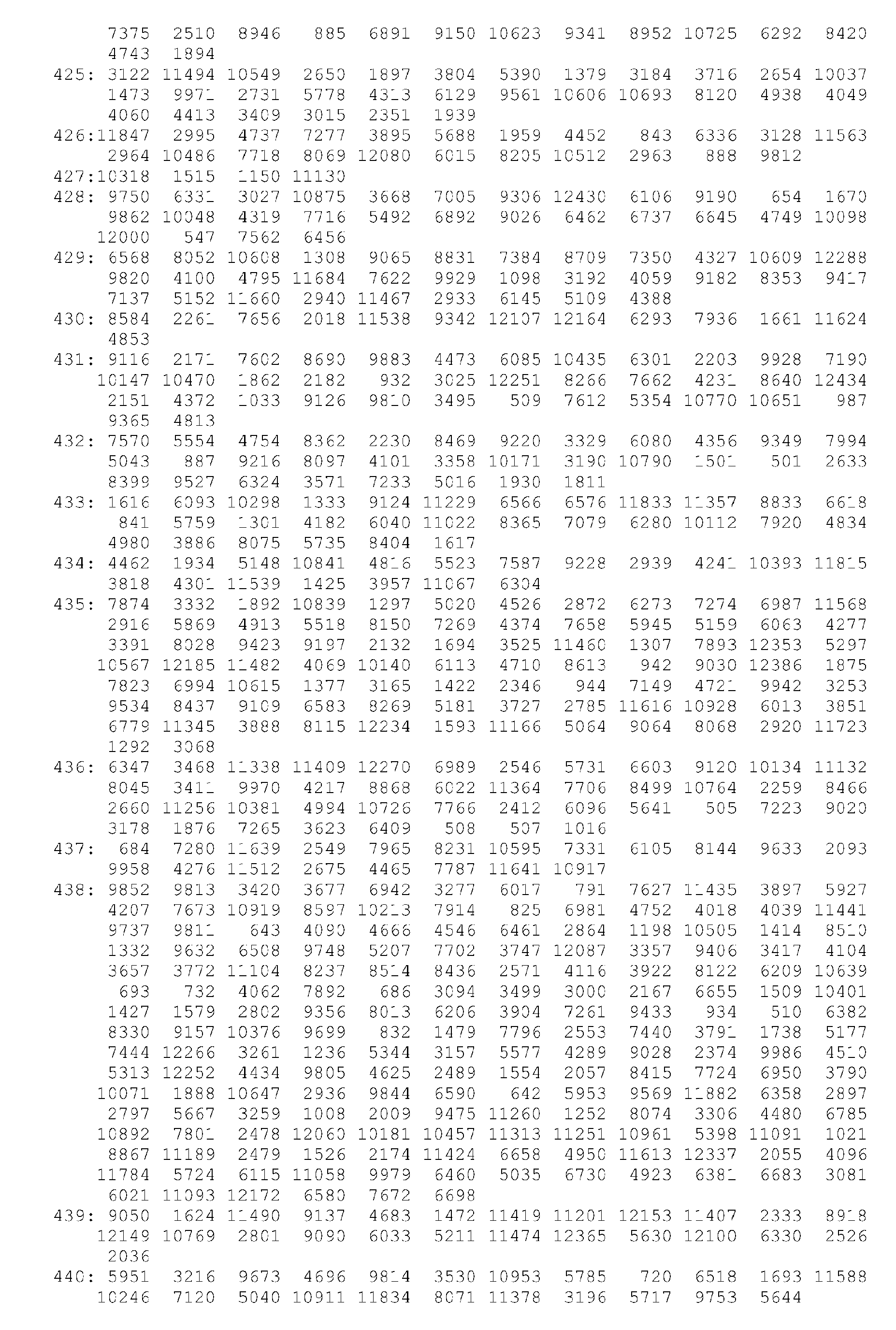

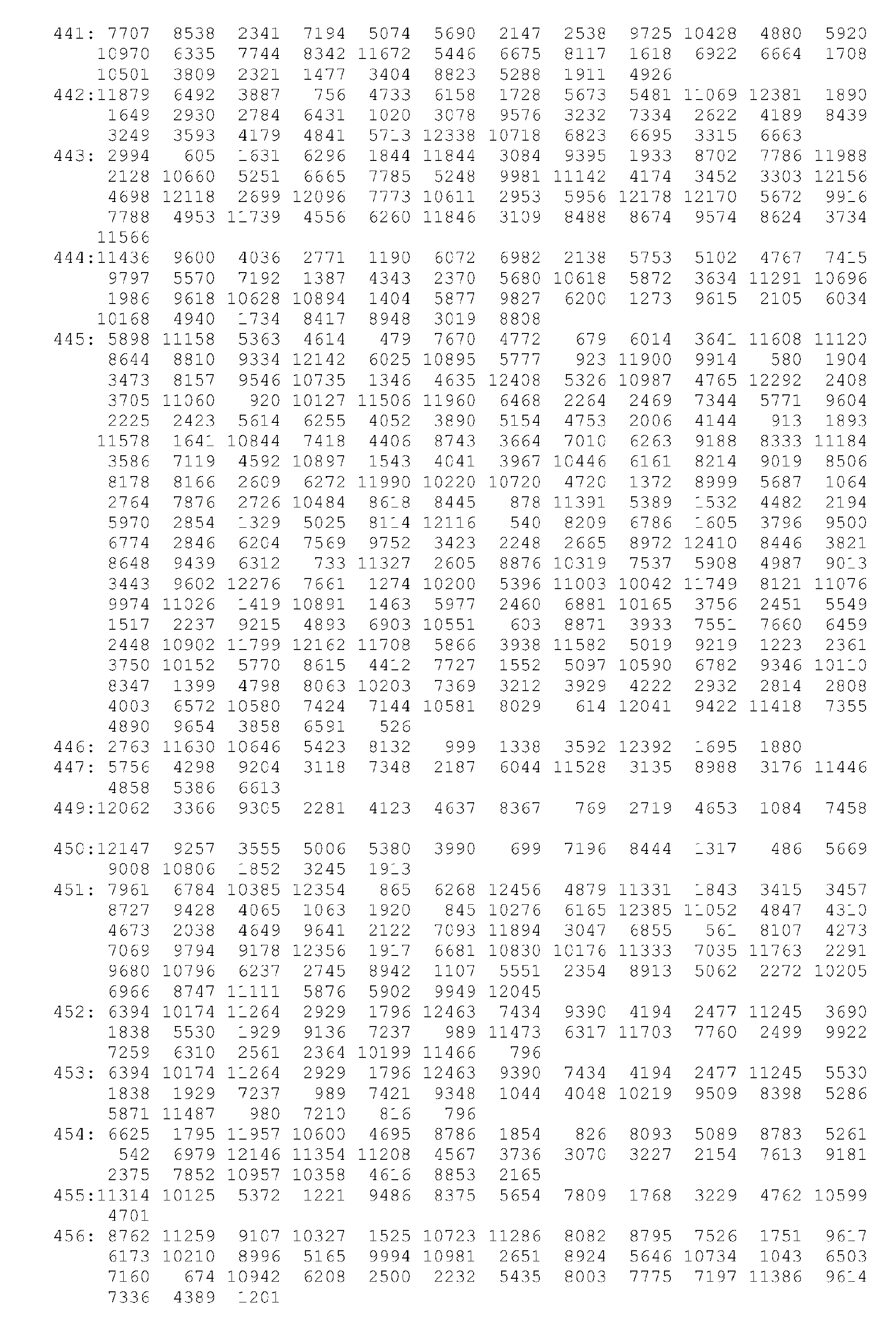

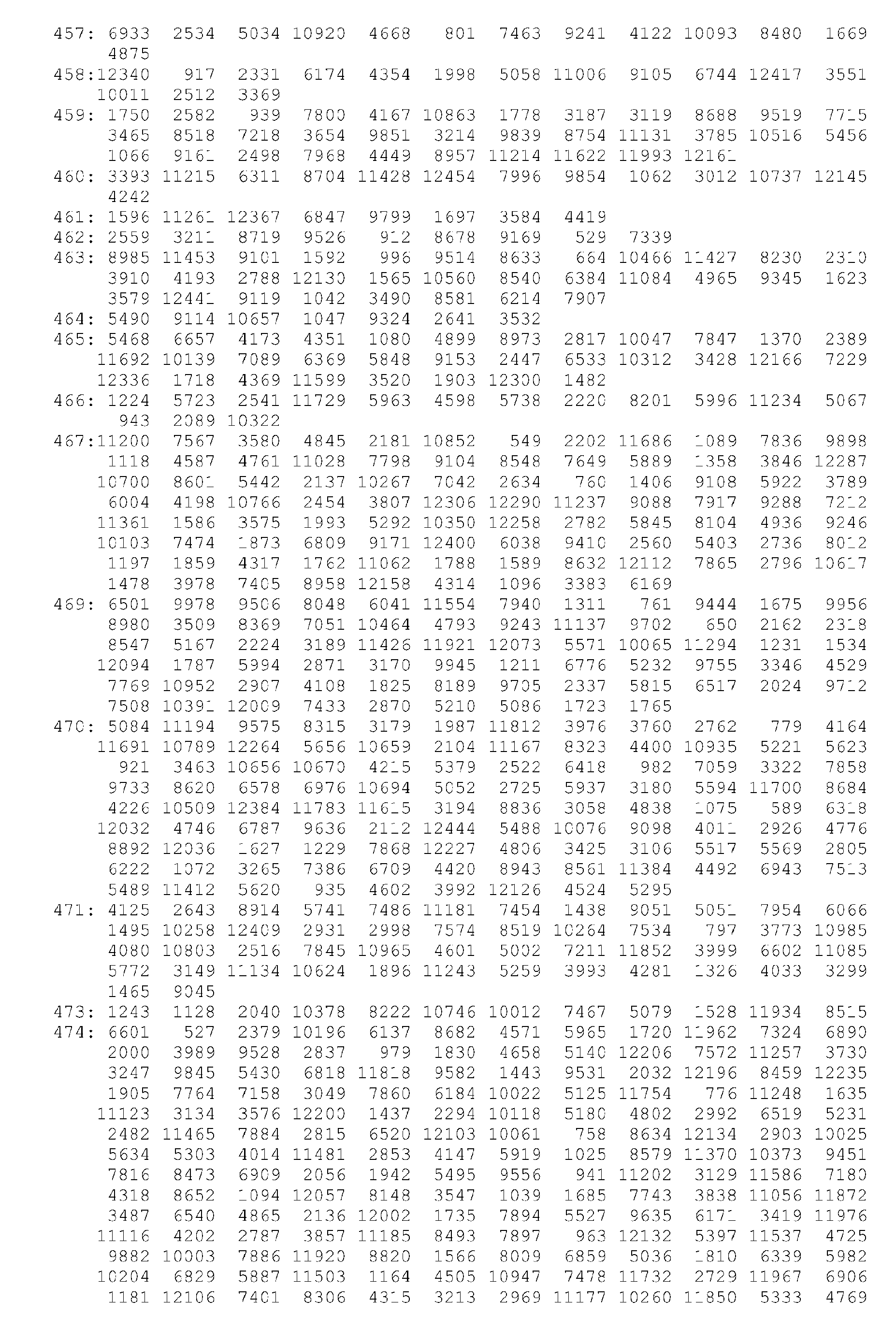

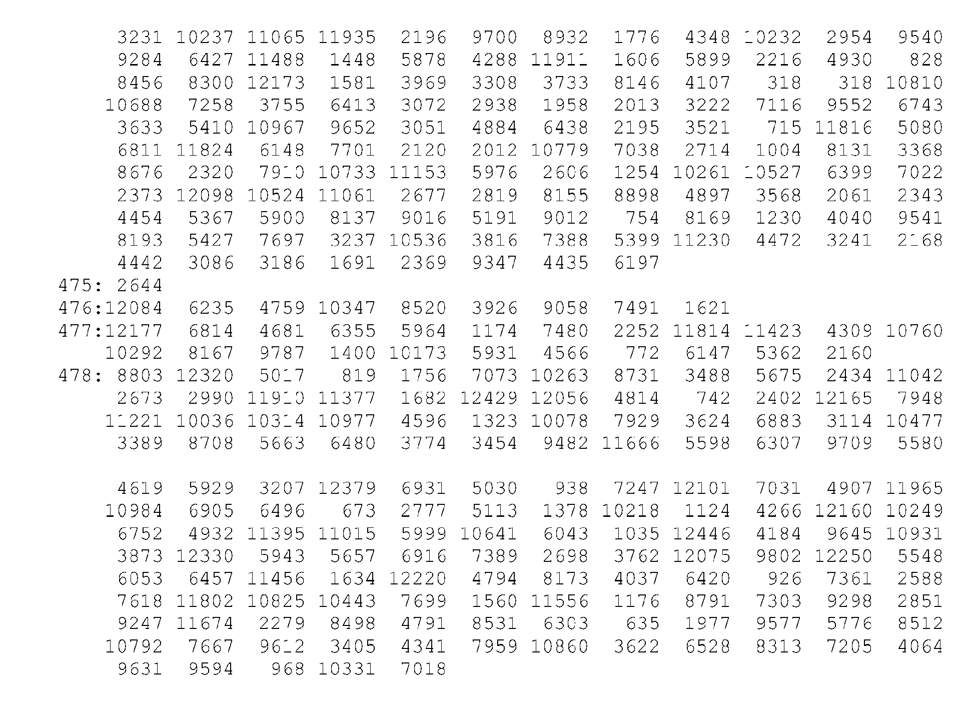

Transgenic seed for a crop, wherein the genome of said transgenic seed comprises trait-improving recombinant DNA for expressing a bacterial phytochrome protein.

Transgenic seed as defined above, wherein said protein provides improved tolerance to cold stress

Transgenic seed as defined above, wherein said protein provides improved tolerance to water deficit stress.

Transgenic seed as defined above, wherein said protein provides improved tolerance to reduced nitrogen availability stress.

A transgenic seed as defined above wherein said DNA is derived from a *Pseudomonas florescens.*

Transgenic seed as defined above, wherein transgenic plants grown from said seed exhibit increased yield as compared to similar plants without the recombinant DNA.

Transgenic seed as defined above, wherein
(a) said crop is susceptible to a yield-limiting environment; and
(b) transgenic plants grown from said transgenic seed as defined above thrive in said yield-limiting environment.

Transgenic seed as defined above, wherein said yield-limiting environment is cold stress, water deficit stress or nitrogen availability stress.

A method of facilitating production of a crop comprising providing to a grower of said crop transgenic seed as defined above.

A method as defined above, wherein transgenic plants grown from said seed exhibit increased yield as compared to similar plants without the recombinant DNA.

A method as defined above, wherein
(a) said crop is susceptible to a yield-limiting environment; and
(b) transgenic plants grown from said transgenic seed thrive in said yield-limiting environment.

A method as defined above, wherein said yield-limiting environment is cold stress, water deficit stress or nitrogen availability stress.

## Claims

1. Transgenic seed for a crop, wherein the genome of said transgenic seed comprises trait-improving recombinant DNA from a gene for a protein having amino acid sequence with at least 90% identity to a consensus amino acid sequence in the group consisting of a consensus amino acid sequence for SEQ ID NO: 240 and homologs thereof through a consensus amino acid sequence for SEQ ID NO: 478 and homologs thereof, but excluding SEQ ID NO:391 and homologs thereof.

2. Transgenic seed according to claim 1, wherein said protein has an amino acid sequence selected from the group consisting of SEQ ID NO: 240 through SEQ ID NO: 390, SEQ ID NO: 392 through SEQ ID NO: 478.

3. Transgenic seed according to claim 1, wherein said recombinant DNA is used to produce said protein.

4. Transgenic seed according to claim 1, wherein said recombinant DNA is used to suppress the level of an endogenous protein.

5. Transgenic seed according to claim 1, wherein
(a) said protein has the function of a specific protein that has been demonstrated in a model plant with efficacy for an improved trait as compared to a plant without said recombinant DNA wherein said specific protein and trait are as indicated in Table 5; and
(b) transgenic plants grown from said transgenic seed exhibit said improved trait

6. Transgenic seed according to claim 3, wherein said protein provides the trait indicated in Table 5 for a model plant -expressed protein which has an amino acid sequence which was used with homologs to build said consensus amino acid sequence and wherein transgenic plants grown from said transgenic seed exhibit an improved trait which was demonstrated in the model plant expressing said model plant-expressed protein

7. Transgenic seed according to claim 1, wherein transgenic plants grown from said seed exhibit increased yield as compared to similar plants without the recombinant DNA.

8. Transgenic seed according to claim 1, wherein
(a) said crop is susceptible to a yield-limiting environment; and
(b) transgenic plants grown from said transgenic seed thrive in said yield-limiting environment.

9. Transgenic seed according to claim 8, wherein
(a) transgenic plants grown from said seed exhibit increased yield as compared to similar plants without the recombinant DNA when said plants are grown in a yield-limiting environment of water deficit stress and said protein has the function of the protein with an amino acid sequence selected from the group consisting of SEQ ID NO: 268 through 294, 298, 345 through 357, 359, 367 through 369, 374, 398 through 424, 435, 439, 463 through 478, and homologs thereof;
(b) transgenic plants grown from said seed exhibit increased yield as compared to similar plants without the recombinant DNA when said plants are grown in a yield-limiting environment of heat stress and said protein has the function of the protein with an amino acid sequence selected from the group consisting of SEQ ID NO: 268 through 294, 298, 347, 359, 367-369, 435, and homologs thereof.
(c) transgenic plants grown from said seed exhibit increased yield as compared to similar plants without the recombinant DNA when said plants are grown in a yield-limiting environment of high salinity stress and said protein has the function of the protein with an amino acid sequence selected from the group consisting of SEQ ID NO: 298, 424, 439, 463 through 478, and homologs thereof;
(d) transgenic plants grown from said seed exhibit increased yield as compared to similar plants without the recombinant DNA when said plants are grown in a yield-limiting environment of cold stress and said protein has the function of the protein with an amino acid sequence selected from the group consisting of SEQ ID NO: 240 through 267, 276, 277, 298, 346, 366, 367, 434, and homologs thereof;
(e) transgenic plants grown from said seed exhibit increased yield as compared to similar plants without the recombinant DNA when said plants are grown in a yield-limiting environment of reduced nitrogen availability stress and said protein has the function of the protein with amino acid sequence of SEQ ID NO: 315 through 344, 364, 370 through 373, 438, and homologs thereof;
(f) transgenic plants grown from said seed exhibit increased yield as compared to similar plants without the recombinant DNA when said plants are grown in a yield-limiting environment of shade stress and said protein has the function of the protein with an amino acid sequence selected from the group consisting of SEQ ID NO: 295 through 314, 370, 427, 436, 437, and homologs thereof.

10. A recombinant DNA construct comprising a promoter functional in a plant cell operably linked to trait-improving recombinant DNA from gene for a protein having an amino acid sequence with at least 90% identity to a consensus amino acid sequence in the group consisting of a consensus amino acid sequence for SEQ ID NO: 240 and homologs thereof through a consensus amino acid sequence for SEQ ID NO: 478 and homologs thereof, but excluding SEQ ID NO:391 and homologs thereof.

11. A recombinant DNA construct according to claim 10, wherein said protein has an amino acid sequence selected from the group consisting of SEQ ID NO: 240 and through SEQ ID NO: 390, SEQ ID NO: 392 through SEQ ID NO: 478,

12. A recombinant DNA construct according to claim 10, wherein said recombinant DNA is used to produce provides said protein.

13. A recombinant DNA construct according to claim 10, wherein said recombinant DNA is used to suppress the level of an endogenous protein.

14. A method of facilitating production of a crop comprising providing to a grower of said crop transgenic seed of claim 1.

15. A method according to claim 14, wherein transgenic plants grown from said seed exhibit increased yield as compared to similar plants without the recombinant DNA.

16. A method according to claim 14, wherein
(a) said crop is susceptible to a yield-limiting environment; and
(b) transgenic plants grown from said transgenic seed thrive in said yield-limiting environment.

17. A method according to claim 16, wherein
(a) transgenic plants grown from said seed exhibit increased yield as compared to similar plants without the recombinant DNA when said plants are grown in a yield-limiting environment of water deficit stress and said protein has the function of the protein with an amino acid sequence selected from the group consisting of SEQ ID NO: 268 through 294, 298, 345 through 357, 359, 367 through 369, 374, 398 through 424, 435, 439, 463 through 478, and homologs thereof;
(b) transgenic plants grown from said seed exhibit increased yield as compared to similar plants without the recombinant DNA when said plants are grown in a yield-limiting environment of heat stress and said protein has the function of the protein with an amino acid sequence selected from the group consisting of SEQ ID NO: 268 through 294,298, 347, 359, 367 through 369, 435, and homologs thereof;
(c) transgenic plants grown from said seed exhibit increased yield as compared to similar plants without the recombinant DNA when said plants are grown in a yield-limiting environment of high salinity stress and said protein has the function of the protein with an amino acid sequence selected from the group consisting of SEQ ID NO: 298, 424, 43 9, 463 through 478, and homologs thereof;
(d) transgenic plants grown from said seed exhibit increased yield as compared to similar plants without the recombinant DNA when said plants are grown in a yield-limiting environment of cold stress and said protein has the function of the protein with an amino acid sequence selected from the group consisting of SEQ ID NO: 240 through 267, 276, 277, 298, 346, 366, 367, 434 and homologs thereof;
(e) transgenic plants grown from said seed exhibit increased yield as compared to similar plants without the recombinant DNA when said plants are grown in a yield-limiting environment of reduced nitrogen availability stress and said protein has the function of the protein with amino acid sequence of SEQ ID NO: 315 through 344, 364, 370 through 373, 438 and homologs thereof;
(f) transgenic plants grown from said seed exhibit increased yield as compared to similar plants without the recombinant DNA when said plants are grown in a yield-limiting environment of shade stress and said protein has the function of the protein with an amino acid sequence selected from the group consisting of SEQ ID NO: 295 through 314, 370, 427, 436, 437 and homologs thereof.
